# EUROPEAN PATENT SPECIFICATION

(11) **EP 3 579 858 B1**
(45) Date of publication and mention of the grant of the patent: **25.11.2026**
(21) Application number: 18750827.0
(22) Date of filing: 07.02.2018
(51) Int. Cl.: A61K 38/17, C12N 9/22, C12N 15/113, A61P 3/04, A61P 13/12, A61P 25/00, A61P 25/08, A61P 25/28, A61P 27/02, A61P 43/00

(54) **GENE THERAPY FOR HAPLOINSUFFICIENCY**
GENTHERAPIE FÜR HAPLOINSUFFIZIENZ
THÉRAPIE GÉNIQUE CONTRE L'HAPLO-INSUFFISANCE

(30) Priority: 07.02.2017 US 201762455988 P
(43) Date of publication of application: 18.12.2019
(73) Proprietor: The Regents of the University of California, Oakland, CA 94607 (US)
(72) Inventor: AHITUV, Nadav, Oakland, California 94607-5200 (US); MATHARU, Navneet, Oakland, California 94607-5200 (US)
(74) Representative: J A Kemp LLP
(86) International application number: PCT/US2018/017186
(87) International publication number: WO 2018/148256

(56) References cited:
- EP-A1- 3 045 537
- EP-A1- 3 510 152
- WO-A1-2014/204723
- WO-A1-2014/204729
- WO-A1-2016/083360
- WO-A1-2016/187717
- WO-A2-2015/089486
- US-A1- 2016 039 893
- LI-FANG JIN ET AL: "Generation of genetically modified mice using CRISPR/Cas9 and haploid embryonic stem cell systems", ZOOLOGICAL RESEARCH, 1 July 2016 (2016-07-01), Kunming, pages 205 - 2132095, XP055435292, Retrieved from the Internet <URL:https://www.cnki.net/kcms/doi/10.13918/j.issn.2095-8137.2016.4.205.html> DOI: 10.13918/j.issn.2095-8137.2016.4.205
- NAVNEET MATHARU ET AL: "CRISPR-mediated activation of a promoter or enhancer rescues obesity caused by haploinsufficiency", SCIENCE, vol. 363, no. 6424, 13 December 2018 (2018-12-13), US, pages eaau0629, XP055748390, ISSN: 0036-8075, DOI: 10.1126/science.aau0629
- LI ET AL.: "An episomal CRISPR/Cas9 system to derive vector-free gene modified mammalian cells", PROTEIN CELL, vol. 7, no. 9, 29 July 2016 (2016-07-29), pages 689 - 691, XP036044476

## Description

### FIELD OF INVENTION

The present disclosure relates generally to methods and compositions for activating transcription in mammalian cells.

### BACKGROUND OF THE INVENTION

Genomic alterations resulting in reduced transcription or activity of one or more genes or gene products are a causative factor in a myriad of mammalian diseases. One such genomic alteration is haploinsufficiency, in which there is only one functional copy of a gene and that single copy does not produce enough of the gene product to produce a wild-type phenotype. Other diseases are caused by genomic alterations in one or both copies of a gene that alter the gene product so that it exhibits a reduction, but not elimination, in activity. In still other diseases, genomic alterations reduce transcription or reduce transcript stability of one or both copies of a gene, such that there is insufficient gene product to produce a wild-type phenotype. Numerous approaches have been attempted to treat such diseases by augmenting the amount or activity of the one or more genes reduced in transcription or activity. Such approaches include delivery into the genome of a wild-type copy of the one or more genes. Recently, targeted introduction into a genome has been demonstrated using methods and compositions based on clustered regularly interspaced short palindromic repeats (CRISPR), Zinc Finger Nucleases (ZFNs) (see, Umov et al., Nat. Rev. Genet., 11:636-646 (2010) or transcription activator-like effector nucleases (TALENs) (see, Joung and Sander, Nat. Rev. Mol. Cell Biol., 1:49-55 (2013). Other approaches for increasing transcription of one or more target genes include the use of antisense oligomers that promote constitutive splicing (see, US 2016/0298121). However, there remains a need for alternative methods and compositions for increasing the transcription of target genes to treat diseases caused by their reduced transcription, amount, or activity.

WO 2015/089486 describes systems, methods and compositions for sequence manipulation with optimized functional CRISPR-CAS systems.

EP 3045537 describes engineering and optimization of systems, methods and compositions for sequence manipulation with functional domains.

WO 2014/204729 describes delivery, use and therapeutic applications of CRISPR-CAS systems and compositions for targeting disorders and diseases using viral components.

WO 2014/204723 describes oncogenic models based on delivery and use of CRISPR-CAS systems, vectors and compositions.

Jin, Li-Fang, and Jin-Song Li, Zoological research, vol. 37,4 (2016): 205-13 describes the generation of genetically modified mice using CRISPR/Cas9 and haploid embryonic stem cell systems.

WO 2016/083360 describes the diagnosis of lung cancer.

### SUMMARY OF THE INVENTION

The invention provides a composition comprising:
i) a guide RNA, wherein the guide RNA comprises:
   a) a targeting region that, under conditions present in a nucleus of the cell, specifically hybridizes to a promoter region or an enhancer region operably linked to a wild-type copy of a haploinsufficient gene, wherein the haploinsufficient gene is SCN2A, SIM1 or MC4R; and
   b) a CRISPR nuclease-binding region that specifically binds a CRISPR nuclease under conditions present in a nucleus of the cell or a region that specifically binds to the CRISPR nuclease-binding region; and
ii) the CRISPR nuclease,
for use in a method of treating haploinsufficiency disease in a mammalian subject, the method comprising contacting a cell of the subject with the composition;
- wherein the contacting forms a complex comprising the CRISPR nuclease bound to the guide RNA, wherein the targeting region of the guide RNA in the complex is hybridized to the promoter or enhancer;
- wherein the complex comprises a catalytically inactive CRISPR nuclease and a transcriptional activation domain, and
- wherein the complex activates transcription of the wild-type copy of the SCN2A, SIM1 or MC4R gene that in the absence of transcriptional activation by the complex does not produce enough of a corresponding gene product to produce a wild-type phenotype in the subject, in an amount and for a duration sufficient to treat the haploinsufficiency disease in the subject.

The invention also provides an isolated mammalian host cell comprising:
I.) a genome comprising at least one functional copy of a target gene, wherein the target gene is selected from the group consisting of SCN2A, SIM1 and MC4R, wherein the functional cop(y/ies) in the absence of transcriptional activation by a heterologous complex do not produce enough of a corresponding gene product to produce a wild-type phenotype in an organism; and
II.) the heterologous complex, wherein the heterologous complex comprises:
   a) a guide RNA, wherein the guide RNA comprises:
      i.) a targeting region that specifically hybridizes to a promoter region or an enhancer region operably linked to the functional cop(y/ies) of the target gene under conditions present in a nucleus of the cell; and
      ii.) a CRISPR nuclease-binding region that specifically binds a CRISPR nuclease under conditions present in a nucleus of the cell; and
   b) the CRISPR nuclease,
      - wherein the guide RNA of the heterologous complex comprising the CRISPR nuclease bound to the guide RNA is hybridized to the promoter or enhancer;
      - wherein the CRISPR nuclease is catalytically inactive, and
      - wherein the complex activates transcription of the functional cop(y/ies) of the SCN2A, SIM1 or MC4R target gene in an amount and for a duration sufficient to produce a wild-type phenotype when the host cell is present in an organism.

### SUMMARY OF TECHNICAL TEACHINGS

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Described herein are methods and compositions for increasing transcription of target genes in a mammalian (e.g., human) subject. The inventors have discovered that such increased transcription can be achieved with a transcription-activating guide-RNA (gRNA) construct (e.g., as part of a dCAS9/gRNA complex) targeted to a promoter or enhancer region of a gene. Moreover, the inventors have discovered that transcriptional activation in amounts and for periods of time that are sufficient to treat a disease can be achieved with a non-integrating vector. In some cases, the methods and compositions for transcriptional activation do not covalently modify the genome of the host mammal by endonuclease cleavage, nicking, and/or repair. In some cases, the non-integrating vector is an episomal vector, such as an adeno associated viral vector.

The present invention provides a composition comprising: i) a guide RNA, wherein the guide RNA comprises: a) a targeting region that, under conditions present in a nucleus of the cell, specifically hybridizes to a promoter region or an enhancer region operably linked to a wild-type copy of a haploinsufficient gene, wherein the haploinsufficient gene is SCN2A, SIM1 or MC4R; and b) a CRISPR nuclease-binding region that specifically binds a CRISPR nuclease under conditions present in a nucleus of the cell or a region that specifically binds to the CRISPR nuclease-binding region; and ii) the CRISPR nuclease, for use in a method of treating haploinsufficiency disease in a mammalian subject, the method comprising contacting a cell of the subject with the composition; -wherein the contacting forms a complex comprising the CRISPR nuclease bound to the guide RNA, wherein the targeting region of the guide RNA in the complex is hybridized to the promoter or enhancer; -wherein the complex comprises a catalytically inactive CRISPR nuclease and a transcriptional activation domain, and -wherein the complex activates transcription of the wild-type copy of the SCN2A, SIM1, or MC4R gene that in the absence of transcriptional activation by the complex does not produce enough of a corresponding gene product to produce a wild-type phenotype in the subject, in an amount and for a duration sufficient to treat the haploinsufficiency disease in the subject. In some embodiments, the mammalian subject is treated with a host cell obtained from the subject. In one embodiment, the mammalian subject is treated with a host cell obtained from a different (distinct) mammalian subject. In some embodiments, the host cell is an isolated mammalian host cell. In another embodiment, the host cell comprises an isolated mammalian host cell having one functional copy of a target gene.

In some embodiments, the contacting comprises contacting the cell with an episomal vector encoding the guide RNA or the CRISPR nuclease. In some embodiments, the contacting comprises contacting the cell with an episomal vector encoding the guide RNA and the CRISPR nuclease. In some embodiments, the contacting comprises contacting the cell with an episomal vector encoding the guide RNA and a second episomal vector encoding the CRISPR nuclease. In some embodiments, the episomal vector(s) are non-integrating. In some embodiments, the episomal vector(s) are non-replicating. In some embodiments, the episomal vector(s) are adeno-associated virus (AAV) vectors. In some embodiments, the episomal vector(s) independently comprise a first and a second end, wherein the first end and second end each independently comprise an AAV inverted terminal repeat.

In some embodiments, the CRISPR nuclease comprises (i) a nuclease domain that has been modified to eliminate nuclease and nicking activity and (ii) a transcriptional activation domain. In some embodiments, the CRISPR nuclease comprises a Cas9 or Cpf1 nuclease. In some embodiments, the CRISPR nuclease has a modification and the modification comprises a mutation at positions corresponding to D10 and H840 of *S. pyogenes* Cas9. In some embodiments, the CRISPR nuclease comprises a D10A, H840A *S. pyogenes* dCas9. In some embodiments, the CRISPR nuclease comprises a S. *aureus* dCas9. In some embodiments the S. *aureus* dCas9 comprises one or more mutations in one of the following residues: E782, K929, N968, R1015. In some embodiments, the guide RNA comprises a dead guide sequence.

In some embodiments, the guide RNA comprises a transcriptional activation binding domain, wherein the transcriptional activation binding domain specifically binds a composition comprising one or more transcriptional activation domains. In some embodiments, the complex comprising the CRISPR nuclease bound to the guide RNA further comprises a transcriptional activation domain selected from the group consisting of HSF1, VP16, VP64, p65, MyoD1, RTA, SET7/9, VPR, histone acetyltransferase p300, an hydroxylase catalytic domain of a TET family protein *(e.g.,* TET1 hydroxylase catalytic domain), LSD1, CIB1, AD2, CR3, EKLF1, GATA4, PRVIE, p53, SP1, MEF2C, TAX, and PPARγ. In some embodiments, the CRISPR nuclease is a CRISPR nuclease-VP64 fusion polypeptide.

In some embodiments, the guide RNA comprises a scaffold region. In some embodiments, the scaffold region comprises an ms2, f6, PP7, com, or L7a ligand sequence. In some embodiments, the scaffold region of the guide RNA in the complex is bound to a transcriptional activation domain fused to an MCP polypeptide, a COM polypeptide, a PCP polypeptide, or an L7a polypeptide. In some aspects, the haploinsufficient gene is *SIM1*, *Leptin, Leptin receptor, MC4R, SCN2A, SETD5, PAX6, PKD1, MC3R, POMC, STAT3, STATS, SOCS3, GHR, NPY, NPYIR, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPT1B, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.*

In some aspects, the targeting region of the guide RNA is encoded by or specifically hybridizes to: SEQ ID NO:1 (GACACGGAATTCATTGCCAG), SEQ ID NO:2 ( CTGCGGGTTAGGTCTACCGG), SEQ ID NO:3 (GTTGAGCGCTCAGTCCAGCG), SEQ ID NO:4 (TCCCGACGTCGTGCGCGACC), or SEQ ID NO:5 (GCTCTGAATCTTACTACCCG). In some aspects, the targeting region of the guide RNA is encoded by or specifically hybridizes to: SEQ ID NO:6 (GCTGTTAACTAAAGACAGGG), SEQ ID NO:7 (GTGGTCTGGGTGATCTCATG), SEQ ID NO:8 (GACAAAGGAACATCTGAGAGG), SEQ ID NO:9 (GTGATCTCATGGGGAAGAGG), or SEQ ID NO:10 (GGCTTTGATCGTGGTCTGGG). In some aspects, the targeting region of the guide RNA is encoded by or specifically hybridizes to: SEQ ID NO:11 (GCGAGCCCAGTCGCGTGGGG), or SEQ ID NO: 12 (GCCAAGAATTGGCCAAAGGG), SEQ ID NO:34 (GTCAAAGGGGCATATGGAAGG), SEQ ID NO:35 (GGGAAGAAAGCCCCACTTGG), SEQ ID NO:36 (GCCCAGTCGCGTGGGGGGGG), or SEQ ID NO:37 (GGAGCGCGAGTGTCACTCGG). In an embodiment, the targeting region of the guide RNA is encoded by or specifically hybridizes to: SEQ ID NO:38 (GCTCACTGTAGGACCCGAGCC), SEQ ID NO:39 (GACGCGGCGCTCATTGGCCAA), SEQ ID NO:40 (CGAGCCGCGAGCCCAGTCGCG), SEQ ID NO:41 (TCCCCCCCCCCCCCCACGCGA), SEQ ID NO:42 (GTCACTCACCCCGATTGGCCA), or SEQ ID NO:43 (CGCGAGCCCAGTCGCGTGGGG). In some embodiments, the targeting region of the guide RNA is encoded by or specifically hybridizes to: SEQ ID NO:44 (GTTGGCTTATCCAAACATCTC), SEQ ID NO:45 (ATGTTAAGCAAGGGTAATAGA), SEQ ID NO:46 (CTGTGAAAGGAATACAATTCA), SEQ ID NO: 47 (GCCAATTCTTGGCAACCGAGC), or SEQ ID NO:49 (AATTAGCAGACAGCTTGGTAC). In some aspects, the targeting region of the guide RNA is encoded by or specifically hybridizes to: SEQ ID NO:48 (GAATTGGCCAAAGGGAGGGGT). In some embodiments, the targeting region of the guide RNA is encoded by or specifically hybridizes to: SEQ ID NO:51 (CACTGAATACGGATTGGTCAG), SEQ ID NO:52 (GATGTCTCAGAACCACTGAAT), or SEQ ID NO:54 (ACCAATCCGTATTCAGTGGTT). ). In some aspects, the targeting region of the guide RNA is encoded by or specifically hybridizes to: SEQ ID NO:50 (CTGGCTGATTCCCGAGGATTT) or SEQ ID NO:53 (AACCACTGAATACGGATTGGT). In some aspects, the targeting region of the guide RNA is encoded by or specifically hybridizes to: SEQ ID NO:55 (GGCGCGGGGCGGACGGGGCGA), SEQ ID NO:56 (GCGCCCCGGGAACGCGTGGGG), SEQ ID NO:57 (CGCCCCGCGCCGCGCGGGGAG), SEQ ID NO:58 (TCCGCCCCGCGCCGCGCGGGG), SEQ ID NO:59 (GGAACGCGTGGGGCGGAGCTT), SEQ ID NO:60 (GCCCCGCGCCGCGCGGGGAGG), SEQ ID NO:61 (TGCGCCCCGGGAACGCGTGGG), SEQ ID NO:62 (GAACGCGTGGGGCGGAGCTTC), SEQ ID NO:63 (GCGGCGCGGGGCGGACGGGGC), or SEQ ID NO:64 (CCCGTCCGCCCCGCGCCGCGC). In some aspects, the targeting region of the guide RNA is encoded by or specifically hybridizes to: SEQ ID NO:65 (GGCCCACTCGCCGCCAATCAG), SEQ ID NO:66 (GGAAGCCGCCGGGGCCGCCTA), SEQ ID NO:67 (TGATTGGCGGCGAGTGGGCCA), SEQ ID NO:68: (GCCGCCAATCAGCGGAAGCCG), SEQ ID NO:69: (GGCGGCTTCCGCTGATTGGCG), SEQ ID NO:70: (CCGCCAATCAGCGGAAGCCGC), SEQ ID NO:71: (AGCCGCCGGGGCCGCCTAGAG), SEQ ID NO:72: (GCTTCCGCTGATTGGCGGCGA), SEQ ID NO:73: (CGGCGAGTGGGCCAATGGGTG), or SEQ ID NO:74: (CCAATGGGTGCGGGGCGGTGG). In some aspects, the targeting region of the guide RNA is encoded by or specifically hybridizes to: SEQ ID NO:75 (GGCTGCCGGGGCCGCCTAAAG), SEQ ID NO:76 (GGAGGCTGCCGGGGCCGCCTA), SEQ ID NO:77 (GCCGCCAATCAGCGGAGGCTG), SEQ ID NO:78 (CCGCCAATCAGCGGAGGCTGC), SEQ ID NO:79 (TGGCCGGTGCGCCGCCAATCA), SEQ ID NO:80 (GGCCGGTGCGCCGCCAATCAG), SEQ ID NO: 8 1 (CGGCGCACCGGCCAATAAGTG), SEQ ID NO:82(ATAAGTGTGGGGCGGTGGGCG), SEQ ID NO:83 (CCAATAAGTGTGGGGCGGTGG), or SEQ ID NO:84 (CAATAAGTGTGGGGCGGTGGG). In some embodiments, the targeting region of the guide RNA is encoded by or specifically hybridizes to: SEQ ID NO:85 (CCTTTCTATGACCTAGTCGG), SEQ ID NO:86 (CAGAATCAGTAACGCACTGT), SEQ ID NO:87 (GAAACCAGGAGAGATAACCC), SEQ ID NO:88 (GGACCCCAGATATTCTGGAA), SEQ ID NO:89 (TTATTGTTGACTTAACGAAG). In some aspects, the targeting region of the guide RNA is encoded by or specifically hybridizes to: SEQ ID NO:90 (AAAAAGAAGCAAATAGCTAA), or SEQ ID NO:91 (AGAATCAGTAACGCACTGTA). In some embodiments, the targeting region of the guide RNA is encoded by or specifically hybridizes to: SEQ ID NO:92 (TGTTGGTTTATTGGACCCCAGATATTC), SEQ ID NO:93 (TGTTGGAGAAAATTAACTTAGTGCATA), or SEQ ID NO:94 (TGTTGGTATAACTGCCACTAGAGGGCT). In some aspects, the targeting region of the guide RNA is encoded by or specifically hybridizes to SEQ ID NO:95 (AGGAGCCGGGACCCACCGG).

In some embodiments, the cell is a non-dividing cell. In some embodiments, the cell is a neuron. In some embodiments, the cell is a hypothalamus cell. In some embodiments, the contacting comprises injection of nucleic acid encoding the guide RNA and/or the CRISPR nuclease into a region of a brain containing a hypothalamus. In some embodiments, the contacting comprises injection of an adeno-associated viral vector comprising nucleic acid encoding the guide RNA and/or the CRISPR nuclease into a region of a brain containing a hypothalamus. In some aspects, the haploinsufficiency disease is selected from Table 1. In some aspects, the haploinsufficiency disease is selected from obesity, autism, epilepsy, intellectual disability, aniridia, and polycystic kidney disease. In some embodiments, the haploinsufficiency disease is obesity.

The present invention also provides an isolated mammalian host cell comprising: I.) a genome comprising at least one functional copy of a target gene, wherein the target gene is selected from the group consisting of SCN2A, SIM1 and MC4R, wherein the functional cop(y/ies) in the absence of transcriptional activation by a heterologous complex do not produce enough of a corresponding gene product to produce a wild-type phenotype in an organism; and II.) the heterologous complex, wherein the heterologous complex comprises: a) a guide RNA, wherein the guide RNA comprises: i.) a targeting region that specifically hybridizes to a promoter region or an enhancer region operably linked to the functional cop(y/ies) of the target gene under conditions present in a nucleus of the cell; and ii.) a CRISPR nuclease-binding region that specifically binds a CRISPR nuclease under conditions present in a nucleus of the cell; and b) the CRISPR nuclease, -wherein the guide RNA of the heterologous complex comprising the CRISPR nuclease bound to the guide RNA is hybridized to the promoter or enhancer; -wherein the CRISPR nuclease is catalytically inactive, and -wherein the complex activates transcription of the functional cop(y/ies) of the SCN2A, SIM1 or MC4R target gene in an amount and for a duration sufficient to produce a wild-type phenotype when the host cell is present in an organism.

In some embodiments, the genome comprises a single functional copy of the target gene. In some embodiments, the single functional copy of the target gene comprises a haploinsufficient gene. In some embodiments, the genome comprises less than two functional copies of the target gene.

### BRIEF DESCRIPTION OF THE DRAWINGS

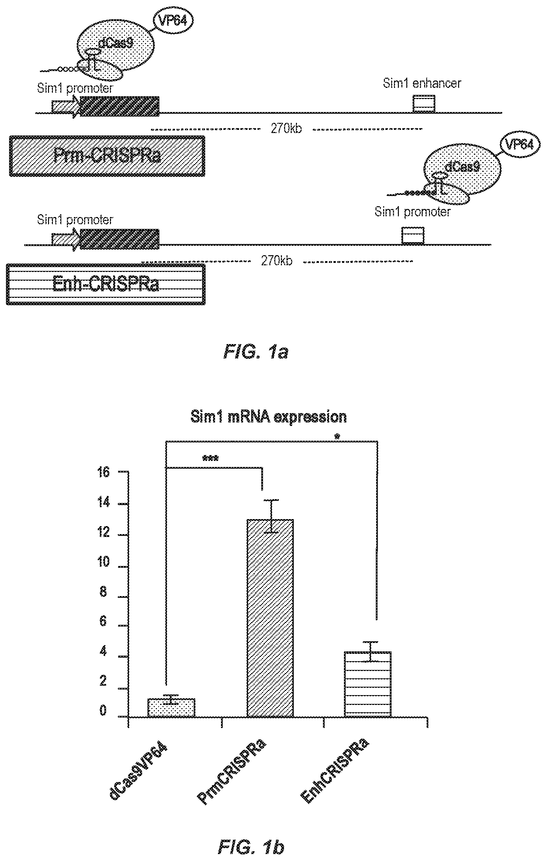

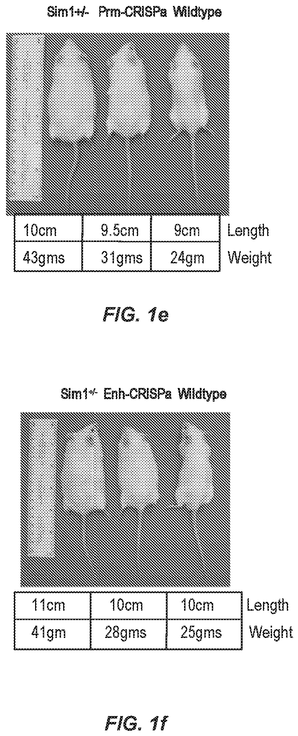

**Figs. 1A****-F: Transgenic CRISPRa *Sim1* overexpression *in vitro* and *in vivo. A,*** Schema of the mouse *Sim1* genomic locus. **B,** CRISPRa in Neuro-2A cells targeting the *Sim1* promoter (Pr) or enhancer (Enh). Results are expressed as mRNA fold-increase normalized to beta-actin using the ΔΔCT method. The mean values±s.d. were obtained from 3 independent experiments. * = p-value < 0.001 *** = p-value < 0.0005 (ANOVA, Tukey test). **C,** Schema showing the various mouse lines and mouse transgenic CRISPRa concept. **D,** Weekly weight measurements of wild-type littermates, *Sim1*^{+/-}, *H11P^{CAG-dCas9-VP64}* X *ROSA26^{Sim1Pr-sgRNA}* and *H11P^{CAG-dCas9-VP64}* X *ROSA26^{SCE2En-sgRNA}.* At least 10 male and female mice were measured per genotype. Mean values±s.d are shown. **E-F,** Pictures showing 20 week old mice for each genotype: *Sim1*^{+/-}, *H11P^{CAG-dCas9-VP64}* X *ROSA26^{Sim1Pr-sgRNA}* and wild-type littermate **(E)** and *Sim1*^{+/-}, *H11P^{CAG-dCas9-VP64}* X *ROSA26^{SCE2En-sgRNA}* and wild-type littermate **(F).** Length and weight of each mice are depicted above and below respectively.

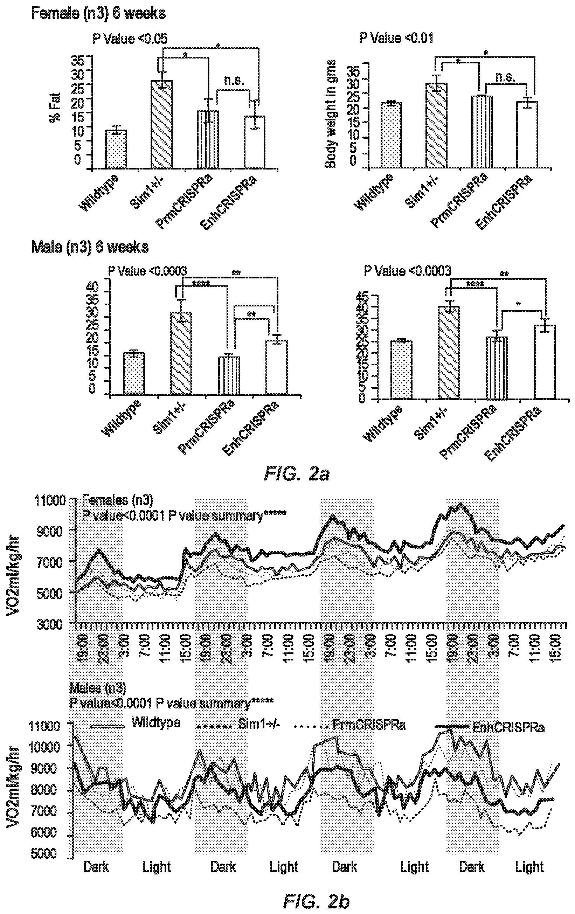

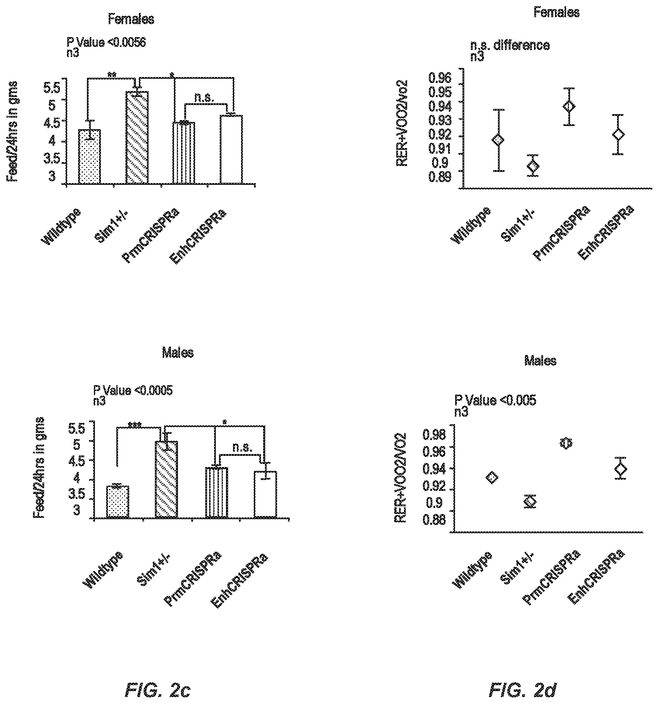

**Figs. 2A-D** **Body composition and metabolic analyses of *Sim1* CRISPRa transgenic mice. A,** Estimated percent fat in wild-type littermates, *Sim1*^{+/-}, *H11P^{CAG-dCas9-VP64}* X *ROSA26^{Sim1Pr-sgRNA}* (PrmCRISPRa) and *H11P^{CAG-dCas9-VP64} X ROSA26^{SCE2En-sgRNA}* (EnhCRISPRa) as determined by Dual Energy X-ray Absorptiometry (DEXA) or Echo Magnetic Resonance Imaging (EchoMRI), with their corresponding body weight measurements. The mean values±s.d. were obtained from 3 females and 3 males. **B,** Metabolic chamber energy expenditure analyses for 3 males and 3 females for all four genotypes determined over a 4 day period. **C,** Food intake for all four genotypes determined over a 4 day period. Mean values ± s.d. were obtained from 3 females and 3 males. * = p-value < 0.001; *** = p-value < 0.0005; n.s = non-significant (ANOVA, Tukey test). **D,** Respiratory exchange ratio (RER; VCO2/VO2) for all four genotypes obtained from 3 females and 3 males and plotted as mean values ± s.d.

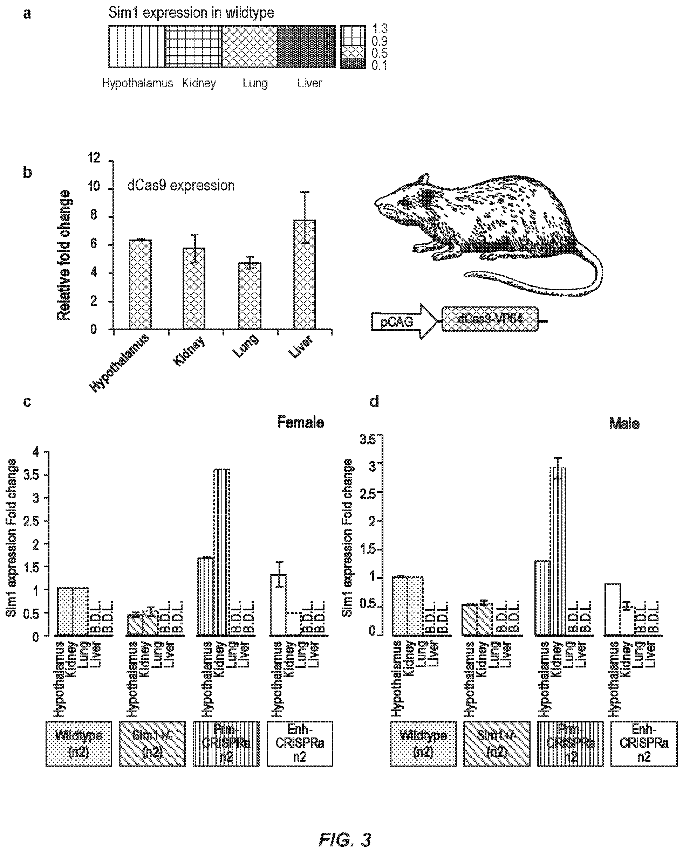

**Figs. 3A-D** ***dCas9* and *Sim1* mRNA expression levels in CRISPRa transgenic mice.** A, Heatmap of *Sim1* tissue expression. Red and grey filled squares signify tissues where *Sim1* is expressed and not expressed, respectively as determined in our wild-type mice. **B,** *dCas9* mRNA expression in the hypothalamus, kidney, lung and liver from 4 *Sim1*^{+/-} *X H11P^{CAG-dCas9- VP64}* mice. The mean values±s.d were determined based on mRNA fold-increase normalized to beta-actin (for hypothalamus) and Rpl38 (for kidney, lung, liver) using the ΔΔCT method. **C-D,** Sim1 mRNA expression in the hypothalamus, kidney, lung and liver for the following genotypes: wild-type littermates, *Sim1*^{+/-}, *H11P^{CAG-dCas9-VP64}* X *ROSA26^{Sim1Pr-sgRNA}* (PrmCRISPRa) and *H11P^{CAG-dCas9-VP64}* X *ROSA26^{SCE2En-sgRNA}* (Enh-CRISPRa) from 2 females **(C)** and 2 male **(D).** The mean values±s.d were determined based on mRNA fold-increase compared to wild-type littermates and normalized to beta-actin or Rpl38 using the ΔΔCT method. B.D.L = below detected levels.

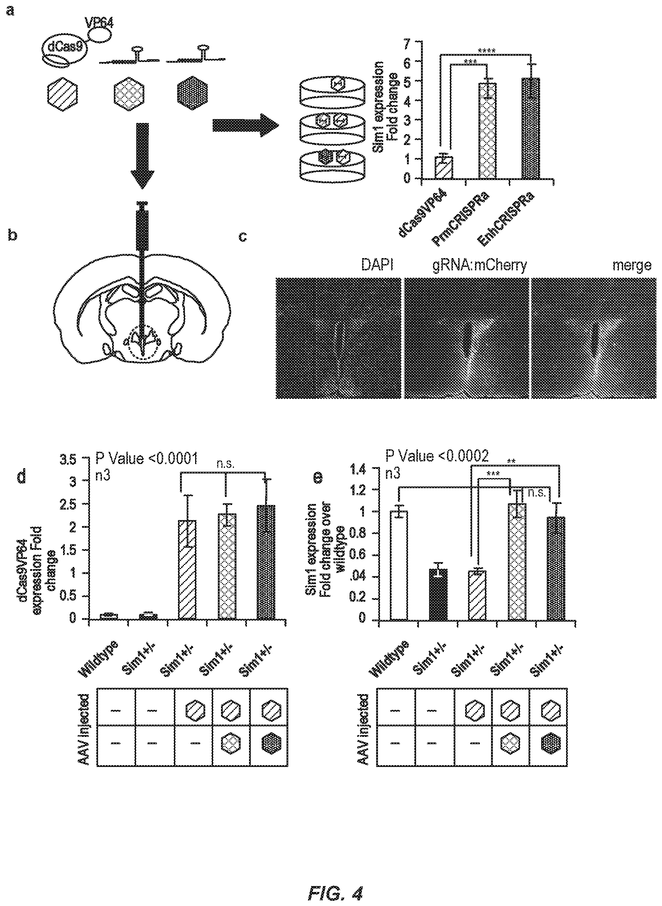

**Figs. 4A-E** **CRISPRa *Sim1* overexpression *in vitro* and *in vivo* using AAV. A,** AAV CRISPRa in Neuro-2A cells using virons containing: *pCMV-dCas9-VP64* (dCas9-VP64), *pCMV-dCas9-VP64* along with *pSim1Pr-mCherry* (PrmCRIPSRa) *and pCMV-dCas9-VP64* along with *pSCE2En-mCherry* (EnhCRISPRa). Results are expressed as mRNA fold-increase normalized to beta-actin using the ΔΔCT method. The mean values±s.d. were obtained from 3 independent experiments. *** = p-value < 0.0005 (ANOVA, Tukey test). **B,** Schema showing the PVN injected region. **C,** Immunohistochemistry of *pSim1Pr-mCherry* injected hypothalamus from 20 week old mice showing mCherry expression in the PVN. **D-E,** *Cas9* (d) and *Sim1* (e) mRNA expression from *pCMV-dCas9-VP64* (dCas9-VP64), *pCMV-dCas9-VP64 + pSim1Pr-mCherry* (PrmCRIPSRa, n=3) and *pCMV-dCas9-VP64 + pSCE2En-mCherry* (EnhCRISPRa, n=4) from injected mice. The mean values±s.d were determined based on mRNA fold-increase compared to *Sim1*^{+/-} mice and normalized to beta-actin using the ΔΔCT method.

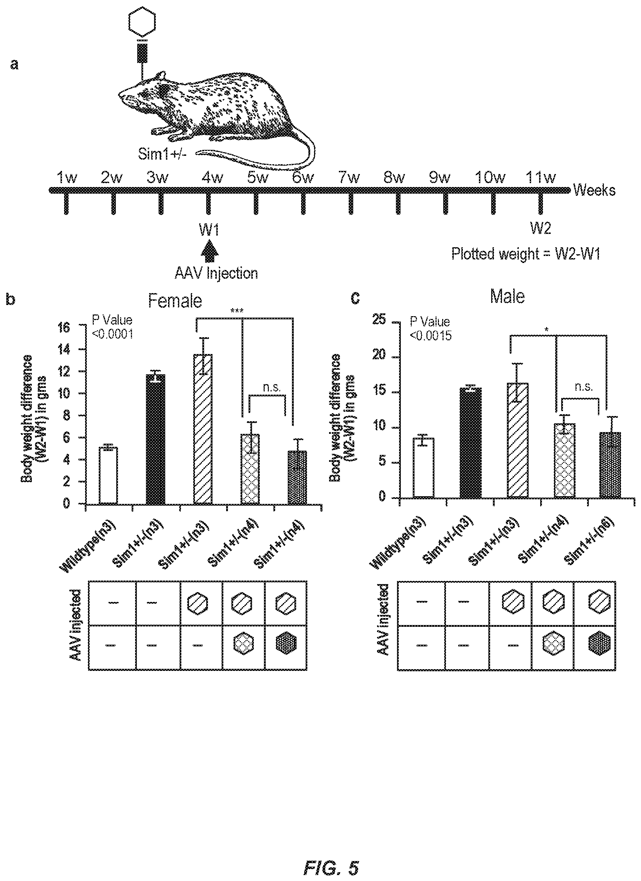

**Figs. 5A-C** **CRISPRa-AAV injection in PVN reduces weight gain in *Sim1*+/- mice. A,** Timeline for weight measurement post CRISPRa-AAV injection in PVN. **B-C,** Weight gain determined over a 7 week period from *Sim1*^{+/-} mice injected *with pCMV-dCas9-VP64* (dCas9-VP64), *pCMV-dCas9-VP64 + pSim1Pr-mCherry* (Prm-CRIPSRa) *pCMV-dCas9-VP64 + pSCE2En-mCherry* (Enh-CRISPRa) compared to un-injected wild-type littermates and *Sim1*^{+/-} mice. Mean values±s.d are shown from 3 females **(B)** and 3 males **(C).** * = p-value < 0.001 *** = p-value < 0.0005 n.s = non-significant; (ANOVA, Tukey test).

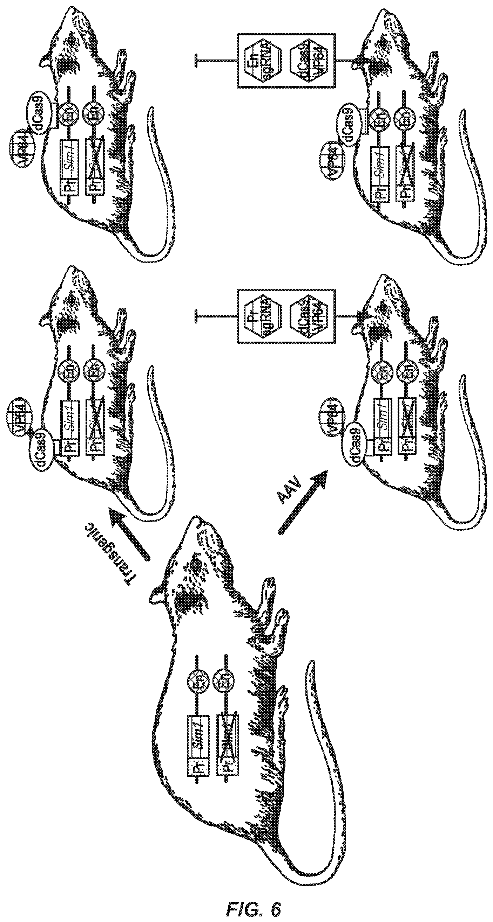

**Fig. 6** Schema of CRISPRa haploinsufficiency rescue experiments. The obesity phenotype in *Sim1*^{+/-} mice was rescued via CRISPRa by targeting either the *Sim1* promoter or enhancer using both a transgenic and postnatal AAV approach.

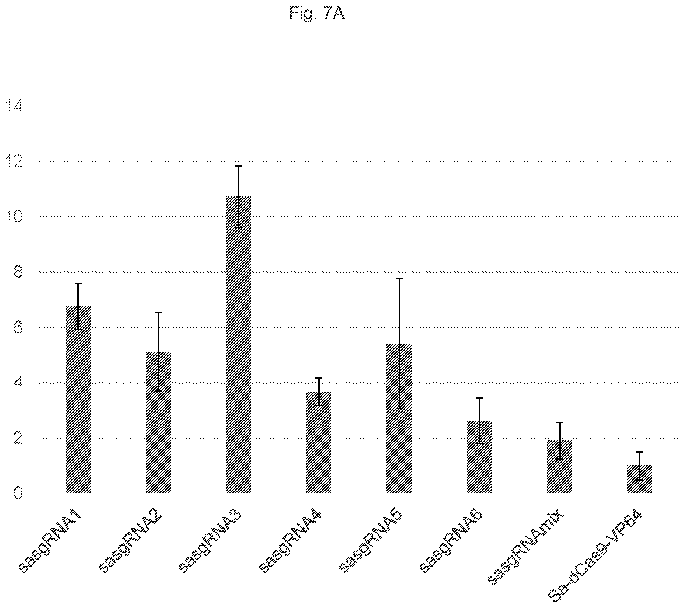

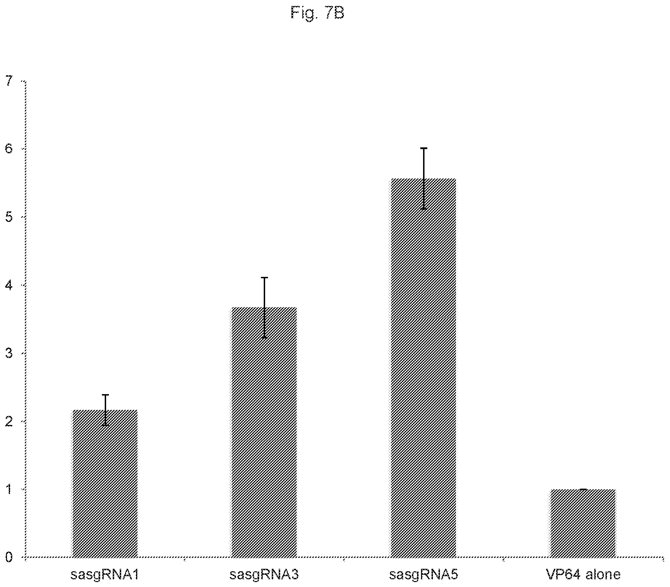

**Fig. 7A-7B****: CRISPRa *Sim1* overexpression *in vitro.*** **Fig. 7A****,** shows an exemplary S. *aureus* CRISPRa system targeting the *Sim1* promoter (Pr) by transfection of various sgRNA's (SEQ ID NOS:38-43) into Neuro-2A (N2A) cells. Results are expressed as mRNA fold-increase normalized to Sa-dCas9-VP64. The mean values±s.d. were obtained from 3 independent experiments. **Fig. 7B****,** shows an exemplary *S. aureus* CRISPRa in N2A cells targeting the *Sim1* promoter (Pr) after infection of AAV's containing select sgRNA's (SEQ ID NOS:38, 40, or 42) into N2A cells. Results are expressed as mRNA fold-increase normalized to VP64 alone. The mean values±s.d. were obtained from 3 independent experiments.

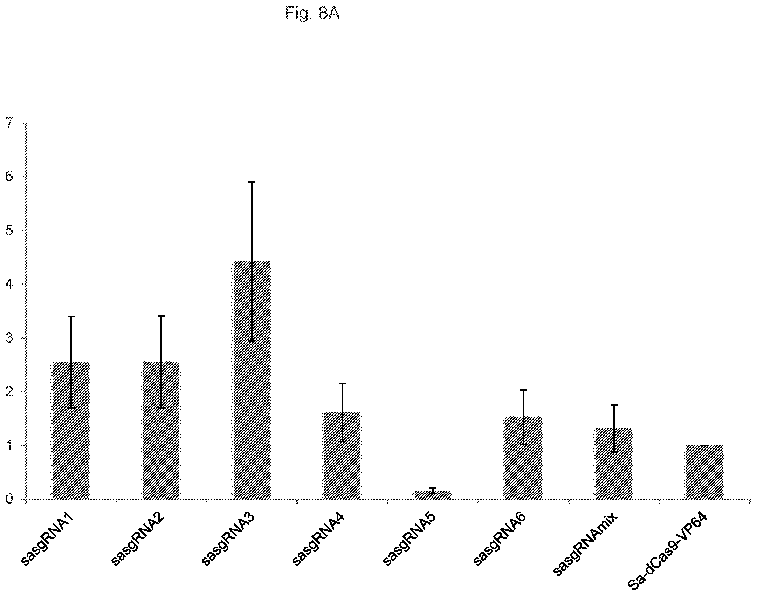

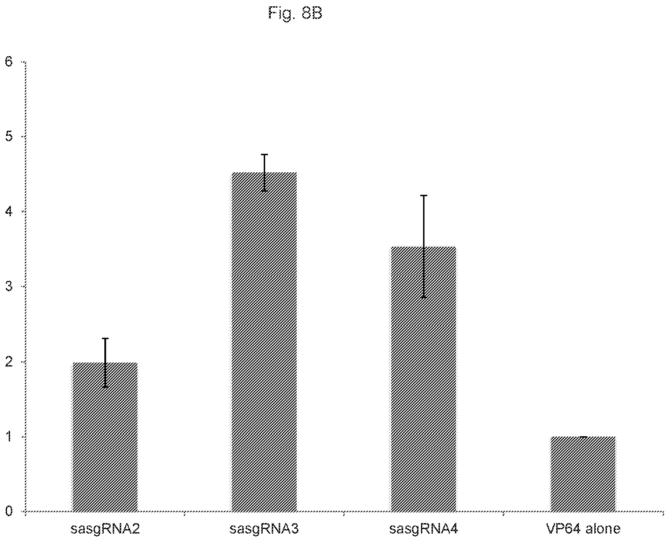

**Fig. 8A-8B****: CRISPRa *Sim1* overexpression *in vitro.*** **Fig. 8A****,** shows an exemplary S. *aureus* CRISPRa system targeting the *Sim1* SCE2 enhancer (Enh) by transfection of various sgRNA's (SEQ ID NOS:44-49) into N2A cells. Results are expressed as mRNA fold-increase normalized to Sa-dCas9-VP64. The mean values±s.d. were obtained from 3 independent experiments. **Fig. 8B****,** shows an exemplary *S. aureus* CRISPRa system targeting the *Sim1* SCE2 enhancer (Enh) after infection of AAV's containing select sgRNA's (SEQ ID NOS:45, 46, or 47) into N2A cells. Results are expressed as mRNA fold-increase normalized to VP64 alone. The mean values±s.d. were obtained from 3 independent experiments.

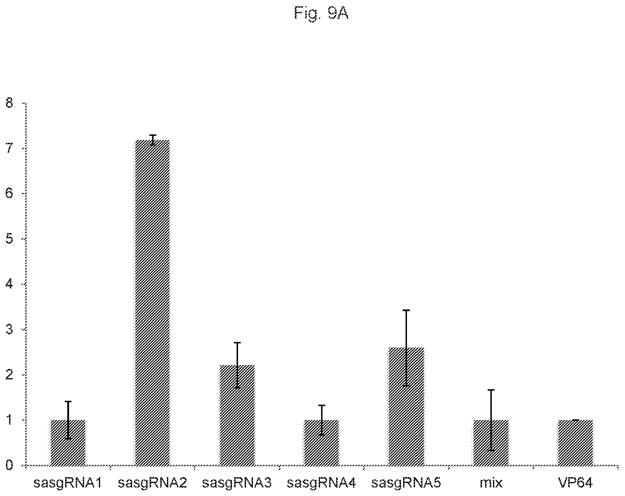

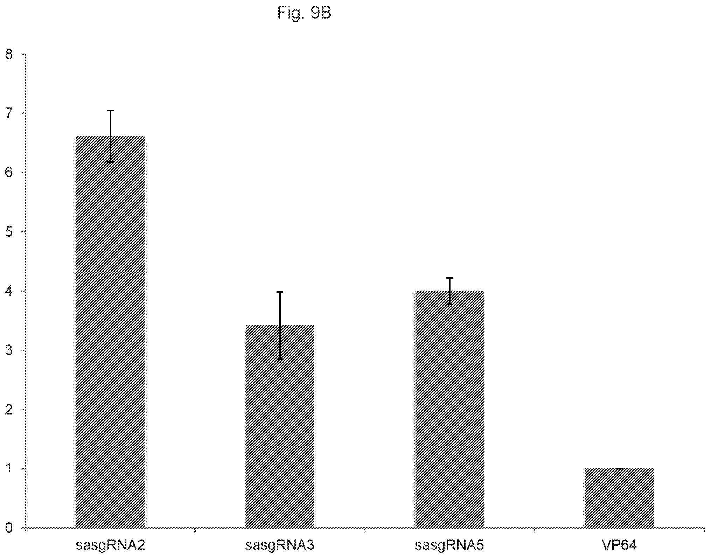

**Fig. 9A-9B****: CRISPRa *Mc4r* overexpression *in vitro.*** **Fig. 9A****,** shows an exemplary S. *aureus* CRISPRa system targeting the *Mc4r* promoter (Pr) by transfection of various sgRNA's (SEQ ID NOS:50-54) into N2A cells. Results are expressed as mRNA fold-increase normalized to VP64. The mean values±s.d. were obtained from 3 independent experiments. **Fig. 9B****,** shows an exemplary *S. aureus* CRISPRa system targeting the *Mc4r* promoter (Pr) after infection of AAV's containing select sgRNA's (SEQ ID NOS:51, 52, or 54) into N2A cells. Results are expressed as mRNA fold-increase normalized to VP64. The mean values±s.d. were obtained from 3 independent experiments.

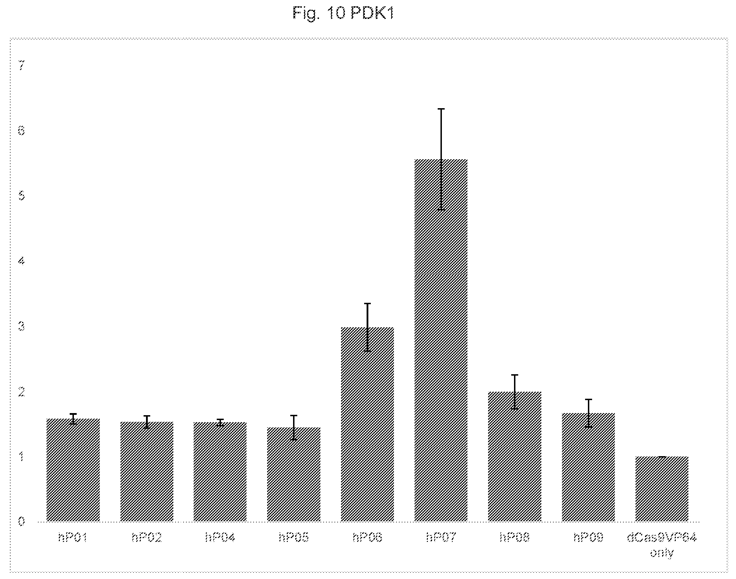

**Fig. 10****: CRISPRa *PKD1* overexpression *in vitro.*** An exemplary *S. aureus* CRISPRa system targeting the *PKD1* promoter (Pr) by transfection of human promoter sgRNA's (SEQ ID NOS:55-64) into human HEK293T cells. Results are expressed as mRNA fold-increase normalized to dCas9-VP64. The mean values±s.d. were obtained from 3 independent experiments.

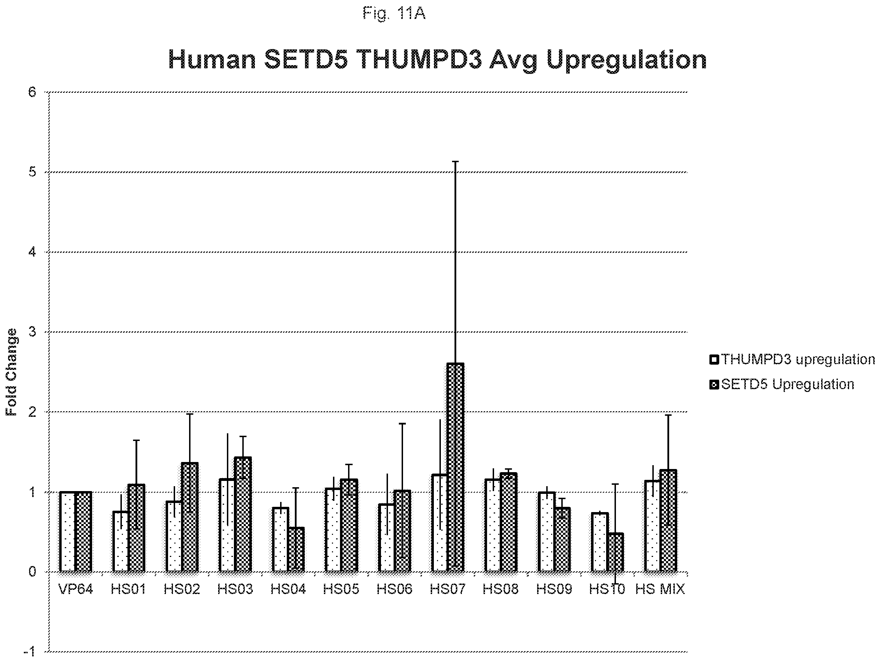

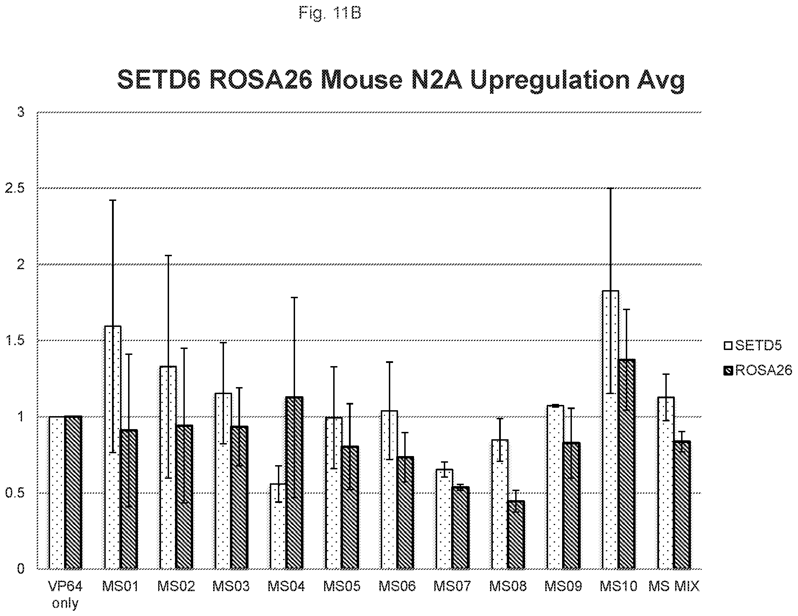

**Fig. 11A-11B****: CRISPRa *SETD5* overexpression *in vitro.*** **Fig. 11A****,** shows an exemplary *S. aureus* CRISPRa system targeting the *SETD5* promoter (Pr) or THUMPD3 by transfection of human promoter sgRNA's (SEQ ID NOS:65-74) into human HEK293T cells. HS MIX refers to transfection of an equimolar concentration of each of HS01-HS10 into human HEK293T cells. Results are expressed as mRNA fold-increase normalized to VP64 alone. The mean values±s.d. were obtained from 3 independent experiments. **Fig. 11B****,** shows an exemplary *S. aureus* CRISPRa system targeting the *SETD5* promoter (Pr) or ROSA26 by transfection of mouse promoter sgRNA's (SEQ ID NOS:75-84) into mouse Neuro-2A cells. MS MIX refers to transfection of an equimolar concentration of each of MS01-MS10 into mouse Neuro-2A cells. Results are expressed as mRNA fold-increase normalized to VP64 alone. The mean values±s.d. were obtained from 3 independent experiments.

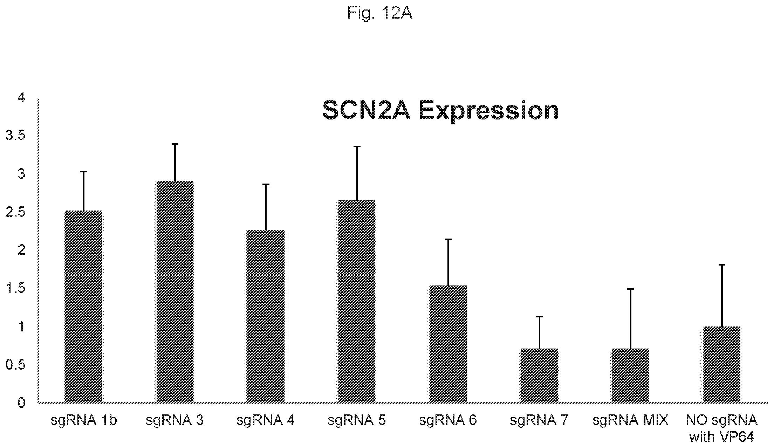

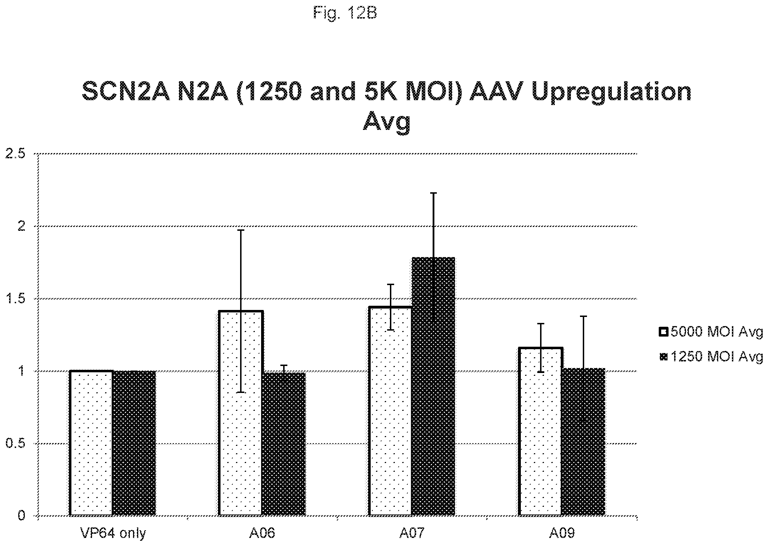

**Fig. 12A-12B****: CRISPRa *Scn2A* overexpression *in vitro.*** **Fig. 12A****,** shows an exemplary *S. pyogenes* (Sp) Cas9 CRISPRa system targeting the *Scn2a* promoter (Pr) by transfection of various sgRNA's (SEQ ID NOS:85-91) into N2A cells. Results are expressed as mRNA fold-increase normalized to VP64 alone. The mean values±s.d. were obtained from 3 independent experiments. **Fig. 12B****,** shows an exemplary *S. aureus* CRISPRa system targeting the *Scn2a* promoter (Pr) after infection of AAV's containing select sgRNA's (SEQ ID NOS:92-94) into N2A cells. Two different multiplicity of infection (MOI) were used: 5,000 and 1,250 viral genome (vg/ml). Results are expressed as mRNA fold-increase normalized to VP64 alone. The mean values±s.d. were obtained from 3 independent experiments.

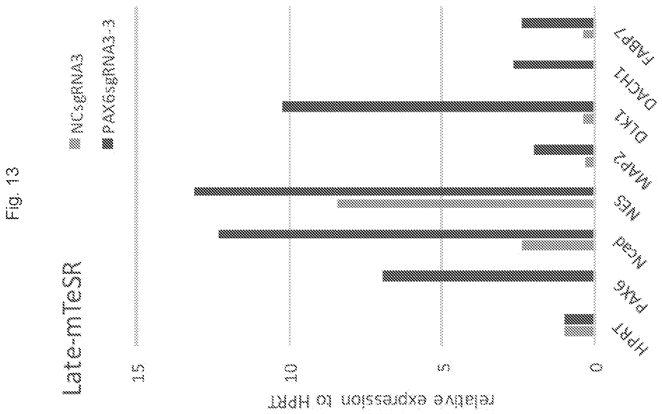

**Fig. 13****: CRISPRa *PAX6* overexpression *in vitro.*** shows an exemplary *S. pyogenes* (Sp) Cas9 CRISPRa system targeting the *PAX6* promoter (Pr) by lentiviral delivery of human promoter sgRNA (SEQ ID NO:95) into human H1-ESC cells differentiated into neurons. Results are expressed as relative expression to HPRT. The mean values±s.d. were obtained from 3 independent experiments. Additional neuronal markers are shown to demonstrate that *PAX6* CRISPRa leads to neural induction of H1-ESCs.

### DEFINITIONS

As used in this specification and the appended claims, the singular forms "a," "an," and "the" include plural reference unless the context clearly dictates otherwise.

"Treating" refers to any indicia of success in the treatment or amelioration or prevention of the disease, condition, or disorder, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters; including the results of an examination by a physician. Accordingly, the term "treating" includes the administration of the compounds or agents of the present disclosure to prevent or delay, to alleviate, or to arrest or inhibit development of the symptoms or conditions associated with a disease, condition or disorder as described herein. The term "therapeutic effect" refers to the reduction, elimination, or prevention of the disease, symptoms of the disease, or side effects of the disease in the subject. "Treating" or "treatment" using the methods of the present disclosure includes preventing the onset of symptoms in a subject that can be at increased risk of a disease or disorder associated with a disease, condition or disorder as described herein, but does not yet experience or exhibit symptoms, inhibiting the symptoms of a disease or disorder (slowing or arresting its development), providing relief from the symptoms or side-effects of a disease (including palliative treatment), and relieving the symptoms of a disease (causing regression). Treatment can be prophylactic (to prevent or delay the onset of the disease, or to prevent the manifestation of clinical or subclinical symptoms thereof) or therapeutic suppression or alleviation of symptoms after the manifestation of the disease or condition. The term "treatment," as used herein, includes preventative (e.g., prophylactic), curative or palliative treatment.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologues, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al, Mol. Cell. Probes 8:91-98 (1994)). The term nucleic acid is used interchangeably with gene, cDNA, and mRNA encoded by a gene.

The term "gene" means the segment of DNA involved in producing a polypeptide chain. It may include regions preceding and following the coding region (leader and trailer) as well as intervening sequences (introns) between individual coding segments (exons).

A "promoter" is defined as an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription.

An "expression cassette" is a nucleic acid construct, generated recombinantly or synthetically, with a series of specified nucleic acid elements that permit transcription of a particular polynucleotide sequence in a host cell. An expression cassette may be part of a plasmid, viral genome, or nucleic acid fragment. Typically, an expression cassette includes a polynucleotide to be transcribed, operably linked to a promoter.

A "reporter gene" encodes proteins that are readily detectable due to their biochemical characteristics, such as enzymatic activity or chemifluorescent features. One specific example of such a reporter is green fluorescent protein. Fluorescence generated from this protein can be detected with various commercially-available fluorescent detection systems. Other reporters can be detected by staining. The reporter can also be an enzyme that generates a detectable signal when contacted with an appropriate substrate. The reporter can be an enzyme that catalyzes the formation of a detectable product. Suitable enzymes include, but are not limited to, proteases, nucleases, lipases, phosphatases and hydrolases. The reporter can encode an enzyme whose substrates are substantially impermeable to eukaryotic plasma membranes, thus making it possible to tightly control signal formation. Specific examples of suitable reporter genes that encode enzymes include, but are not limited to, CAT (chloramphenicol acetyl transferase; Alton and Vapnek (1979) Nature 282: 864-869); luciferase (lux); β-galactosidase; LacZ; β. - glucuronidase; and alkaline phosphatase (Toh, et al. (1980) Eur. J. Biochem. 182: 231-238; and Hall et al. (1983) J. Mol. Appl. Gen. 2: 101). Other suitable reporters include those that encode for a particular epitope that can be detected with a labeled antibody that specifically recognizes the epitope.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g. , hydroxyproline, γ- carboxyglutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups {e.g., norleucine) or modified peptide backbones, but retain the same basic chemical structure as a naturally occurring amino acid. "Amino acid mimetics" refers to chemical compounds having a structure that is different from the general chemical structure of an amino acid, but that functions in a manner similar to a naturally occurring amino acid.

There are various known methods in the art that permit the incorporation of an unnatural amino acid derivative or analog into a polypeptide chain in a site-specific manner, see, e.g., WO 02/086075.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Polypeptide," "peptide," and "protein" are used interchangeably herein to refer to a polymer of amino acid residues. All three terms apply to amino acid polymers in which one or more amino acid residue is an artificial chemical mimetic of a corresponding naturally occurring amino acid, as well as to naturally occurring amino acid polymers and non- naturally occurring amino acid polymers. As used herein, the terms encompass amino acid chains of any length, including full-length proteins, wherein the amino acid residues are linked by covalent peptide bonds.

"Conservatively modified variants" applies to both amino acid and nucleic acid sequences. With respect to particular nucleic acid sequences, "conservatively modified variants" refers to those nucleic acids that encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. In some cases, conservatively modified variants of a CRISPR nuclease such as Cas9 or a guide RNA such as a small guide RNA (sgRNA) can have an increased stability, assembly, or activity as described in WO 2016/011080, including, without limitation, the sgRNAs, sgRNA scaffolds, sgRNA libraries, and sgRNA binding regions described therein.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(see, e.g., Creighton, Proteins, W. H. Freeman and Co., N. Y. (1984)).

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

In the present application, amino acid residues are numbered according to their relative positions from the left most residue, which is numbered 1 , in an unmodified wild- type polypeptide sequence.

As used in herein, the terms "identical" or percent "identity," in the context of describing two or more polynucleotide or amino acid sequences, refer to two or more sequences or subsequences that are the same or have a specified percentage of amino acid residues or nucleotides that are the same. For example, a core small guide RNA (sgRNA) sequence responsible for assembly and activity of a sgRNA :nuclease complex has at least 80% identity, preferably 85%, 90%, 91%, 92%, 93, 94%, 95%, 96%, 97%, 98%, 99%, or 100%) identity, to a reference sequence, when compared and aligned for maximum correspondence over a comparison window, or designated region as measured using one of the following sequence comparison algorithms or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. For sequence comparison of nucleic acids and proteins, the BLAST and BLAST 2.0 algorithms and the default parameters discussed below are used.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et al., eds. 1995 supplement)).

Examples of algorithms that are suitable for determining percent sequence identity and sequence similarity are the BLAST and BLAST 2.0 algorithms, which are described in Altschul et al, (1990) J. Mol. Biol. 215: 403-410 and Altschul et al. (1977) Nucleic Acids Res. 25: 3389-3402, respectively. Software for performing BLAST analyses is publicly available at the National Center for Biotechnology Information website, ncbi.nlm.nih.gov. The algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al, supra). These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=l, N=-2, and a comparison of both strands. For amino acid sequences, the BLASTP program uses as defaults a word size (W) of 3, an expectation (E) of 10, and the BLOSUM62 scoring matrix (see Henikoff & Henikoff, Proc. Natl. Acad. Sci. USA 89: 10915 (1989)).

The BLAST algorithm also performs a statistical analysis of the similarity between two sequences {see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence. Yet another indication that two polypeptides are substantially identical is that the two polypeptides retain identical or substantially similar activity.

A "translocation sequence" or "transduction sequence" refers to a peptide or protein (or active fragment or domain thereof) sequence that directs the movement of a protein from one cellular compartment to another, or from the extracellular space through the cell or plasma membrane into the cell. Translocation sequences that direct the movement of a protein from the extracellular space through the cell or plasma membrane into the cell are "cell penetration peptides." Translocation sequences that localize to the nucleus of a cell are termed "nuclear localization" sequences, signals, domains, peptides, or the like.

Examples of translocation sequences include, without limitation, the TAT transduction domain (see, e.g., S. Schwarze et al, Science 285 (Sep. 3, 1999); penetratins or penetratin peptides (D. Derossi et al, Trends in Cell Biol. 8, 84-87); Herpes simplex virus type 1 VP22 (A. Phelan et al., Nature Biotech. 16, 440-443 (1998), and polycationic (e.g., poly-arginine) peptides (Cell Mol. Life Sci. 62 (2005) 1839-1849). Further translocation sequences are known in the art. Translocation peptides can be fused (e.g. at the amino or carboxy terminus), conjugated, or coupled to a compound of the present disclosure, to, among other things, produce a conjugate compound that may easily pass into target cells, or through the blood brain barrier and into target cells.

As used herein, the term "CRISPR" refers to any one of the naturally occurring Clustered Regularly Interspaced Short Palindromic Repeat systems or loci, or a derivative thereof. CRISPR loci can be found in the genomes of many bacteria and archaea. There are four types of CRISPR systems (e.g., Type I, Type II, Type III, and Type U).

A CRISPR locus can comprise polynucleotide sequences encoding for CRISPR Associated Genes (Cas) genes. Cas genes can be involved in the biogenesis and/or the interference stages of crRNA function. Cas genes can be named according to the organism from which they are derived. For example, Cas genes in *Staphylococcus epidermidis* can be referred to as Csm-type, Cas genes in *Streptococcus thermophilus* can be referred to as Csn-type, and Cas genes in *Pyrococcus furiosus* can be referred to as Cmr-type.

As used herein, the term CRISPR nuclease refers to a polypeptide of, or derived from, a nuclease encoded in any one of the four types of CRISPR loci: Type I, Type II, Type III, and Type U, wherein the natural sequence of the polypeptide exhibits RNA-guided nuclease activity. A CRISPR nuclease can be catalytically inactive. Catalytically inactive CRISPR nucleases do not exhibit nuclease or nickase activity when in complex with an RNA-guide and bound to a nucleic acid target containing a target domain and, in certain embodiments, a PAM sequence. The catalytically inactive CRISPR nuclease can be catalytically inactive due to one or more mutations of the CRISPR nuclease polypeptide sequence, or due to forming a complex with a guide RNA that is sufficient to provide RNA-guided targeting, but insufficient to support catalytic activity (*i.e.,* nuclease or nicking activity). For example, the CRISPR nuclease can be a wild-type CRISPR nuclease (*e.g.,* a Cas9 or Cpfl nuclease) in complex with a dead guide sequence. For example, Cpfl is a Class II CRISPR-Cas system and is described in Zetsche et al., Cell, 163:759-771 (2015). Dead guide sequences and their use are further described in, *e.g.,* WO 2016/094872, including dead guide sequences, complexes between CRISPR nucleases and dead guide sequences, and methods and compositions for making and using such dead guide sequences and complexes containing them.

In certain embodiments, a CRISPR nuclease meets one or both of the following criteria: it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequences, e.g., a naturally occurring CRISPR nuclease. Additional CRISPR nucleases include, without limitation, one or more CRISPR nucleases described in WO 2016/154579.

In certain embodiments, a CRISPR nuclease contains (*i.e.,* is covalently or non-covalently linked to) one or more additional polypeptides or nucleic acids. For example, the CRISPR nuclease can be fused at an amino or carboxy-terminus to one or more transcriptional activation domain polypeptides, one or more DNA-binding polypeptides, one or more affinity tags (*e.g.,* in complex with one or more affinity tag ligands, such as affinity tag ligand-transcriptional activation domain fusion protein(s)), nuclear localization sequences, or a combination thereof.

Exemplary DNA-binding polypeptides include, but are not limited to, the programmable DNA binding domains described in Bolukbasi et al., Nature Methods 12, 1150-1156 (2015), including, e.g., the programmable DNA-binding domains (pDBD), Cas9 variants, and Cas9-pDBD chimeras described therein. Exemplary transcriptional activation domain polypeptides include, but are not limited to, an activation domain of, or combinations of activation domains of, one or more of the following:
- heat shock transcription factor 1 (HSF1), *e.g.,* SEQ ID NO:13 
(EKCLSVACLDKNELSDHLDAMDSNLDNLQTMLSSHGFSVDTSALLDLFSPSVTV
PDMSLPDLDSSLASIQELLSPQEPPRPPEAENSSPDSGKQLVHYTAQPLFLLDPGS
VDTGSNDLPVLFELGEGSYFSEGDGFAEDPTISLLTGSEPPKAKDPTVS)
- viral protein 16 (VP16), *e.g.,* SEQ ID NO:14 (DALDDFDLDML);
- tetrameric VP16 (VP64), *e.g.,* SEQ ID NO:15
   (DALDDFDLDMLGSDALDDFDLDMLGSDALDDFDLDMLGSDALDDFDLDML)
- the p65 NF-Kβ transactivating subunit (p65), *e.g.,* SEQ ID NO:16 
(SQYLPDTDDRHRIEEKRKRTYETFKSIMKKSPFSGPTDPRPPPRRIAVPSRSSASV
PKPAPQPYPFTSSLSTINYDEFPTMVFPSGQISQASALAPAPPQVLPQAPAPAPAPA
 
MVSALAQAPAPVPVLAPGPPQAVAPPAPKPTQAGEGTLSEALLQLQFDDEDLGA
LLGNSTDPAVFTDLASVDNSEFQQLLNQGIPVAPHTTEPMLMEYPEAITRLVTGA
QRPPDPAPAPLGAPGLPNGLLSGDEDFSSIADMDFSALL)
- MyoD1, e.g., SEQ ID NO:17 
(MELLSPPLRDIDLTGPDGSLCSFETADDFYDDPCFDSPDLRFFEDLDPRLVHMGA
LLKPEEHAHFPTAVHPGPGAREDEHVRAPSGHHQAGRCLLWACKACKRKTTNA
DRRKAATMRERRRLSKVNEAFETLKRCTSSNPNQRLPKVEILRNAIRYIEGLQAL
LRDQDAAPPGAAAFYAPGPLPPGRGSEHYSGDSDASSPRSNCSDGMMDYSGPPS
GPRRQNGYDTAYYSEAARESRPGKSAAVSSLDCLSSIVERISTDSPAAPALLLAD
APPESPPGPPEGASLSDTEQGTQTPSPDAAPQCPAGSNPNAIYQVL)
- RTA, *e.g.,* SEQ ID NO:18 
(RDSREGMFLPKPEAGSAISDVFEGREVCQPKRIRPFHPPGSPWANRPLPASLAPTP
TGPVHEPVGSLTPAPVPQPLDPAPAVTPEASHLLEDPDEETSQAVKALREMADTV
IPQKEEAAICGQMDLSHPPPRGHLDELTTTLESMTEDLNLDSPLTPELNEILDTFLN
DECLLHAMHISTGLSIFDTSLF)
- SET7, e.g., SEQ ID NO:19 
(MDSDDEMVEEAVEGHLDDDGLPHGFCTVTYSSTDRFEGNFVHGEKNGRGKFFF
FDGSTLEGYYVDDALQGQGVYTYEDGGVLQGTYVDGELNGPAQEYDTDGRLIF
KGQYKDNIRHGVCWIYYPDGGSLVGEVNEDGEMTGEKIAYVYPDERTALYGKFI
DGEMIEGKLATLMSTEEGRPHFELMPGNSVYHFDKSTSSCISTNALLPDPYESER
VYVAESLISSAGEGLFSKVAVGPNTVMSFYNGVRITHQEVDSRDWALNGNTLSL
DEETVIDVPEPYNHVSKYCASLGHKANHSFTPNCIYDMFVHPRFGPIKCIRTLRA
VEADEELTVAYGYDHSPPGKSGPEAPEWYQVELKAFQATQQK)
- VPR, *e.g.,* SEQ ID NO:20 
(EASGSGRADALDDFDLDMLGSDALDDFDLDMLGSDALDDFDLDMLGSDALDD
FDLDMLINSRSSGSPKKKRKVGSQYLPDTDDRHRIEEKRKRTYETFKSIMKKSPFS
GPTDPRPPPRRIAVPSRSSASVPKPAPQPYPFTSSLSTINYDEFPTMVFPSGQISQAS
ALAPAPPQVLPQAPAPAPAPAMVSALAQAPAPVPVLAPGPPQAVAPPAPKPTQA
GEGTLSEALLQLQFDDEDLGALLGNSTDPAVFTDLASVDNSEFQQLLNQGIPVAP
 
HTTEPMLMEYPEAITRLVTGAQRPPDPAPAPLGAPGLPNGLLSGDEDFSSIADMD
FSALLGSGSGSRDSREGMFLPKPEAGSAISDVFEGREVCQPKRIRPFHPPGSPWAN
RPLPASLAPTPTGPVHEPVGSLTPAPVPQPLDPAPAVTPEASHLLEDPDEETSQAV
KALREMADTVIPQKEEAAICGQMDLSHPPPRGHLDELTTTLESMTEDLNLDSPLT
PELNEILDTFLNDECLLHAMHISTGLSIFDTSLF)
- histone acetyltransferase p300, *e.g.,* SEQ ID NO:21 
(KFSAKRLPSTRLGTFLENRVNDFLRRQNHPESGEVTVRVVHASDKTVEVKPGM
KARFVDSGEMAESFPYRTKALFAFEEIDGVDLCFFGMHVQEYGSDCPPPNQRRV
YISYLDSVHFFRPKCLRTAVYHEILIGYLEYVKKLGYTTGHIWACPPSEGDDYIFH
CHPPDQKIPKPKRLQEWYKKMLDKAVSERIVHDYKDIFKQATEDRLTSAKELPY
FEGDFWPNVLEESIKELEQEEEERKREENTSNESTDVTKGDSKNAKKKNNKKTS
KNKSSLSRGNKKKPGMPNVSNDLSQKLYATMEKHKEVFFVIRLIAGPAANSLPPI
VDPDPLIPCDLMDGRDAFLTLARDKHLEFSSLRRAQWSTMCMLVELHTQSQ)
- an hydroxylase catalytic domain of a TET family protein *(e.g.,* TET1 hydroxylase catalytic domain), e.g., SEQ ID NO:22 
(MSRSRHARPSRLVRKEDVNKKKKNSQLRKTTKGANKNVASVKTLSPGKLKQLI
QERDVKKKTEPKPPVPVRSLLTRAGAARMNLDRTEVLFQNPESLTCNGFTMALR
STSLSRRLSQPPLVVAKSKKVPLSKGLEKQHDCDYKILPALGVKHSENDSVPMQ
DTQVLPDIETLIGVQNPSLLKGKSQETTQFWSQRVEDSKINIPTHSGPAAEILPGPL
EGTRCGEGLFSEETLNDTSGSPKMFAQDTVCAPFPQRATPKVTSQGNPSIQLEEL
GSRVESLKLSDSYLDPIKSEHDCYPTSSLNKVIPDLNLRNCLALGGSTSPTSVIKFL
LAGSKQATLGAKPDHQEAFEATANQQEVSDTTSFLGQAFGAIPHQWELPGADPV
HGEALGETPDLPEIPGAIPVQGEVFGTILDQQETLGMSGSVVPDLPVFLPVPPNPIA
TFNAPSKWPEPQSTVSYGLAVQGAIQILPLGSGHTPQSSSNSEKNSLPPVMAISNV
ENEKQVHISFLPANTQGFPLAPERGLFHASLGIAQLSQAGPSKSDRGSSQVSVTST
VHVVNTTVVTMPVPMVSTSSSSYTTLLPTLEKKKRKRCGVCEPCQQKTNCGECT
YCKNRKNSHQICKKRKCEELKKKPSVVVPLEVIKENKRPQREKKPKVLKADFDN
KPVNGPKSESMDYSRCGHGEEQKLELNPHTVENVTKNEDSMTGIEVEKWTQNK
KSQLTDHVKGDFSANVPEAEKSKNSEVDKKRTKSPKLFVQTVRNGIKHVHCLPA
ETNVSFKKFNIEEFGKTLENNSYKFLKDTANHKNAMSSVATDMSCDHLKGRSNV
 
LVFQQPGFNCSSIPHSSHSIINHHASIHNEGDQPKTPENIPSKEPKDGSPVQPSLLSL
MKDRRLTLEQVVAIEALTQLSEAPSENSSPSKSEKDEESEQRTASLLNSCKAILYT
VRKDLQDPNLQGEPPKLNHCPSLEKQSSCNTVVFNGQTTTLSNSHINSATNQAST
KSHEYSKVTNSLSLFIPKSNSSKIDTNKSIAQGIITLDNCSNDLHQLPPRNNEVEYC
NQLLDSSKKLDSDDLSCQDATHTQIEEDVATQLTQLASIIKINYIKPEDKKVESTP
TSLVTCNVQQKYNQEKGTIQQKPPSSVHNNHGSSLTKQKNPTQKKTKSTPSRDR
RKKKPTVVSYQENDRQKWEKLSYMYGTICDIWIASKFQNFGQFCPHDFPTVFGK
ISSSTKIWKPLAQTRSIMQPKTVFPPLTQIKLQRYPESAEEKVKVEPLDSLSLFHLK
TESNGKAFTDKAYNSQVQLTVNANQKAHPLTQPSSPPNQCANVMAGDDQIRFQ
QVVKEQLMHQRLPTLPGISHETPLPESALTLRNVNVVCSGGITVVSTKSEEEVCSS
SFGTSEFSTVDSAQKNFNDYAMNFFTNPTKNLVSITKDSELPTCSCLDRVIQKDK
GPYYTHLGAGPSVAAVREIMENRYGQKGNAIRIEIVVYTGKEGKSSHGCPIAKW
VLRRSSDEEKVLCLVRQRTGHHCPTAVMVVLIMVWDGIPLPMADRLYTELTENL
KSYNGHPTDRRCTLNENRTCTCQGIDPETCGASFSFGCSWSMYFNGCKFGRSPSP
RRFRIDPSSPLHEKNLEDNLQSLATRLAPIYKQYAPVAYQNQVEYENVARECRLG
SKEGRPFSGVTACLDFCAHPHRDIHNMNNGSTVVCTLTREDNRSLGVIPQDEQL
HVLPLYKLSDTDEFGSKEGMEAKIKSGAIEVLAPRRKKRTCFTQPVPRSGKKRAA
MMTEVLAHKIRAVEKKPIPRIKRKNNSTTTNNSKPSSLPTLGSNTETVQPEVKSET
EPHFILKSSDNTKTYSLMPSAPHPVKEASPGFSWSPKTASATPAPLKNDATASCGF
SERSSTPHCTMPSGRLSGANAAAADGPGISQLGEVAPLPTLSAPVMEPLINSEPST
GVTEPLTPHQPNHQPSFLTSPQDLASSPMEEDEQHSEADEPPSDEPLSDDPLSPAE
EKLPHIDEYWSDSEHIFLDANIGGVAIAPAHGSVLIECARRELHATTPVEHPNRNH
PTRLSLVFYQHKNLNKPQHGFELNKIKFEAKEAKNKKMKASEQKDQAANEGPE
QSSEVNELNQIPSHKALTLTHDNVVTVSPYALTHVAGPYNHWV)
- LSD1, *e.g.,* SEQ ID NO:23 
(GMDVTLLEARDRVGGRVATFRKGNYVADLGAMVVTGLGGNPMAVVSKQVN
MELAKIKQKCPLYEANGQAVPKEKDEMVEQEFNRLLEATSYLSHQLDFNVLNN
KPVSLGQALEVVIQLQEKHVKDEQIEHWKKIVKTQEELKELLNKMVNLKEKIKE
LHQQYKEASEVKPPRDITAEFLVKSKHRDLTALCKEYDELAETQGKLEEKLQELE
ANPPSDVYLSSRDRQILDWHFANLEFANATPLSTLSLKHWDQDDDFEFTGSHLT
VRNGYSCVPVALAEGLDIKLNTAVRQVRYTASGCEVIAVNTRSTSQTFIYKCDA
 
VLCTLPLGVLKQQPPAVQFVPPLPEWKTSAVQRMGFGNLNKVVLCFDRVFWDP
SVNLFGHVGSTTASRGELFLFWNLYKAPILLALVAGEAAGIMENISDDVIVGRCL
AILKGIFGSSAVPQPKETVVSRWRADPWARGSYSYVAAGSSGNDYDLMAQPITP
GPSIPGAPQPIPRLFFAGEHTIRNYPATVHGALLSGLREAGRIADQFLGAMYTLPR
QATPGVPAQQSPSM)
- CIB1, *e.g.,* SEQ ID NO:24 
(MGGSGSRLSKELLAEYQDLTFLTKQEILLAHRRFCELLPQEQRSVESSLRAQVPF
EQILSLPELKANPFKERICRVFSTSPAKDSLSFEDFLDLLSVFSDTATPDIKSHYAFR
IFDFDDDGTLNREDLSRLVNCLTGEGEDTRLSASEMKQLIDNILEESDIDRDGTIN
LSEFQHVISRSPDFASSFKIVL)
- AD2, *e.g.,* SEQ ID NO:25 
(MNQPQRMAPVGTDKELSDLLDFSMMFPLPVTNGKGRPASLAGAQFGGSGLED
RPSSGSWGSGDQSSSSFDPSRTFSEGTHFTESHSSLSSSTFLGPGLGGKSGERGAY
ASFGRDAGVGGLTQAGFLSGELALNSPGPLSPSGMKGTSQYYPSYSGSSRRRAA
DGSLDTQPKKVRKVPPGLPSSVYPPSSGEDYGRDATAYPSAKTPSSTYPAPFYVA
DGSLHPSAELWSPPGQAGFGPMLGGGSSPLPLPPGSGPVGSSGSSSTFGGLHQHE
RMGYQLHGAEVNGGLPSASSFSSAPGATYGGVSSHTPPVSGADSLLGSRGTTAG
SSGDALGKALASIYSPDHSSNNFSSSPSTPVGSPQGLAGTSQWPRAGAPGALSPSY
DGGLHGLQSKIEDHLDEAIHVLRSHAVGTAGDMHTLLPGHGALASGFTGPMSLG
GRHAGLVGGSHPEDGLAGSTSLMHNHAALPSQPGTLPDLSRPPDSYSGLGRAGA
TAAASEIKREEKEDEENTSAADHSEEEKKELKAPRARTSPDEDEDDLLPPEQKAE
REKERRVANNARERLRVRDINEAFKELGRMCQLHLNSEKPQTKLLILHQAVSVIL
NLEQQVRERNLNPKAACLKRREEEKVSGVVGDPQMVLSAPHPGLSEAHNPAGH
M)
- CR3, *e.g.,* SEQ ID NO:26 
(




 

























)
- GATA4, *e.g.,* SEQ ID NO:27 
(MYQSLAMAANHGPPPGAYEAGGPGAFMHGAGAASSPVYVPTPRVPSSVLGLS
YLQGGGAGSASGGASGGSSGGAASGAGPGTQQGSPGWSQAGADGAAYTPPPVS
PRFSFPGTTGSLAAAAAAAAREAAAYSSGGGAAGAGLAGREQYGRAGFAGSY
SSPYPAYMADVGASWAAAAAASAGPFDSPVLHSLPGRANPAARHPNLDMFDDF
 
SEGRECVNCGAMSTPLWRRDGTGHYLCNACGLYHKMNGINRPLIKPQRRLSAS
RRVGLSCANCQTTTTTLWRRNAEGEPVCNACGLYMKLHGVPRPLAMRKEGIQT
RKRKPKNLNKSKTPAAPSGSESLPPASGASSNSSNATTSSSEEMRPIKTEPGLSSH
YGHSSSVSQTFSVSAMSGHGPSIHPVLSALKLSPQGYASPVSQSPQTSSKQDSWN
SLVLADSHGDIITA)
- p53, *e.g.,* SEQ ID NO:28 
(






)
- SP1, e.g., SEQ ID NO:29 
(MSDQDHSMDEMTAVVKIEKGVGGNNGGNGNGGGAFSQARSSSTGSSSSTGGG
GQESQPSPLALLAATCSRIESPNENSNNSQGPSQSGGTGELDLTATQLSQGANGW
QIISSSSGATPTSKEQSGSSTNGSNGSESSKNRTVSGGQYVVAAAPNLQNQQVLT
GLPGVMPNIQYQVIPQFQTVDGQQLQFAATGAQVQQDGSGQIQIIPGANQQIITN
RGSGGNIIAAMPNLLQQAVPLQGLANNVLSGQTQYVTNVPVALNGNITLLPVNS
VSAATLTPSSQAVTISSSGSQESGSQPVTSGTTISSASLVSSQASSSSFFTNANSYST
TTTTSNMGIMNFTTSGSSGTNSQGQTPQRVSGLQGSDALNIQQNQTSGGSLQAG
QQKEGEQNQQTQQQQILIQPQLVQGGQALQALQAAPLSGQTFTTQAISQETLQN
LQLQAVPNSGPIIIRTPTVGPNGQVSWQTLQLQNLQVQNPQAQTITLAPMQGVSL
GQTSSSNTTLTPIASAASIPAGTVTVNAAQLSSMPGLQTINLSALGTSGIQVHPIQG
LPLAIANAPGDHGAQLGLHGAGGDGIHDDTAGGEEGENSPDAQPQAGRRTRRE
ACTCPYCKDSEGRGSGDPGKKKQHICHIQGCGKVYGKTSHLRAHLRWHTGERP
FMCTWSYCGKRFTRSDELQRHKRTHTGEKKFACPECPKRFMRSDHLSKHIKTHQ
NKKGGPGVALSVGTLPLDSGAGSEGSGTATPSALITTNMVAMEAICPEGIARLAN
SGINVMQVADLQSINISGNGF)
- MEF2C, *e.g.,* SEQ ID NO:30 
(MGRKKIQITRIMDERNRQVTFTKRKFGLMKKAYELSVLCDCEIALIIFNSTNKLF
QYASTDMDKVLLKYTEYNEPHESRTNSDIVETLRKKGLNGCDSPDPADDSVGH
SPESEDKYRKINEDIDLMISRQRLCAVPPPNFEMPVSIPVSSHNSLVYSNPVSSLGN
PNLLPLAHPSLQRNSMSPGVTHRPPSAGNTGGLMGGDLTSGAGTSAGNGYGNPR
NSPGLLVSPGNLNKNMQAKSPPPMNLGMNNRKPDLRVLIPPGSKNTMPSVSEDV
DLLLNQRINNSQSAQSLATPVVSVATPTLPGQGMGGYPSAISTTYGTEYSLSSAD
LSSLSGFNTASALHLGSVTGWQQQHLHNMPPSALSQLGACTSTHLSQSSNLSLPS
TQSLNIKSEPVSPPRDRTTTPSRYPQHTRHEAGRSPVDSLSSCSSSYDGSDREDHR
NEFHSPIGLTRPSPDERESPSVKRMRLSEGWAT)
- TAX, *e.g.,* SEQ ID NO:31 
(MAHFPGFGQSLLFGYPVYVFGDCVQGDWCPISGGLCSARLHRHALLATCPEHQI
TWDPIDGRVIGSALQFLIPRLPSFPTQRTSKTLKVLTPPITHTTPNIPPSFLQAMRKY
SPFRNGYMEPTLGQHLPTLSFPDPGLRPQNLYTLWGGSVVCMYLYQLSPPITWPL
LPHVIFCHPGQLGAFLTNVPYKRIEELLYKISLTTGALIILPEDCLPTTLFQPARAPV
TLTAWQNGLLPFHSTLTTPGLIWTFTDGTPMISGPCPKDGQPSLVLQSSSFIFHKF
QTKAYHPSFLLSHGLIQYSSFHSLHLLFEEYTNIPISLLFNEKEADDNDHEPQISPG
GLEPPSEKHFRETEV)
- PPARγ, *e.g.,* SEQ ID NO:32 
(








)
 or
- SET9, *e.g.,* SEQ ID NO:33 
(MDSDDEMVEEAVEGHLDDDGLPHGFCTVTYSSTDRFEGNFVHGEKNGRGKFFF
FDGSTLEGYYVDDALQGQGVYTYEDGGVLQGTYVDGELNGPAQEYDTDGRLIF
KGQYKDNIRHGVCWIYYPDGGSLVGEVNEDGEMTGEKIAYVYPDERTALYGKFI
DGEMIEGKLATLMSTEEGRPHFELMPGNSVYHFDKSTSSCISTNALLPDPYESER
VYVAESLISSAGEGLFSKVAVGPNTVMSFYNGVRITHQEVDSRDWALNGNTLSL
DEETVIDVPEPYNHVSKYCASLGHKANHSFTPNCIYDMFVHPRFGPIKCIRTLRA
VEADEELTVAYGYDHSPPGKSGPEAPEWYQVELKAFQATQQK),
 or
one or more of the transcriptional activation domains described in Chavez et al., Nat Methods. 2015 Apr; 12(4): 326-328, including activation domain polypeptides and encoding polynucleotides, Cas9 *(e.g.,* dCas9) polypeptides and encoding polynucleotides, and fusion proteins, and complexes (e.g., with sgRNA) thereof.

In some cases, the CRISPR nuclease is fused to one or more affinity tags. For example, the CRISPR nuclease may be a component of a SunTag. Exemplary SunTags or SunTag components include, without limitation, one or more of the affinity tagged CRISPR nucleases or affinity tag ligands, and fusion proteins thereof, described in WO 2016/011070. In one embodiment, the CRISPR nuclease contains one or more affinity tags that are non-covalently bound to one or more ligand-transcriptional activation domain fusion proteins. In such embodiments, the transcriptional activation domain fused to the affinity tag ligand can be, *e.g.,* one or more of the transcriptional activation domains described herein, such as those of SEQ ID NOs:13-33, a transcriptional activation domain described in WO 2016/011070, or a combination or derivative thereof.

As used herein, the terms "Cas9," "Cas9 molecule," and the like, refers to a Cas9 polypeptide or a nucleic acid encoding a Cas9 polypeptide. A "Cas9 polypeptide" is a polypeptide that can form a complex with a guide RNA (gRNA) and bind to a nucleic acid target containing a target domain and, in certain embodiments, a PAM sequence. Cas9 molecules include those having a naturally occurring Cas9 polypeptide sequence and engineered, altered, or modified Cas9 polypeptides that differ, *e.g.,* by at least one amino acid residue, from a reference sequence, *e.g.,* the most similar naturally occurring Cas9 molecule. A Cas9 molecule may be a Cas9 polypeptide or a nucleic acid encoding a Cas9 polypeptide. A Cas9 molecule may be a nuclease (an enzyme that cleaves both strands of a double-stranded nucleic acid), a nickase (an enzyme that cleaves one strand of a double-stranded nucleic acid), or a catalytically inactive (or dead) Cas9 molecule. A Cas9 molecule having nuclease or nickase activity is referred to as a "catalytically active Cas9 molecule" (a "caCas9" molecule). A Cas9 molecule lacking the ability to cleave or nick target nucleic acid is referred to as a "catalytically inactive Cas9 molecule" (a "ciCas9" molecule) or a "dead Cas9" ("dCas9").

In certain embodiments, a Cas9 molecule meets one or both of the following criteria: it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequence, e.g., a naturally occurring Cas9 molecule.

In some embodiments, the Cas9 molecule is *a S. pyogenes* Cas9 (SpCas9) or variant thereof. In some embodiments, the Cas9 molecule is a *S. aureus* Cas9 (SaCas9) or variant thereof (see, *e.g.,* FIGS 7A-11B herein). In some embodiments, the Cas9 molecule is a *Campylobacter jejuni* Cas9 (CjCas9) or variant thereof (see, *Kim et al*., *Nat. Comm.,* 8, 14500 (2017). In some embodiments, the Cas9 molecule is a *Neisseria meningitides* Cas9 (NmCas9) or variant thereof (see, US Patent No.: 9,074,199). In some embodiments, the Cas9 molecule is a Streptococcus *thermophilus* Cas9 (StCas9) or variant thereof (see, *e.g.,* Xu et al., Cell Mol Life Sci., 72:383-99 (2014)). In some embodiments, the Cas9 molecule is a dCas9 molecule.

In certain embodiments, the Cas9 molecule is *a S. pyogenes* Cas9 variant. In certain embodiments, the Cas9 variant is the EQR variant. In certain embodiments, the Cas9 variant is the VRER variant. In certain embodiments, the dCas9 molecule is *a S. pyogenes* Cas9 variant. In certain embodiments, the Cas9 variant is the EQR variant. In certain embodiments, the Cas9 variant is the VRER variant. In certain embodiments, a Cas9 system comprises a Cas9 molecule, *e.g.,* a Cas9 molecule described herein, e.g., the Cas9 EQR variant or the Cas9 VRER variant.

In certain embodiments, the Cas9 molecule is a S. *aureus* Cas9 variant. In certain embodiments, the Cas9 variant is the KKH (E782K/N968K/R1015H) variant (see, e.g., Kleinstiver et al., Nature 523, 481-485 (23 July 2015); and Leenay et al. Molecular Cell, Vol. 62, Issue 1, 2016, p. 137), especially with regard to Cas *(e.g.,* Cas9) variants such as those having altered PAM specificities). In certain embodiments, the Cas9 variant is the E782K/K929R/R1015H variant (see, *e.g.,* Kleinstiver 2015). In certain embodiments, the Cas9 variant is the E782K/K929R/N968K/R1015H variant (see, *e.g.,* Kleinstiver 2015). In certain embodiments the Cas9 variant comprises one or more mutations in one of the following residues: E782, K929, N968, R1015. In certain embodiments the Cas9 variant comprises one or more of the following mutations: E782K, K929R, N968K, R1015H and R1015Q (see, e.g., Kleinstiver 2015). In certain embodiments, a Cas9 system comprises a Cas9 molecule, *e.g.,* a Cas9 molecule described herein, *e.g.,* the Cas9 KKH variant.

As used herein, the terms "Cpfl," "Cpfl molecule," and the like, refers to a Cpfl polypeptide or a nucleic acid encoding a Cpf1 polypeptide. A "Cpfl polypeptide" is a polypeptide that can form a complex with a guide RNA (gRNA) and bind to a nucleic acid target containing a target domain and, in certain embodiments, a PAM sequence. Cpfl molecules include those having a naturally occurring Cpf1 polypeptide sequence and engineered, altered, or modified Cpfl polypeptides that differ, *e.g.,* by at least one amino acid residue, from a reference sequence, e.g., the most similar naturally occurring Cpfl molecule. A Cpfl molecule may be a Cpfl polypeptide or a nucleic acid encoding a Cpf1 polypeptide. Examplary Cpfl polypeptides include those isolated from *Prevotella, Francisella* novicida (FnCpf1), *Lachnospiraceae bacterium* (LbCpf1) and *Acidaminococcus sp. (AsCpf1) (see, e.g.,* Tóth et al., Biology Direct, 11:46 (2016).

In certain embodiments, a Cpf1 molecule meets one or both of the following criteria: it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequence, e.g., a naturally occurring Cpf1 molecule.

As used herein, the term "gRNA molecule" or "gRNA" refers to a guide RNA which is capable of targeting a CRISPR nuclease to a target nucleic acid. In one embodiment, the term "gRNA molecule" refers to a guide ribonucleic acid. In another embodiment, the term "gRNA molecule" refers to a nucleic acid encoding a gRNA. In one embodiment, a gRNA molecule is non-naturally occurring. In one embodiment, a gRNA molecule is a synthetic gRNA molecule.

The guide RNA can be a scaffold RNA that binds to one or more protein or nucleic acid ligands (scaffold RNA ligands). The ligands can be fused or otherwise covalently or non-covalently linked to transcriptional activation domains. In an alternative aspect, the scaffold RNA is not a guide RNA in that it does not specifically associate with a CRISPR nuclease. Exemplary scaffold RNAs, and CRISPR nuclease/scaffold RNA complexes, and methods of making and using such, are described in, *e.g.,* WO 2016/054106 (describing CRISPR-associating and CRISPR independent scaffold RNAs) and Zhang et al., Scientific Reports 5, Article No. 16277 (2015); Konermann et al., 2015, Nature 517:583-8 (describing CRISPR/gRNA-directed synergistic activation mediators (SAM)).

"Subject," as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human. In another embodiment, the subject is poultry. In another embodiment, the subject is piscine. In certain embodiments, the subject is a human, and in certain of these embodiments the human is an infant, child, young adult, or adult.

As used herein, the terms "target nucleic acid" or "target gene" refer to a nucleic acid which is being targeted for binding, e.g., by a CRISPR nuclease in complex with a guide RNA, a guide-RNA, or a scaffold RNA. In certain embodiments, a target nucleic acid comprises one gene, or a promoter or enhancer region operably linked to one gene. In certain aspects, a target nucleic acid may comprise one or more genes, e.g., two genes, three genes, four genes, or five genes, or promoters or enhancer regions operably linked to one or more genes. In one embodiment, a target nucleic acid may comprise a promoter region, or control region, of a gene. In one embodiment, a target nucleic acid may comprise an intron of a gene. In another embodiment, a target nucleic acid may comprise an exon of a gene. In one embodiment, a target nucleic acid may comprise a coding region of gene. In one embodiment, a target nucleic acid may comprise a non-coding region of a gene. In some embodiments, the target nucleic acid is a control region, promoter, enhancer, intron, exon, transcription start site, coding region, or non-coding region of a gene listed in Table 1 herein.

In some embodiments, the target nucleic acid is a control region, promoter, enhancer, intron, exon, transcription start site, coding region, or non-coding region of a gene in the same pathway as a gene listed in Table 1 herein. The target nucleic acid can, *e.g.,* be a control region, promoter, enhancer, intron, exon, transcription start site, coding region, or non-coding region of a gene upstream and in the same pathway as a gene listed in Table 1 herein. Additionally, where two or more genes or positions are targeted, or alternatively, the target nucleic acid can, *e.g.,* be a control region, promoter, enhancer, intron, exon, transcription start site, coding region, or non-coding region of a gene downstream and in the same pathway as a gene listed in Table 1 herein. Additionally, where two or more genes or positions are targeted, or alternatively, the target nucleic acid can, *e.g.,* be a control region, promoter, enhancer, intron, exon, transcription start site, coding region, or non-coding region of a gene in a parallel pathway as a gene listed in Table 1 herein. Exemplary genes in the same pathway or a parallel pathway as one or more of the genes listed in Table 1 are described e.g., in the KEGG pathway database (available at www.genomejp/kegg/pathway.html).

"Target position" as used herein, refers to a site on a target nucleic acid that is hybridized to a guide RNA *(e.g.,* in complex with a CRISPR nuclease) or scaffold RNA. Optimized target positions include, without limitation, one or more target positions optimized for transcriptional activation that are described in WO 2016/011080.

"Episomal vector" or "episomally propagating vector" refers to a plasmid or viral vector that persists or propagates in a mammalian cell as an episomal element. Episomal vectors described herein can encode one or more components (e.g., CRISPR nuclease, guide RNA, zinc finger nuclease, TALEN, TAL effector, scaffold RNA, transcriptional activator, affinity element, or combination thereof) for treatment of a disease or condition by transcriptional activation (e.g., a disease or condition of Table 1). Episomal vectors include, but are not limited to, Adeno-associated virus (AAV) vectors, and Epstein-barr virus (EBV) vectors. Suitable AAV vectors and methods for making and using such AAV vectors, *e.g.,* for delivering the vectors into target cells are described in Samulski Ret al. (1987), J. Virol. 61: 3096-3101; Walsh et al., Proc. Soc. Exp. Biol. Med. 204:289-300 (1993); Fisher K J et al. (1996), J. Virol, 70: 520-532; Samulski Ret al. (1989), J. Virol. 63: 3822-3826; U.S. Pat. No. 5,252,479; U.S. Pat. No. 5,139,941; U.S. Pat. No. 5,436,146; International Patent Application No. WO 94/13788; and International Patent Application No. WO 93/24641.

As used herein, the term "Zinc Finger Nuclease" refers to a zinc finger DNA binding protein (or zinc finger DNA binding domain within a larger protein) that binds DNA in a sequence-specific manner through one or more zinc fingers, which are regions of amino acid sequence within the zinc finger binding domain whose structure is stabilized through coordination of a zinc ion. The term zinc finger DNA binding protein is often abbreviated as zinc finger nuclease or ZFN.

As used herein, the term "transcription activator-like effector nuclease" refers to a protein, that includes a transcription activator-like effector DNA-binding domain fused to a DNA cleavage domain, that binds DNA in a sequence-specific manner. The term transcription activator-like effector nuclease is often abbreviated to TALEN.

### DETAILED DESCRIPTION OF THE INVENTION

### Introduction

Described herein are methods and compositions for treating a disease in a mammalian subject associated with, exacerbated by, or caused by reduced transcription of a gene, reduced amount of a gene product, or reduced activity of a gene product by increasing transcription of a target gene. Such methods and compositions can be useful, *e.g.,* for treating a haploinsufficiency disease in the subject. Haploinsufficiency diseases that can be treated by the methods and compositions described herein include, without limitation, one or more of the diseases listed in Table 1. Table 1 provides the Entrez Gene ID (column 2) from the national center for bioinformatics (NCBI) and corresponding gene symbol (column 1) provided by the human genome nomenclature committee (HGNC), a pubmed ID (PMID) citation to a supporting reference (column 4), and a brief description of the associated disorder (column 5). The table is adapted from Supplementary Table 1 of Dang et al., European Journal of Human Genetics (2008) 16, 1350-57and the ClinVar (https://www.ncbi.nlm.nih.gov/clinvar) and ClinGen (https://www.clinicalgenome.org) databases.

### Nucleases

Described herein, a host cell is contacted with one or more nucleases. In some aspects, the nuclease is a endonuclease, site-specific recombinase, transposase, topoisomerase, zinc finger nuclease, TALEN, and includes modified derivatives and variants thereof.

In some embodiments, a nuclease is capable of targeting a designated nucleotide or region within the target site. In some embodiments, the nuclease is capable of targeting a region positioned between the 5' and 3' regions of the target site. In another embodiment, the nuclease is capable of targeting a region positioned upstream or downstream of the 5' and 3' regions of the target site (e.g., upstream or downstream of the transcription start site (TSS)). A recognition sequence is a polynucleotide sequence that is specifically recognized and/or bound by the nuclease. The length of the recognition site sequence can vary, and includes, for example, nucleotide sequences that are at least 10, 12, 14, 16, 18, 19, 20, 21, 22, 23, 24, 25, 26, 27, 28, 29, 30, 35, 40, 45, 50, 55, 60, 65, 70 or more nucleotides in length. In some embodiments, the recognition sequence is palindromic, *i.e.,* the sequence on one DNA strand reads the same in the opposite direction on the complementary DNA strand. In some embodiments, the target site of the nuclease is within the recognition sequence.

### Zinc Finger Nuclease

In some aspects, the nuclease is a zinc-finger nuclease (ZFN). ZFNs typically comprise a zinc finger DNA binding domain and a nuclease domain. Generally, ZFNs include two zinc finger arrays (ZFAs), each of which is fused to a single subunit of a non-specific endonuclease, such as the nuclease domain from the FokI enzyme, which becomes active upon dimerization. Typically, a single ZFA consists of 3 or 4 zinc finger domains, each of which is designed to recognize a specific nucleotide triplet (GGC, GAT, *etc.*). A ZFN composed of two "3-finger" ZFAs is therefore capable of recognizing an 18 base pair target site (*i.e.,* recognition sequence); an 18 base pair recognition sequence is generally unique, even within large genomes such as those of humans and plants. By directing the co-localization and dimerization of the two FokI nuclease monomers, ZFNs generate a functional site-specific endonuclease that can target a particular locus (*e.g.,* gene, promotor or enhancer).

Zinc-finger nucleases useful in the methods described herein include those that are known and ZFN that are engineered to have specificity for one or more target sites described herein (e.g., promotor or enhancer nucleotide sequence). Zinc finger domains are amenable for designing polypeptides which specifically bind a selected polynucleotide recognition sequence within a target site of the host cell genome. ZFN can comprise an engineered DNA-binding zinc finger domain linked to a non-specific endonuclease domain, for example, a nuclease domain from a Type IIs endonuclease such as HO or FokI. In some examples, a zinc finger DNA binding domain can be fused to a site-specific recombinase, transposase, or a derivative thereof that retains DNA nicking and/or cleaving activity.

In a preferredaspect, additional functionalities can be fused to the zinc-finger binding domain, including but not limited to, transcriptional activator domains (such as VP16, VP48, VP64, VP160 and the like) or transcription repressor domains (such as KRAB). In one aspect, the zinc finger nuclease is engineered such that the zinc finger nuclease comprises a transcriptional activator domain selected from VP16, VP48, VP64 or VP160. In one aspect, the zinc finger nuclease is engineered such that the zinc finger nuclease comprises a transcriptional activator domain selected from HSF1, VP16, VP64, p65, RTA, MyoD1, SET7, VPR, histone acetyltransferase p300, TET1 hydroxylase catalytic domain, LSD1, CIB1, AD2, CR3, GATA4, p53, SP1, MEF2C, TAX, PPAR-gamma, and SET9. For example, engineered zinc finger transcriptional activator that interact with a promoter region of the gamma-globulin gene was shown to enhance fetal hemoglobin production in primer adult erythroblasts (Wilber et al., Blood, 115(15):3033-3041). Other polydactyl zinc-finger transcription factors are also known in the art, including those disclosed in Beerli and Barbas (see, Nature Technology, (2002) 20:135-141).

Each zinc finger domain recognizes three consecutive base pairs in the target DNA. For example, a three finger domain recognizes a sequence of nine contiguous nucleotides, with a dimerization requirement of the nuclease, two sets of zinc finger triplets are used to bind a 18 nucleotide recognition sequence. Useful zinc finger modules include those that recognize various GNN and ANN triplets (Dreier et al., (2001) J Biol Chem 276:29466-78; Dreier et al., (2000) J Mol Biol 303:489-502; Liu et al., (2002) J Biol Chem 277:3850-6), as well as those that recognize various CNN or TNN triplets (Dreier et al., (2005) J Biol Chem 280:35588-97; Jamieson et al., (2003) Nature Rev Drug Discovery 2:361-8). See also, Durai et al., (2005) Nucleic Acids Res 33:5978-90; Segal, (2002) Methods 26:76-83; Porteus and Carroll, (2005) Nat Biotechnology 23:967-73; Pabo et al., (2001) Ann Rev Biochem 70:313-40; Wolfe et al., (2000) Ann Rev Biophys Biomol Struct 29:183-212; Segal and Barbas (2001) Curr Opin Biotechnol 12:632-7; Segal et al., (2003) Biochemistry 42:2137-48; Beerli and Barbas, (2002) Nat Biotechnol 20:135-41; Carroll et al., (2006) Nature Protocols 1: 1329; Ordiz et al., (2002) Proc Natl Acad Sci USA 99:13290-5; Guan et al., (2002) Proc Natl Acad Sci USA 99:13296-301; WO2002099084; WO00/42219; WO02/42459; WO2003062455; US20030059767; US Patent Application Publication Number 2003/0108880; U.S. Pat. Nos. 6,140,466, 6,511,808 and 6,453,242. Useful zinc-finger nucleases also include those described in WO03/080809; WO05/014791; WO05/084190; WO08/021207; WO09/042186; WO09/054985; and WO10/065123.

In some aspects, a ZFN comprises a fusion protein having a cleavage domain of a Type IIS restriction endonuclease fused to an engineered zinc finger binding domain, wherein the binding domain further comprises one or more transcriptional activators. In some aspects, the type IIS restriction endonuclease is selected from a HO endonuclease or a FokI endonuclease. In some aspects, the zinc finger binding domain comprises 3, 4, 5 or 6 zinc fingers. In another aspect, the zinc finger binding domain specifically binds to a recognition sequence corresponding to a promoter or enhancer described herein (*e.g.,* SIM1, MC4R, PKD1, SETD5, THUMPD3, SCN2A and PAX6 promotor or enhancer). In one aspect, the one or more transcriptional activators is selected from VP16, VP48, VP64, or VP160. Generally, the DNA-binding domain of a ZFN contains between 3 and 6 individual zinc finger repeats and can recognize between 9 and 18 contiguous nucleotides. Each ZFN can be designed to target a specific target site in the host cell genome, *e.g.,* a promotor sequence, an enhancer sequence, or exon/intron within a gene.

### TALENs

The nuclease may be a TALEN. TAL effectors (TALEs) are proteins secreted by *Xanthomonas* bacteria and play an important role in disease or triggering defense mechanisms, by binding host DNA and activating effector-specific host genes. see, *e.g.,* Gu et al. (2005) Nature 435:1122-5; Yang et al., (2006) Proc. Natl. Acad. Sci. USA 103:10503-8; Kay et al., (2007) Science 318:648-51; Sugio et al., (2007) Proc. Natl. Acad. Sci. USA 104:10720-5; Romer et al., (2007) Science 318:645-8; Boch et al., (2009) Science 326(5959):1509-12; and Moscou and Bogdanove, (2009) 326(5959):1501. A TALEN comprises a TAL effector DNA-binding domain fused to a DNA cleavage domain. The DNA binding domain interacts with DNA in a sequence-specific manner through one or more tandem repeat domains. The repeated sequence typically comprises 33-34 highly conserved amino acids with divergent 12^{th} and 13^{th} amino acids. These two positions, referred to as the Repeat Variable Diresidue (RVD) are highly variable and show a strong correlation with specific nucleotide recognition (Boch et al., (2009) Science 326(5959):1509-12; and Moscou and Bogdanove, (2009) 326(5959):1501). This relationship between amino acid sequence and DNA recognition sequence has allowed for the engineering of specific DNA-binding domains by selecting a combination of repeat segments containing the appropriate RVDs.

The TAL-effector DNA binding domain can be engineered to bind to a target DNA sequence and fused to a nuclease domain, *e.g.,* a Type IIS restriction endonuclease, such as FokI (see *e.g.,* Kim et al. (1996) Proc. Natl. Acad. Sci. USA 93:1156-1160). In some aspects, the nuclease domain can comprises one or more mutations (e.g., FokI variants) that improve cleavage specificity (see, Doyon et al., (2011) Nature Methods, 8 (1): 74-9*)* and cleavage activity *(*Guo et al., (2010) Journal of Molecular Biology, 400 (1): 96-107*).* Other useful endonucleases that can be used as the nuclease domain include, but are not limited to, HhaI, HindIII, Nod, BbvCI, EcoRI, BglI, and AlwI. In some aspects, the TALEN can comprise a TAL effector DNA binding domain comprising a plurality of TAL effector repeat sequences that bind to a specific nucleotide sequence (i.e., recognition sequence) in the target DNA. While not to be construed as limiting, TALENs useful for the methods provided herein include those described in WO10/079430 and U.S. Patent Application Publication No. 2011/0145940.

In some aspects, the TAL effector DNA binding domain can comprise 10 or more DNA binding repeats, and preferably 15 or more DNA binding repeats. In some aspects, each DNA binding repeat comprises a RVD that determines recognition of a base pair in the target DNA, and wherein each DNA binding repeat is responsible for recognizing one base pair in the target DNA. In some aspects, the RVD comprises one or more of: HD for recognizing C; NG for recognizing T; NI for recognizing A; NN for recognizing G or A; NS for recognizing A or C or G or T; N* for recognizing C or T, where * represents a gap in the second position of the RVD; HG for recognizing T; H* for recognizing T, where * represents a gap in the second position of the RVD; IG for recognizing T; NK for recognizing G; HA for recognizing C; ND for recognizing C; HI for recognizing C; HN for recognizing G; NA for recognizing G; SN for recognizing G or A; and YG for recognizing T.

In a preferred aspect, the TALEN is engineered such that the TAL effector comprises one or more transcriptional activator domains (e.g., VP16, VP48, VP64 or VP160). For example, engineered TAL effectors having a transcriptional activator domain at the c-terminus of the TAL effector were shown to modulate transcription of Sox2 and Klf4 genes in human 293FT cells (Zhang et al., Nature Biotechnology, 29(2):149-153 (2011). Other TAL effector transcription factors (TALE-TFs) are also known in the art, including those disclosed in Perez-Pinera et al., (Nature Methods, (2013) 10(3):239-242) that demonstrated modulation of IL1RN, KLK3, CEACAM5 and ERBB2 genes in human 293T cells using TALE-TFs. In some aspects, the one or more transcriptional activator domains are located adjacent to the nuclear localization signal (NLS) present in the C-terminus of the TAL effector. In another aspect, the TALE-TFs can bind nearby sites upstream or downstream of the transcriptional start site (TSS) for a target gene. In one aspect, the TAL effector comprises a transcriptional activator domain selected from VP16, VP48, VP64 or VP160. In another aspect, the TAL effector comprises a transcriptional activator domain selected from HSF1, VP16, VP64, p65, RTA, MyoD1, SET7, VPR, histone acetyltransferase p300, TET1 hydroxylase catalytic domain, LSD1, CIB1, AD2, CR3, GATA4, p53, SP1, MEF2C, TAX, PPAR-gamma, and SET9.

In some aspects, the TALEN comprises a TAL effector DNA-binding domain fused to a DNA cleavage domain, wherein the TAL effector comprises a transcriptional activator. In some aspects, the DNA cleavage domain is of a Type IIS restriction endonuclease selected from a HO endonuclease or a FokI endonuclease. In some aspects, the TAL effector DNA-binding domain specifically binds to a recognition sequence corresponding to a promoter region or enhancer region disclosed herein (*e.g.,* SIM1, MC4R, PKD1, SETD5, THUMPD3, SCN2A and PAX6 promotor or enhancer). Generally, the DNA-binding domain of a TALEN is designed to target a specific target site in the host cell, *e.g.,* a promotor sequence or an enhancer sequence.

In some aspects, the target site for the zinc finger nuclease or TALEN is endogenous to the host cell, such as a native locus in the host cell genome. In some aspects, the target site is selected according to the type of nuclease to be utilized in the method. If the nuclease to be utilized is a zinc finger nuclease, optimal target sites may be selected using a number of publicly available online resources. See, *e.g.,* Reyon et al., BMC Genomics 12:83 (2011). Publicly available methods for engineering zinc finger nucleases include: (1) Context-dependent Assembly (CoDA), (2) Oligomerized Pool Engineering (OPEN), (3) Modular Assembly, (4) ZiFiT (internet-accessible software for the design of engineered zinc finger arrays), (5) ZiFDB (internet-accessible database of zinc fingers and engineered zinc finger arrays), and (6) ZFNGenome. For example, OPEN is a publicly available protocol for engineering zinc finger arrays with high specificity and *in vivo* functionality, and has been successfully used to generate ZFNs that function efficiently in plants, zebrafish, and human somatic and pluripotent stem cells. OPEN is a selection-based method in which a pre-constructed randomized pool of candidate ZFAs is screened to identify those with high affinity and specificity for a desired target sequence. Additionally, ZFNGenome is a GBrowse-based tool for identifying and visualizing potential target sites for OPEN-generated ZFNs. ZFNGenome provides a compendium of potential ZFN target sites in sequenced and annotated genomes of model organisms. ZFNGenome includes more than 11 million potential ZFN target sites, mapped within the fully sequenced genomes of seven model organisms; S. *cerevisiae, C. reinhardtii, A. thaliana, D. melanogaster, D. rerio, C. elegans,* and *H. sapiens.* ZFNGenome provides information about each potential ZFN target site, including its chromosomal location and position relative to transcription initiation site(s). Users can query ZFNGenome using several different criteria (e.g., gene ID, transcript ID, target site sequence).

In some aspects, if the nuclease is a TALEN, optimal target sites may be selected in accordance with the methods described by Sanjana et al., Nature Protocols, 7:171-192 (2012). TALENs function as dimers, and a pair of TALENs, referred to as the left and right TALENs, target sequences on opposite strands of DNA. TALENs are engineered as a fusion of the TALE DNA-binding domain and a monomeric FokI catalytic domain. To facilitate FokI dimerization, the left and right TALEN target sites are generally selected with a spacing of approximately 14-20 bases.

In some aspects, the one or more nucleases useful for the methods described herein are provided, e.g., delivered into the host cell as a purified protein. In some aspects, the one or more nucleases are provided via polynucleotide(s) comprising a nucleic acid encoding the nuclease. In another aspect, the one or more nucleases can be introduced into the host cell as purified RNA which can be directly translated in the host cell nucleus. In a preferred aspect, the polynucleotide comprising a nucleic acid encoding the nuclease comprises an expression vector that allows for the expression of the nuclease within a host cell. Suitable expression vectors include episomal vectors.

In some aspects, where the nuclease functions as a dimer requiring the separate expression of each monomer, e.g., zinc finger nucleases and TALENs, each monomer of the dimer may be expressed from the same episomal vector or from different episomal vectors. In another aspect, where multiple nucleases are introduced to the cell to introduce double-strand breaks at different target sites, the nucleases can be encoded on a single episomal vector or on separate episomal vectors.

In one aspect, the present disclosure provides a method of treating a haploinsufficiency disease in a mammalian subject, the method comprising contacting a cell of the subject with a composition comprising a zinc finger nuclease or TALEN that, under conditions present in a nucleus of the cell, the zinc finger nuclease or TALEN specifically hybridizes to a promoter region or an enhancer region; wherein the contacting forms a complex comprising the DNA binding domain of the zinc finger nuclease or TALEN, and the promoter region or enhancer region, wherein the complex activates transcription of the wild-type copy of the haploinsufficient gene in an amount and for a duration sufficient to treat the haploinsufficiency disease in the subject. In some aspect, the promoter or enhancer region corresponds to a promoter or enhancer region (i.e., control region) of any of the genes listed in Table 1.

In some aspects, the contacting comprises contacting the cell with an episomal vector encoding the zinc finger nuclease or TALEN. In some aspects, the episomal vector(s) are non-integrating. In some aspects, the zinc finger nuclease or TALEN has been modified to comprises one or more transcriptional activation domains. In one aspect, the one or more transcriptional activation domains is selected from the group consisting of HSF1, VP16, VP64, p65, MyoD1, RTA, SET7/9, VPR, histone acetyltransferase p300, an hydroxylase catalytic domain of a TET family protein (e.g., TET1 hydroxylase catalytic domain), LSD1, CIB1, AD2, CR3, EKLF1, GATA4, PRVIE, p53, SP1, MEF2C, TAX, and PPARγ. In some aspect, the transcriptional activaton domain is VP64. In some aspects, the haploinsufficient gene is SIM1, Leptin, Leptin receptor, MC4R, SCN2A, SETD5, PAX6, PKD1, MC3R, POMC, STAT3, STAT5, SOCS3, GHR, NPY, NPY1R, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPT1B, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.

**Table 1: Genes Associated With Haploinsufficiency Diseases**

| **Gene Symbol** | **Entrez Gene ID** | **Chromosome** | **PubMed (PMID)** | **Disorder/ Syndrome** |
|---|---|---|---|---|
| TP73 | 7161 | 1 | 11454718 | prostate hyperplasia and prostate cancer |
| DFFB | 1677 | 1 | 16156899 | oligodendroglioma development |
| KCNAB2 | 8514 | 1 | 11580756 | characteristic craniofacial abnormalities, mental retardation, and epilepsy with 1p36 deletion syndrome |
| CHD5 | 26038 | 1 | - | monosomy 1p36 syndrome |
| CAMTA1 | 23261 | 1 | 15709179 | tumors development |
| PINK1 | 65018 | 1 | 15349860 | sporadic early-onset parkinsonism |
| SAM68 | 10657 | 1 | 17927519 | mammary tumor onset and tumor multiplicity |
| KCNQ4 | 9132 | 1 | - | DEAFNESS, AUTOSOMAL DOMINANT NONSYNDROMIC SENSORINEURAL 2 |
| GLUT1 | 6513 | 1 | 12029447, 11477212, 11136715, 16497725 | Facilitated glucose transporter protein type 1 (GLUT1) deficiency syndrome |
| MYH | 4595 | 1 | 16292541 | hepatocellular carcinoma and cholangiocarcinom |
| FOXE3 | 2301 | 1 | 11980846 | anterior segment dysgenesis similar to Peters' anomaly |
| HUD | 1996 | 1 | 16278682 | poor prognosis |
| INK4C | 1031 | 1 | 16260494 | medulloblastoma formation |
| NFIA | 4774 | 1 | 17530927 | Complex central nervous system (CNS) malformations and urinary tract defects |
| CCN1 | 3491 | 1 | 17023674 | delayed formation of the ventricular septum in the embryo and persistent ostium primum atrial septal defects |
| ABCA4 | 24 | 1 | - | Stargardt disease, retinitis pigmentosa-19, and macular degeneration age-related 2 |
| WNT2B | 7482 | 1 | 17351355 | mental retardation, short stature and colobomata |
| ADAR | 103 | 1 | 16536805 | dyschromatosis symmetrica hereditaria |
| ATP1A2 | 477 | 1 | - | familial hemiplegic migraine type 2 |
| MPZ | 4359 | 1 | - | neurologic diseases, including CHN, DSS, and CMT1B |
| MYOC | 4653 | 1 | - | hereditary juvenile-onset open-angle glaucoma |
| HRPT2 | 79577 | 1 | 16458039 | Ossifying fibroma (progressive enlargement of the affected jaw) |
| LRH-1 | 2494 | 1 | 17670946, 15684064 | inflammatory bowel disease |
| IRF6 | 3664 | 1 | - | van der Woude syndrome and popliteal pterygium syndrome |
| PROX1 | 5629 | 1 | - | Lymphatic vascular defects, adult-onset obesity |
| TP53BP2 | 7159 | 1 | - | no suppression of tumor growth |
| NLRP3 | 114548 | 1 | - | CINCA syndrome |
| ID2 | 3398 | 2 | 15569159 | Congenital hydronephrosis |
| MYCN | 4613 | 2 | 15821734 | reduced brain size and intestinal atresias in Feingold syndrome |
| GCKR | 2646 | 2 | 9570959 | one form of maturity onset diabetes of the young |
| SPAST | 6683 | 2 | - | SPASTIC PARAPLEGIA 4 |
| MSH6 | 2956 | 2 | 10751599 | limitation of mismatch repair |
| FSHR | 2492 | 2 | 14502087 | degenerative changes in the central nervous system |
| SPR | 6697 | 2 | 15241655 | dopa-responsive dystonia |
| PAX8 | 7849 | 2 | - | congenital hypothyroidism |
| SMADIP1 | 9839 | 2 | 11595972, 16688751 | syndromic Hirschsprung disease |
| RPRM | 56475 | 2 | 15592418 | tumorigenesis, no suppression of tumor growth |
| SCN1A | 6323 | 2 | 16865694, 16075041 | Severe myoclonic epilepsy of infancy (SMEI) or Dravet syndrome |
| HOXD13 | 3239 | 2 | 12900906 | foot malformations |
| COL3A1 | 1281 | 2 | - | Ehlers-Danlos syndrome type IV, and with aortic and arterial aneurysms |
| SLC40A1 | 30061 | 2 | 16135412 | ferroportin disease |
| SATB2 | 23314 | 2 | - | craniofacial dysmorphologies, cleft palate |
| SUMO1 | 7341 | 2 | 17606301, 16990542 | nonsyndromic cleft lip and palate |
| BMPR2 | 659 | 2 | 11115378 | primary pulmonary hypertension |
| XRCC5 | 7520 | 2 | 16325483 | retarded growth, increased radiosensitivity, elevated p53 levels and shortened telomeres |
| PAX3 | 5077 | 2 | 12070244, 9731536 | developmental delay and autism |
| STK25 | 10494 | 2 | 15521982 | mild-to-moderate mental retardation with an Albright hereditary osteodystrophy-like phenotype |
| CHL1 | 10752 | 3 | - | 3p deletion (3p-) syndrome |
| SRGAP3 | 9901 | 3 | 12195014 | severe mental retardation |
| VHL | 7428 | 3 | 16061637 | increased lung cancer susceptibility |
| GHRL | 51738 | 3 | - | GHRELIN POLYMORPHISM |
| PPARG | 5468 | 3 | 15073042 | susceptibility to mammary, ovarian and skin carcinogenesis |
| SRG3 | 6599 | 3 | 17255092 | proteasomal degradation |
| RASSF1A | 11186 | 3 | 11585766 | pathogenesis of a variety of cancers, no suppression of tumor growth |
| TKT | 7086 | 3 | - | reduced adipose tissue and female fertility |
| MITF | 4286 | 3 | 10952390, 9170159 | Waardenburg syndrome type 2 |
| FOXP1 | 27086 | 3 | - | tumors development |
| ROBO1 | 6091 | 3 | - | predispose to dyslexia |
| DIRC2 | 84925 | 3 | - | onset of tumor growth |
| ATP2C1 | 27032 | 3 | 15811312, 17597066 | orthodisease, skin disorder |
| FOXL2 | 668 | 3 | 11468277 | blepharophimosis syndrome associated with ovarian dysfunction |
| ATR | 545 | 3 | 15282542 | mismatch repair-deficient |
| SI | 6476 | 3 | - | SUCRASE-ISOMALTASE DEFICIENCY, CONGENITAL |
| TERC | 7012 | 3 | 16284252, 15326392 | Autosomal dominant dyskeratosis congenita (AD DC), a rare inherited bone marrow failure syndrome |
| SOX2 | 6657 | 3 | 16529618, 15503273 | hippocampal malformations and epilepsy |
| OPA1 | 4976 | 3 | 16735988, 11017080 | optic atrophy |
| TFRC | 7037 | 3 | - | stressed erythropoiesis and neurologic abnormalities |
| FGFR3 | 2261 | 4 | 9199352 | a variety of skeletal dysplasias, including the most common genetic form of dwarfism, achondroplasia |
| LETM1 | 3954 | 4 | 16719275 | Wolf Hirshhorn syndrome |
| SH3BP2 | 6452 | 4 | - | Wolf-Hirschhorn syndrome |
| MSX1 | 4487 | 4 | 14630905 | oligodontia |
| RBPJ | 3516 | 4 | - | embryonic lethality and formation of arteriovenous malformations |
| PHOX2B | 8929 | 4 | - | predispose to Hirschsprung disease |
| ENAM | 10117 | 4 | 15649948 | Amelogenesis imperfecta (inherited defects of dental enamel formation) |
| MAPK10 | 5602 | 4 | - | epileptic encephalopathy of the Lennox-Gaustaut type |
| PKD2 | 5311 | 4 | 16720597, 10615132 | Autosomal dominant polycystic kidney disease |
| SNCA | 6622 | 4 | 12477695 | familial Parkinson's disease |
| RIEG | 5308 | 4 | 9480756 | Rieger syndrome (RIEG) characterized by malformations of the anterior segment of the eye, failure of the periumbilical skin to involute, and dental hypoplasia |
| ANK2 | 287 | 4 | - | arrhythmia |
| MAD2L1 | 4085 | 4 | 17038523 | optimal hematopoiesis |
| PLK4 | 10733 | 4 | 16025114 | mitotic infidelity and carcinogenesis |
| FBXW7 | 55294 | 4 | - | cancer (breast, ovary) tumors development |
| TERT | 7015 | 5 | - | DYSKERATOSIS CONGENITA |
| SEMA5A | 9037 | 5 | 9464278 | abnormal brain development |
| GDNF | 2668 | 5 | 11774071 | complex human diseases (Hirschsprung-like intestinal obstruction and early-onset lethality ) |
| FGF10 | 2255 | 5 | 16476029, 15654336 | craniofacial development and developmental disorders |
| PIK3R1 | 5295 | 5 | 10829070 | insulin resistance |
| APC | 324 | 5 | 14691304 | familial adenomatous polyposis |
| RAD50 | 10111 | 5 | 16474176 | hereditary breast cancer susceptibility associated with genomic instability |
| SMAD5 | 4090 | 5 | 12064918 | secondary myelodysplasias and acute myeloid leukemias |
| EGR1 | 1958 | 5 | 17420284 | development of myeloid disorders |
| TCOF1 | 6949 | 5 | 17552945, 16465596, 15930015, 15249688 | depletion of neural crest cell precursors, Treacher Collins syndrome |
| NPM1 | 4869 | 5 | 16341035, 16007073 | myelodysplasias and leukemias |
| NKX2-5 | 1482 | 5 | 16470726, 10398271, 15368344 | microcephaly and congenital heart disease |
| MSX2 | 4488 | 5 | 10742104 | pleiotropic defects in bone growth and ectodermal organ formation |
| NSD1 | 64324 | 5 | 16970856, 16547423, 15720303, 15640245, 15539801, 14631206, 14517949, 12687502, 12676901, 11896389 | Sotos syndrome |
| FOXC1 | 2296 | 6 | 14564054, 11170889 | Axenfeld-Rieger anomaly of the anterior eye chamber |
| DSP | 1832 | 6 | 11841538, 11476106, 10594734, 17475244 | skin fragility/woolly hair syndrome; disruption of tissue structure, integrity and changes in keratinocyte proliferation |
| EEF1E1 | 9521 | 6 | - | no suppression of tumor growth |
| TNXA | 7146 | 6 | 15733269 | Ehlers-Danlos syndrome |
| TNX | 7148 | 6 | 15733269 | Elastic fiber abnormalities in hypermobility type Ehlers-Danlos syndrome |
| HMGA1 | 3159 | 6 | - | insulin resistance and diabetes |
| RUNX2 | 860 | 6 | 16270353, 16187316, 15952089, 15566413, 10204840, 9690033, 9207800 | cleidocranial dysplasia |
| CD2AP | 23607 | 6 | 12764198 | glomerular disease susceptibility |
| ELOVL4 | 6785 | 6 | 17311087, 17254625 | defective skin permeability barrier function and neonatal lethality |
| NT5E | 4907 | 6 | 12805562 | Neuropathy target esterase deficiency |
| SIM1 | 6492 | 6 | 16728530, 10587584 | impaired melanocortin-mediated anorexia and activation of paraventricular nucleus neurons |
| COL10A1 | 1300 | 6 | - | Schmid type metaphyseal chondrodysplasia and Japanese type spondylometaphyseal dysplasia |
| PARK2 | 5071 | 6 | - | PARKINSON DISEASE 2 |
| TWIST1 | 7291 | 7 | 16540516, 16237669, 17003487, 15829502, 11854168 | coronal synostosis |
| GLI3 | 2737 | 7 | 15739154, 14608643, 9054938 | Greig cephalopolysyndactyly and Pallister-Hall syndromes |
| GCK | 2645 | 7 | - | non-insulin dependent diabetes mellitus (NIDDM), maturity onset diabetes of the young, type 2 (MODY2) and persistent hyperinsulinemic hypoglycemia of infancy (PHHI) |
| FKBP6 | 8468 | 7 | 15770126 | Williams-Beuren syndrome |
| ELN | 2006 | 7 | 14556246, 10198167, 16820942, 16784071, 16476938, 12016585, 11735026, 10942104, 10885576, 10780788 | cardiovascular disease and connective tissue abnormalities |
| LIMK1 | 3984 | 7 | 9637430 | Williams syndrome (WS), a neurodevelopmental disorder |
| RFC2 | 5982 | 7 | - | growth deficiency as well as developmental disturbances in Williams syndrome |
| GTF3 | 9569 | 7 | 10573005 | abnormal muscle fatiguability |
| GTF2I | 2969 | 7 | - | Williams-Beuren syndrome |
| NCF1 | 653361 | 7 | 15626477 | autosomal recessive chronic granulomatous disease |
| KRIT1 | 889 | 7 | 12404106 | Cerebral Cavernous Malformations (vascular malformations characterised by abnormally enlarged capillary cavities ) |
| COL1A2 | 1278 | 7 | 17898012 | subtle symptoms like recurrent joint subluxation or hypodontia |
| SHFM1 | 7979 | 7 | 17230488 | severe mental retardation, short stature, microcephaly and deafness |
| RELN | 5649 | 7 | 16376115, 11592844 | Cognitive disruption and altered hippocampus synaptic function |
| FOXP2 | 93986 | 7 | 16470794 | Speech and language impairment and oromotor dysprax |
| CAV1 | 857 | 7 | 15816560, 14981899 | 17beta-estradiol-stimulated mammary tumorigenesis |
| ST7 | 7982 | 7 | - | no suppression of tumor growth |
| BRAF | 673 | 7 | - | Cardiofaciocutaneous (CFC) syndrome |
| SHH | 6469 | 7 | 10852374 | Holoprosencephaly, sacral anomalies, and situs ambiguus |
| HLXB9 | 3110 | 7 | 14663834, 12116275 | Currarino syndrome including a presacral mass, sacral agenesis, and anorectal malformation |
| GATA4 | 2626 | 8 | 10096597 | congenital heart disease |
| NKX3-1 | 4824 | 8 | 15734999 | prostate cancer |
| FGFR1 | 2260 | 8 | - | Pfeiffer syndrome, Jackson-Weiss syndrome, Antley-Bixler syndrome, osteoglophonic dysplasia, and autosomal dominant Kallmann syndrome 2 |
| CHD7 | 55636 | 8 | - | CHARGE syndrome |
| CSN5 | 10987 | 8 | 15735686 | TRC8 hereditary kidney cancer |
| EYA1 | 2138 | 8 | - | branchiootorenal dysplasia syndrome, branchiootic syndrome, and sporadic cases of congenital cataracts and ocular anterior segment anomalies |
| TRPS1 | 7227 | 8 | 11285235 | dominantly inherited tricho-rhino-phalangeal (TRP) syndromes |
| DMRT1 | 1761 | 9 | - | failure of testicular development and feminization in male |
| DMRT2 | 10655 | 9 | - | defective testis formation in karyotypic males and impaired ovary function in karyotypic females |
| MLLT3 | 4300 | 9 | - | neuromotor developmental delay, cerebellar ataxia, and epilepsy |
| ARF | 1029 | 9 | 16199529, 12019208 | acute myeloid leukemia |
| CDKN2B | 1030 | 9 | 10388473 | syndrome of cutaneous malignant melanoma and nervous system tumors |
| BAG1 | 573 | 9 | 15560850 | lung tumorigenesis |
| PAX5 | 5079 | 9 | - | pathogenesis of lymphocytic lymphomas |
| GCNT1 | 2650 | 9 | 16778138 | T lymphoma cells resistant to cell death |
| ROR2 | 4920 | 9 | 17632781 | basal cell nevus syndrome (BCNS) |
| PTCH1 | 5727 | 9 | 11922389, 14500378 | Primitive neuroectodermal tumors formation |
| NR5A1 | 2516 | 9 | 14594453 | impaired testicular development, sex reversal, and adrenal failure |
| LMX1B | 4010 | 9 | 15774843, 11668639, 9837817 | nail-patella syndrome |
| ENG | 2022 | 9 | 15718503, 16470589 | Hereditary hemorrhagic telangiectasia type 1 |
| TSC1 | 7248 | 9 | 14633685 | transitional cell carcinoma of the bladder |
| COL5A1 | 1289 | 9 | 16431952, 11391664, 10777716 | Structural abnormalities of the cornea and lid |
| NOTCH1 | 4851 | 9 | 16601454 | aortic valve disease (cardiac malformation and aortic valve calcification) |
| EHMT1 | 79813 | 9 | 16826528, 15805155 | 9q34 subtelomeric deletion syndrome |
| KLF6 | 1316 | 10 | 17297474 | cellular growth dysregulation and tumorigenesis |
| GATA3 | 2625 | 10 | 17046739, 16817354, 15994092, 15705923, 11577985, 11389161 | HDR (hypoparathyroidism, deafness and renal dysplasia) syndrome |
| ANX7 | 310 | 10 | 14608035 | tumorigenesis |
| PTEN | 5728 | 10 | 16938570, 16793127, 16738322, 16288012, 16027169, 15466193, 15001465, 12569555, 12461751, 11553783, 9697695, 12011252 | prostate cancer high-grade prostatic intra-epithelial neoplasias |
| PAX2 | 5076 | 10 | 14569086 | renal-coloboma syndrome |
| FGF8 | 2253 | 10 | 17448458 | several human craniofacial disorders |
| BUB3 | 9184 | 10 | 16600919 | short life span that is associated with the early onset of aging-related features |
| CDKN1C | 1028 | 11 | 10424812 | Beckwith-Wiedemann syndrome |
| NUP98 | 4928 | 11 | - | destruction of securin in mitosis |
| PAX6 | 5080 | 11 | 16866875, 16719277, 16717455, 15480875, 15057935, 12782766, 12552561, 11920832, 11431688, 16646034 | eye diseases |
| WT1 | 7490 | 11 | 8827067, 17931563 | congenital genitourinary (GU) anomalies and/or bilateral disease and tumorigenesis |
| EXT2 | 2132 | 11 | 11137991 | type II form of multiple exostoses |
| ALX4 | 60529 | 11 | 15057119, 9636085 | Tibial aplasia, lower extremity mirror image polydactyly, brachyphalangy, craniofacial dysmorphism and genital hypoplasia |
| FEN1 | 2237 | 11 | 16978612 | neuromuscular and neurodegenerative diseases |
| SF1 | 7536 | 11 | 17940071, 17200175 | mild gonadal dysgenesis and impaired androgenization |
| FGF3 | 2248 | 11 | 17656375 | otodental syndrome |
| FZD4 | 8322 | 11 | 17103440 | complex chromosome rearrangement with multiple abnormalities including growth retardation, facial anomalies, exudative vitreoretinopathy (EVR), cleft palate, and minor digital anomalies |
| ATM | 472 | 11 | 10571946, 10363981 | High incidence of cancer |
| H2AX | 3014 | 11 | 12914700 | genomic instability, early onset of various tumors |
| FLI1 | 2313 | 11 | 15525489 | Paris-Trousseau thrombopenia |
| NFRKB | 4798 | 11 | 11920839 | cellular immunodeficiency, pancytopenia, malformations |
| PHB2 | 11331 | 12 | - | enhanced estrogen receptor function |
| ETV6 | 2120 | 12 | 16643428 | a paediatric pre-B acute lymphoblastic leukaemia |
| CDKN1B | 1027 | 12 | 16951165, 11042700, 10935480 | ErbB2-induced mammary tumor growth |
| COL2A1 | 1280 | 12 | 10819645 | Stickler syndrome |
| KRT5 | 3852 | 12 | - | epidermolysis bullosa simplex |
| MYF6 | 4618 | 12 | 11053684 | myopathy and severe course of Becker muscular dystrophy |
| IGF1 | 3479 | 12 | 15769976 | subtle inhibition of intrauterine and postnatal growth |
| SERCA2 | 488 | 12 | 17116488, 16204033, 11389134 | colon and lung cancer |
| TBX5 | 6910 | 12 | 15289437, 12789647, 12736217, 11572777 | maturation failure of conduction system morphology and function in Holt-Oram syndrome |
| TBX3 | 6926 | 12 | 17265068, 16896345, 12668170, 12376101, 12116211 | ulnar-mammary syndrome |
| HNF1A | 6927 | 12 | 14633861, 12530534 | reduced serum apolipoprotein M levels |
| BRCA2 | 675 | 13 | 15172125 | predisposed to breast, ovarian, pancreatic and other cancers |
| FKHR | 2308 | 13 | 15489287 | Alveolar rhabdomyosarcomas |
| RB1 | 5925 | 13 | 12531801 | Metaphase cytogenetic abnormalities |
| ZIC2 | 7546 | 13 | 11699604, 11285244 | neurological disorderss, behavioral abnormalities |
| LIG4 | 3981 | 13 | - | LIG4 syndrome, nonlymphoid tumorigenesis |
| COCH | 1690 | 14 | 16078052 | unknown |
| NPAS3 | 64067 | 14 | 12746393 | schizophrenia |
| NKX2-1 | 7080 | 14 | - | Choreoathetosis, hypothyroidism, pulmonary alterations, neurologic phenotype and secondary hyperthyrotropinemia, and diseases due to transcription factor defects |
| PAX9 | 5083 | 14 | 16479262, 16333316, 11941488, 11781684 | posterior tooth agenesis |
| BMP4 | 652 | 14 | 16835935 | a contiguous gene syndrome comprising anophthalmia, pituitary hypoplasia, and ear anomalies |
| GCH1 | 2643 | 14 | - | malignant hyperphenylalaninemia and dopa-responsive dystonia |
| SIX6 | 4990 | 14 | 10512683 | bilateral anophthalmia and pituitary anomalies |
| RAD51B | 5890 | 14 | 16778173 | centrosome fragmentation and aneuploidy |
| BCL11B | 64919 | 14 | 17306224 | suppression of lymphomagenesis and thymocyte development |
| SPRED 1 | 161742 | 15 | - | neurofibromatosis type 1-like syndrome |
| BUBR1 | 701 | 15 | 14744753 | enhanced tumor development |
| DLL4 | 54567 | 15 | - | embryonic lethality due to major defects in arterial and vascular development |
| FBN1 | 2200 | 15 | - | Marfan syndrome, isolated ectopia lentis, autosomal dominant Weill-Marchesani syndrome, MASS syndrome, and Shprintzen-Goldberg craniosynostosis syndrome |
| ALDH1A2 | 8854 | 15 | - | facilitate posterior organ development and prevent spina bifida |
| TPM1 | 7168 | 15 | - | type 3 familial hypertrophic cardiomyopathy |
| P450SCC | 1583 | 15 | 11502818 | 46,XY sex reversal and adrenal insufficiency |
| BLM | 641 | 15 | 12242442 | the autosomal recessive disorder Bloom syndrome |
| COUP-TFII | 7026 | 15 | 15384084 | several malformations, pre- and postnatal growth retardation and developmental |
| SOX8 | 30812 | 16 | - | the mental retardation found in ATR-16 syndrome |
| TSC2 | 7249 | 16 | 16027168, 12100629 | differential cancer susceptibility |
| PKD1 | 5310 | 16 | - | autosomal dominant polycystic kidney disease |
| CBP | 1387 | 16 | 11962765 | Rubinstein-Taybi syndrome |
| SOCS1 | 8651 | 16 | 15197228 | severe liver fibrosis and hepatitis-induced carcinogenesis |
| PRM2 | 5620 | 16 | - | infertility |
| PRM1 | 5619 | 16 | - | infertility |
| ABCC6 | 368 | 16 | - | pseudoxanthoma elasticum |
| ERAF | 51327 | 16 | - | subtle erythroid phenotype |
| SALL1 | 6299 | 16 | 16429401 | Townes-Brocks syndrome |
| CBFB | 865 | 16 | 17022082 | delayed cranial ossification, cleft palate, congenital heart anomalies, and feeding difficulties |
| CTCF | 10664 | 16 | 17962299, 15761865 | loss of imprinting of insulin-like growth factor-II in Wilms tumor |
| WWOX | 51741 | 16 | 17575124 | initiation of tumor development |
| FOXF1 | 2294 | 16 | 11943666 | defects in formation and branching of primary lung buds |
| FOXC2 | 2303 | 16 | 16910099, 16081467, 15624441, 12719382, 11694548, 11078474 | the lymphatic/ocular disorder Lymphedema-Distichiasis |
| YWHAE | 7531 | 17 | - | pathogenesis of small cell lung cancer |
| HIC1 | 3090 | 17 | 16724116 | Miller-Dieker syndrome |
| LIS1 | 5048 | 17 | 17148952, 16642511, 9760204 | abnormal cell proliferation, migration and differentiation in the adult dentate gyrus |
| P53 | 7157 | 17 | 15583690, 12517413, 12467136, 11695559, 11532857, 11319275 | male oral squamous cell carcinomas |
| PMP22 | 5376 | 17 | 15955700 | hereditary neuropathy with liability to pressure palsies |
| COPS3 | 8533 | 17 | 10851253 | Circadian rhythm abnormalities of melatonin in Smith-Magenis syndrome |
| RAI1 | 10743 | 17 | 17041942, 17024248, 16845274, 15690371, 15565467 | Smith-Magenis syndrome |
| TOP3A | 7156 | 17 | - | Smith-Magenis syndrome |
| SHMT1 | 6470 | 17 | - | Smith-Magenis syndrome |
| RNF 13 5 | 84282 | 17 | 17632510 | phenotypic abnormalities including overgrowth |
| NF1 | 4763 | 17 | 16893911, 16835260, 15804420, 15676286, 15103551, 12124168, 9187663, 17103458 | neurofibromatosis type 1 |
| SUZ12 | 23512 | 17 | - | mental impairment in constitutional NF1 microdeletions |
| MEL-18 | 7703 | 17 | 12196719 | breast carcinogenesis |
| KLHL10 | 317719 | 17 | - | disrupted spermiogenesis |
| STAT5B | 6777 | 17 | 15870688 | striking amelioration of IL-7-induced mortality and disease development |
| STAT5A | 6776 | 17 | 15870688 | striking amelioration of IL-7-induced mortality and disease development |
| BECN1 | 8678 | 17 | - | autophagy function, and tumor suppressor function |
| BRCA1 | 672 | 17 | 17420720, 17404506, 15289302 | shortened life span and ovarian tumorigenesis |
| PGRN | 2896 | 17 | 17168647, 16862115 | neurodegeneration |
| MAPT | 4137 | 17 | - | neuronal cell death, neurodegenerative disorders such as Alzheimer's disease, Pick's disease, frontotemporal dementia, cortico-basal degeneration and progressive supranuclear palsy |
| CSH1 | 1442 | 17 | 14642004 | Silver-Russell syndrome |
| POLG2 | 11232 | 17 | - | mtDNA deletions causes COX deficiency in muscle fibers and results in the clinical phenotype |
| PRKAR1A | 5573 | 17 | 15371594 | Carney complex, a familial multiple neoplasia syndrome |
| SOX9 | 6662 | 17 | 17142326, 11606049, 8894698, 8001137 | skeletal dysplasias |
| NHERF 1 | 9368 | 17 | 17078868 | breast tumours |
| FSCN2 | 25794 | 17 | 16043865 | photoreceptor degeneration, autosomal dominant retinitis pigmentosa |
| DSG1 | 1828 | 18 | 17194569 | diseases of epidermal integrity |
| DSG2 | 1829 | 18 | - | ARRHYTHMOGENIC RIGHT VENTRICULAR DYSPLASIA |
| TCF4 | 6925 | 18 | 17478476 | Pitt-Hopkins syndrome, a syndromic mental disorder |
| FECH | 2235 | 18 | 10068685 | protoporphyria |
| MC4R | 4160 | 18 | 12851322, 12639913, 10598807 | increased adiposity and linear growth |
| GALR1 | 2587 | 18 | - | uncontrolled proliferation and neoplastic transformation |
| SALL3 | 27164 | 18 | - | 18q deletion syndrome |
| LKB1 | 6794 | 19 | 12218179 | Peutz-Jeghers syndrome |
| PNPLA6 | 10908 | 19 | 15094302 | organophosphorus-induced hyperactivity and toxicity |
| RYR1 | 6261 | 19 | - | malignant hyperthermia susceptibility, central core disease, and minicore myopathy with external ophthalmoplegia |
| TGFB1 | 7040 | 19 | 17114585 | Aggressive pancreatic ductal adenocarcinoma |
| RPS19 | 6223 | 19 | - | Diamond-Blackfan anemia |
| DMPK | 1760 | 19 | 10021468 | cardiac disease in myotonic dystrophy |
| CRX | 1406 | 19 | 10892846 | photoreceptor degeneration, Leber congenital amaurosis type III and the autosomal dominant cone-rod dystrophy 2 |
| PRPF31 | 26121 | 19 | - | retinitis pigmentosa with reduced penetrance |
| JAG1 | 182 | 20 | 11861489, 11139239, 10590916, 17786115, 11152664, 10534349 | Alagille syndrome |
| PAX1 | 5075 | 20 | 12774041 | Klippel-Feil syndrome |
| GDF5 | 8200 | 20 | 16532400, 12357473 | Multiple-synostosis syndrome |
| HNF4A | 3172 | 20 | 10905494 | monogenic autosomal dominant non-insulin-dependent diabetes mellitus type I |
| SALL4 | 57167 | 20 | 16790473 | Okihiro syndrome |
| MC3R | 4159 | 20 | - | susceptibility to obesity |
| RAE1 | 8480 | 20 | 16355229 | premature separation of sister chromatids, severe aneuploidy and untimely degradation of securin |
| GNAS | 2778 | 20 | 17652219, 15579796 | reduced activation of a downstream target in epithelial tissues |
| EDN3 | 1908 | 20 | - | Hirschsprung disease |
| KCNQ2 | 3785 | 20 | 12700166 | epilepsy susceptibility |
| SOX18 | 54345 | 20 | 17290276 | mental retardation |
| SLC5A3 | 6526 | 21 | - | brain inositol deficiency |
| RUNX1 | 861 | 21 | 17394134, 16364766, 15339695, 15061191, 11830488, 11721958, 15297309, 14556655, 11756147, 10684580 | The 8p11 myeloproliferative syndrome |
| DYRK1A | 1859 | 21 | 12192061 | neurological defects, developmental delay |
| COL6A1 | 1291 | 21 | - | autosomal dominant disorder, Bethlem myopathy |
| PRODH | 5625 | 22 | 17028864 | 22q 11 Deletion syndrome |
| DGCR2 | 9993 | 22 | - | DiGeorge syndrome |
| HIRA | 7290 | 22 | 9063745, 8111380 | DiGeorge syndrome (craniofacial, cardiac and thymic malformations) |
| TBX1 | 6899 | 22 | 16969581, 16684884, 15778864, 12539040, 12351571, 11242049 | 22q11 deletion syndrome and schizophrenia |
| COMT | 1312 | 22 | 16848928 | 22q11.2 deletion syndrome |
| RTN4R | 65078 | 22 | - | schizophrenia susceptibility (schizoaffective disorders are common features in patients with DiGeorge/velocardiofacial syndrome) |
| PCQAP | 51586 | 22 | 11414760 | DiGeorge syndrome |
| LZTR1 | 8216 | 22 | - | DiGeorge syndrome |
| INI1 | 6598 | 22 | 16912184 | pituitary tumorigenesis |
| MYH9 | 4627 | 22 | 16162639 | hematological abnormalities |
| SOX10 | 6663 | 22 | 11641219 | the etiology of Waardenburg/Hirschsprung disease |
| FBLN1 | 2192 | 22 | - | limb malformations |
| PPARA | 5465 | 22 | - | prostate cancer |
| PROSAP2 | 85358 | 22 | 11431708, 12065602 | The terminal 22q13.3 deletion syndrome, characterized by severe expressive-language delay, mild mental retardation, hypotonia, joint laxity, dolichocephaly, and minor facial dysmorphisms |
| SHOX | 6473 | X | 17881654, 17726696, 16776105, 16319696, 15356038, 15173321, 15118270, 14981722, 14557470, 14513876, 14513875, 12673642, 12510982, 12439897, 12116254, 12035792, 11889214, 11701728, 11546827, 11523902, 11503163, 11408757, 11134233, 10905666, 10878753, 10842291, 10798359, 10749976, 10599728 | congenital form of growth failure, the aetiology of "idiopathic" short stature and the growth deficits and skeletal anomalies in Leri Weill, Langer and Turner syndrome |
| P2RY8 | 286530 | X | 15466006 | mentally retarded males |
| NLGN4X | 57502 | X | - | autism and Asperger syndrome |
| TRAPPC2 | 6399 | X | - | spondyloepiphyseal dysplasia tarda |
| RPS4X | 6191 | X | - | unknown |
| CSF2RA | 1438 | X | 8950669 | growth deficiency |
| CHRDL1 | 91851 | X | 3196642 | topographic retinotectal projection and in the regulation of retinal angiogenesis in response to hypoxia |
| SF3B4 | 10262 | 1 | 24003905, 27127115, | Nager syndrome, Hepatocellular carcinoma and Rodriguez Acrofacial Dysotosis |
| CTNND2 | 1501 | 5 | 29127138, 25839933 | Intellectual disability, epilepsy |
| AAGAB | 79719 | 15 | 26608363, 25771163 | Buschke-Fischer-Brauer and punctate palmoplantar keratoderma |
| ABCD1 | 215 | X | 26454440, 29136088 | adrenoleukodystrophy |
| AKT3 | 10000 | 1 | 28969385, 27297869 | Developmental disorders and breast cancer |
| ANKRD 11 | 29123 | 16 | 28422132, 27605097 | KBG syndrome |
| ANOS1 | 3730 | X | 28780519, 25892360 | Kallmann syndrome |
| AP1S2 | 8905 | X | 17617514, 23756445, | Mental retardation |
| AR | 367 | X | 29051026 | Kennedy's disease and androgen insensitivity |
| ARSE | 415 | X | 20301713, 23470839 | chondrodysplasia punctata |
| ARX | 170302 | X | 25044608 | cognitive disability and epilepsy |
| ASXL1 | 171023 | 20 | 27616637 | myelodysplastic syndromes and chronic myelomonocytic leukemia |
| ATP7A | 538 | X | 22992316, 24754450 | Menkes disease, X-linked distal spinal muscular atrophy, and occipital horn syndrome |
| ATP8A2 | 51761 | 13 | 20683487 | cerebellar ataxia and cognitive disabilities |
| ATRX | 546 | X | 20301622 | cognitive disabilities as well as alpha-thalassemia (ATRX) syndrome |
| AUTS2 | 26053 | 7 | 26717414 | autism spectrum disorders, intellectual disability, and developmental delay |
| AVPR2 | 554 | X | 27565746, 27117808 | Nephrogenic Diabetes Insipidus (NDI) |
| BAG3 | 9531 | 10 | 28211974 | cardiomyopathy |
| BCL11A | 53335 | 2 | 28891213 | Autism and intellectual development |
| BCOR | 54880 | X | 26573325 | sarcoma of the kidney |
| BMPR1A | 657 | 10 | 26383923 | Intellectual disability |
| BRWD3 | 254065 | X | 24462886, 17668385 | cognitive disabilities and X-linked macrocephaly |
| BTK | 695 | X | 19039656 | agammaglobulinemia |
| CACNA1C | 775 | 12 | 28493952, 26204268 | Autism |
| CASK | 8573 | X | 28783747, 24927672 | FG syndrome 4, intellectual disability and microcephaly |
| CDH1 | 999 | 16 | 26182300 | breast, colorectal, thyroid, gastric and ovarian cancer |
| CDKL5 | 6792 | X | 27265524, 26701947 | infantile spasm syndrome (ISSX), also known as X-linked West syndrome, and Rett syndrome (RTT). |
| CHD2 | 1106 | 15 | 26677509 | Neurodevelopmental disorders |
| CHD8 | 57680 | 14 | 26921529, 25989142, 2673379 | Autism |
| CHM | 1121 | X | 27820636 | choroideremia |
| CHRM3 | 1131 | 1 | 26959877 | Schizophrenia |
| CLCN5 | 1184 | X | 27117801, 29058463 | Dent disease and renal tubular disorders complicated by nephrolithiasis |
| CNKSR2 | 22866 | X | 22511892 | Intellectual disability |
| CNTN4 | 152330 | 3 | 21308999 | autism spectrum disorders |
| CNTNAP2 | 26047 | 7 | 27439707 | neurodevelopmental disorders, including Gilles de la Tourette syndrome, schizophrenia, epilepsy, autism, ADHD and intellectual disability |
| COL11A1 | 1301 | 1 | 21035103 | Fibrochondrogenesis, Stickler syndrome and with Marshall syndrome |
| COL1A1 | 1277 | 17 | 28102596 | imperfecta types I-IV, Ehlers-Danlos syndrome type VIIA, Ehlers-Danlos syndrome Classical type, Caffey Disease and idiopathic osteoporosis |
| CREBBP | 1387 | 16 | 27342041 | Rubinstein-Taybi syndrome (RTS) and acute myeloid leukemia |
| CRYBB2 | 1415 | 22 | 25489230, 25964531, | Cataracts and prostate cancer |
| CUL4B | 8450 | X | 24898194 | Intellectual disability |
| CYBB | 1536 | X | 27917630 | chronic granulomatous disease (CGD |
| DCX | 1641 | X | 25868952 | pilepsy, cognitive disability, subcortical band heterotopia and lissencephaly syndrome |
| DICER1 | 23405 | 14 | 24761742 | familial tumor susceptibility syndrome |
| DKC1 | 1736 | X | 27570172, 25499969 | X-linked dyskeratosis congenita |
| DLG3 | 1741 | X | 19795139 | cognitive disability |
| DMD | 1756 | X | 28247318 | uchenne muscular dystrophy (DMD), Becker muscular dystrophy (BMD), and cardiomyopathy |
| DSC2 | 1824 | 18 | 26310507 | arrhythmogenic right ventricular dysplasia-11, and cancer |
| EBP | 10682 | X | 22121851 | Chondrodysplasia punctata 2 |
| EDNRB | 1910 | 13 | 8852658 | Hirschsprung disease type 2 |
| EDA | 1896 | X | 25846883 | X-linked hypohidrotic ectodermal dysplasia |
| EFNB 1 | 1947 | X | 15959873 | craniofrontonasal syndrome |
| EFTUD2 | 9343 | 17 | 26507355 | mandibulofacial dysostosis with microcephaly |
| EMX2 | 2018 | 10 | 8528262 | schizencephaly |
| EP300 | 2033 | 22 | 25712426 | Rubinstein-Taybi syndrome and epithelial cancer |
| ERF | 2077 | 19 | 26097063 | craniosynostosis |
| ERMARD | 55780 | 6 | 24056535 | Periventricular nodular heterotopia |
| EXT1 | 2131 | 8 | 24009674 | Multiple osteochondromas |
| EYA4 | 2070 | 6 | 15735644 | Cardiomyopathy and hearing loss |
| F8 | 2157 | X | 28777843 | hemophilia A |
| F9 | 2158 | X | 28007939 | hemophilia B or Christmas disease |
| FAM58A | 92002 | X | 18297069 | STAR syndrome |
| FANCB | 2187 | X | 21910217 | VACTERL syndrome |
| FAS | 355 | 10 | 21490157 | Autoimmune lymphoproliferative syndrome |
| FGD1 | 2245 | X | 27199457 | dysplasia in Aarskog-Scott syndrome and a syndromatic form of X-linked cognitive disability |
| FLCN | 201163 | 17 | 28970150 | Birt-Hogg-Dube syndrome |
| FLG | 2312 | 1 | 21514438 | ichthyosis vulgaris |
| FLNA | 2316 | X | 22238415 | Periventricular nodular heterotopias, otopalatodigital syndromes, frontometaphyseal dysplasia, Melnick-Needles syndrome, and X-linked congenital idiopathic intestinal pseudoobstruction |
| FOXG1 | 2290 | 14 | 28851325 | Rett syndrome |
| FRMD7 | 90167 | X | 25678693 | congenital nystagmus |
| FTSJ1 | 24140 | X | 18401546 | cognitive disability |
| GATA2 | 2624 | 3 | 21670465, 21892158 | monocytopenia and mycobacterial infection syndrome and Emberger syndrome |
| GATA6 | 2627 | 18 | 25706805 | congenital defects and cardiomyopathy |
| GDI1 | 2664 | X | 21736009 | cognitive disability |
| GJA5 | 2702 | 1 | 25992486 | atrial fibrillation |
| GJA8 | 2703 | 1 | 28526010 | zonular pulverulent cataracts, nuclear progressive cataracts, and cataract-microcornea syndrome |
| GK | 2710 | X | 10851254 | glycerol kinase deficiency |
| GLA | 2717 | X | 28723748 | Fabry disease |
| GLI2 | 2736 | 2 | 25974718 | Greig cephalopolysyndactyly syndrome, Pallister-Hall syndrome, preaxial polydactyly type IV, postaxial polydactyly types A1 and B |
| GLMN | 11146 | 1 | 15689436 | glomuvenous malformations |
| GPC3 | 2719 | X | 28371070 | Simpson-Golabi-Behmel syndrome |
| GRIA3 | 2892 | X | 19449417 | Intellectual disability |
| GRIN2A | 2903 | 16 | 27683935 | epilepsy and speech disorder |
| GRIN2B | 2904 | 12 | 27818011 | neurodevelopmental disorders autism, attention deficit hyperactivity disorder, epilepsy and schizophrenia |
| HCCS | 3052 | X | - | microphthalmia syndrome |
| HDAC4 | 9759 | 2 | 20691407 | Mental retardation |
| HMGA2 | 8091 | 12 | 25809938 | Silver-Russell syndrome |
| HNF1B | 6928 | 17 | 27838256 | Intellectual disability |
| HNRNPK | 3190 | 9 | 26173930 | Intellectual disability |
| HPRT1 | 3251 | X | 29185864 | Lesch-Nyhan syndrome or gout |
| HNRNPU | 3192 | 1 | 28393272 | epileptic encephalopathy and intellectual disability |
| IDS | 3423 | X | 27246110 | Hunter syndrome |
| IGF1R | 3480 | 15 | 21811077 | Familial short statute |
| IKBKG | 8517 | X | 27037530 | inncontinentia pigmenti, hypohidrotic ectodermal dysplasia, and immunodeficiencies |
| IL 1RAPL 1 | 11141 | X | 21933724 | intellectual disability |
| KANSL1 | 284058 | 17 | 20301783 | intellectual disability |
| KAT6B | 23522 | 10 | 26334766 | Say-Barber-Biesecker/Young-Simpson syndrome |
| KCNH2 | 3757 | 7 | 24530480 | long QT syndrome type 2 |
| KDM5C | 8242 | X | 25666439 | cognitive disability |
| KDM6A | 7403 | X | 23076834 | Kabuki syndrome |
| KIAA2022 | 340533 | X | 27358180 | cognitive disability and epilepsy |
| KIF11 | 3832 | 10 | 22653704 | microcephaly |
| KMT2A | 4297 | 11 | 28911906 | Acute lymphoid leukemias and acute myeloid leukemias |
| KMT2D | 8085 | 12 | 27530205 | Kabuki syndrome |
| L1CAM | 3897 | X | | Masa syndrome and L1 syndrome |
| LAMP2 | 3920 | X | 28627787 | Danon disease |
| LDLR | 3949 | 19 | 28873201 | Familial hypercholesterolemia |
| LEMD3 | 23592 | 12 | 26694706 | Buschke-Ollendorff syndrome and melorheostosis |
| LHX4 | 89884 | 1 | 25871839 | hypopituitarism |
| LMNA | 4000 | 1 | 20127487 | cardiomyopathy |
| LRP5 | 4041 | 11 | 27228167 | familial exudative vitreoretinopathy |
| MAGEL2 | 54551 | 15 | 26365340, | Prader-Willi syndrome (PWS) |
| MAGT1 | 84061 | X | 24130152 | intellectual disability |
| MAOA | 4128 | X | 8211186 | Mental retardation |
| MAP2K2 | 5605 | 19 | 25487361 | cardiofaciocutaneous syndrome |
| MBD5 | 55777 | | 27786435, 25271084, 24885232 | Microcephaly, intellectual disabilities, speech impairment, and seizures |
| MECP2 | 4204 | X | 29141583 | Rett syndrome |
| MED 13L | 23389 | 12 | 28371282, 28645799 | Intellectual disability |
| MEF2C | 4208 | 5 | 27255693 | cognitive disability, epilepsy, and cerebral malformation |
| MEIS2 | 4212 | 15 | 25712757 | Intellectual disability |
| MEN1 | 4221 | 11 | 9510467, 15105049, 21763627 | Multiple Endocrine Neoplasia type 1 |
| MIDI | 4281 | X | 25304119 | Opitz syndrome |
| MLH1 | 4292 | 3 | 15942939 | colon cancer |
| MNX1 | 3110 | 7 | 24095820 | Currarino syndrome |
| MSH2 | 4436 | 2 | 26498247 | hereditary nonpolyposis colon cancer |
| MSH6 | 2956 | 2 | 6099011 | hereditary nonpolyposis colon cancer, colorectal cancer, and endometrial cancer |
| MTAP | 4507 | 9 | 22464254 | diaphyseal medullary stenosis with malignant fibrous histiocytoma (DMSMFH). |
| MTM1 | 4534 | X | 21488203 | X-linked myotubular myopathy |
| MYBPC3 | 4607 | 11 | 27348999 | familial hypertrophic cardiomyopathy |
| MYLK | 4638 | 3 | 28602422 | Megacystis Microcolon Intestinal Hypoperistalsis Syndrome |
| MYT1L | 23040 | 2 | 22547139 | schizophrenia |
| NDP | 4693 | X | 27217716 | Norrie disease |
| NF2 | 4771 | 22 | 11159946 | neurofibromatosis type II |
| NFIX | 4784 | 19 | 26200704 | Marshall-Smith syndrome or Sotos-like syndrome |
| NHS | 4810 | X | 28557584 | Nance-Horan syndrome |
| NIPBL | 25836 | 5 | 26701315 | Cornelia de Lange syndrome |
| NODAL | 4838 | 10 | 19064609 | Cardiovascular malformations |
| NOG | 9241 | 17 | 25391606 | symphalangism (SYM1) and multiple synostoses syndrome (SYNS1) |
| NR0B1 | 190 | X | 25968435 | congenital adrenal hypoplasia and hypogonadotropic hypogonadism |
| NRXN1 | 9378 | 2 | 26279266 | Pitt-Hopkins-like syndrome-2 and schizophrenia |
| NSDHL | 50814 | X | 26014843 | CHILD syndrome |
| NXF5 | 55998 | X | 11566096 | Mental retardation |
| NYX | 60506 | X | 26234941 | X-linked congenital stationary night blindness |
| OCRL | 4952 | X | 27059748 | oculocerebrorenal syndrome of Lowe and also Dent disease |
| OFD1 | 8481 | X | 28371265 | oral-facial-digital syndrome type I and Simpson-Golabi-Behmel syndrome type 2 |
| OPHN1 | 4983 | X | 17845870 | X-linked cognitive disability |
| OTC | 5009 | X | 26446336 | Hyperammonemia |
| OTX2 | 5015 | 14 | 27299576, 28388256 | syndromic microphthalmia 5 and pituitary hormone deficiency 6 |
| PAFAH1B1 | 5048 | 17 | 11754098 | Lissencephaly |
| PAK2 | 5062 | 3 | 21841781 | intellectual disability |
| PAK3 | 5063 | X | 18523455 | intellectual disability |
| PCDH19 | 57526 | X | 27179713 | epileptic encephalopathy and autism |
| PDHA1 | 5160 | X | 10679936 | X-linked Leigh syndrome |
| PGK1 | 5230 | X | 16567715 | neurological impairment |
| PHEX | 5251 | X | 27840894 | Hypophospatemic rickets |
| PHF6 | 84295 | X | 22190899 | cognitive disability and epilepsy |
| PHF8 | 23133 | X | 17594395 | Mental retardation and cleft palate |
| PIGA | 5277 | X | 24706016 | encephalopathies |
| PITX3 | 5309 | 10 | 16565358 | Ocular and neurological disorders |
| PKP2 | 5318 | 12 | 27030002 | cardiomyopathy |
| PLP1 | 5354 | X | 27793435 | Pelizaeus-Merzbacher disease and spastic paraplegia type 2 |
| POLR1D | 51082 | 13 | 24603435 | Treacher Collins syndrome (TCS) |
| PORCN | 64840 | X | 23696273 | focal dermal hypoplasia |
| PQBP1 | 10084 | X | 21204222 | cognitive disability |
| PRPS1 | 5631 | X | 26089585 | Charcot-Marie-Tooth disease and Arts syndrome |
| PRRT2 | 112476 | 16 | 22744660 | paroxysmal kinesigenic dyskinesias |
| PTHLH | 5744 | 12 | 26733284 | osteochondoplasia |
| PTPN11 | 5781 | 12 | 28328117 | Noonan syndrome |
| RAB39B | 116442 | X | 20159109 | cognitive disability, epilepsy, and macrocephaly |
| RASA1 | 5921 | 5 | 26969842 | capillary malformations and Parkes Weber syndrome |
| RBFOX1 | 54715 | 16 | 26174448 | Epilepsy |
| RET | 5979 | 10 | - | Hirschsprung disease |
| RP2 | 6102 | X | 16969763 | Retinal dystrophies |
| RPS 17 | 6218 | 15 | 23812780 | Diamond-Blackfan anemia |
| RPS24 | 6229 | 10 | 17186470 | Diamond-Blackfan anemia |
| RPS26 | 6231 | 12 | 22045982 | Diamond-Blackfan anemia |
| RPS6KA3 | 6197 | X | 26297997 | Coffin-Lowry syndrome |
| RS1 | 6247 | X | 26043410 | retinoschisis |
| SCN2A | 6326 | 2 | 26291284 | Epilepsy and autism |
| SCN5A | 6331 | 3 | 28069705 | Long QT syndrome type 3 |
| SDHAF2 | 54949 | 11 | 21224366 | paraganglioma |
| SDHB | 6390 | 1 | 27839933 | paraganglioma |
| SDHC | 6391 | 1 | 26652933 | paraganglioma |
| SDHD | 6392 | 11 | 28924001 | paraganglioma |
| SETBP1 | 26040 | 18 | 28346496 | Schinzel-Giedion syndrome |
| SETD5 | 55209 | 3 | 27375234 | Intellectual disability |
| SGCE | 8910 | 7 | 26783545 | Myoclonus dystonia |
| SH2B1 | 25970 | 16 | 23160192 | Maladaptive behaviors and obesity |
| SH2D1A | 4068 | X | - | Lymphoproliferative syndrome |
| SIX3 | 6496 | 2 | 19346217 | holoprosencephaly |
| SLC16A12 | 387700 | 10 | 18304496 | Juvenile cataracts and renal glucosuria |
| SLC16A2 | 6567 | X | 27805744 | Allan-Herndon-Dudley syndrome |
| SLC2A1 | 6513 | 1 | 25532859 | Paroxysmal exertion-induced dyskinesia |
| SLC4A10 | 57282 | 2 | 18413482 | Epilepsy and mental retardation |
| SLC6A8 | 6535 | X | 24137762 | Mental retardation |
| SLC9A6 | 10479 | X | 25044251 | cognitive disability |
| SMAD3 | 4088 | 15 | 22803640 | Cardiovascular malformations and aneurysms |
| SMAD4 | 4089 | 18 | 18823382 | pancreatic cancer, juvenile polyposis syndrome, and hereditary hemorrhagic telangiectasia syndrome |
| SMARCA4 | 6597 | 19 | 23775540 | Rhabdoid tumor predisposition syndrome |
| SMARCB1 | 6598 | 22 | 28338502 | Rhabdoid tumor predisposition syndrome |
| SMS | 6611 | X | 23696453 | intellectual disability |
| SNURF | 8926 | 15 | 22511895 | Prader-Willi Syndrome |
| SOX11 | 6664 | 2 | 6543203 | Autism and mental retardation |
| SOX5 | 6660 | 12 | 23498568 | Mental retardation |
| SPINK1 | 6690 | 5 | 27159572 | hereditary pancreatitis and tropical calcific pancreatitis |
| SRY | 6736 | Y | 7987333 | gonadal dysgenesis |
| STK11 | 6794 | 19 | 29141581 | Peutz-Jeghers syndrome and cancer |
| STS | 412 | X | 26421812 | X-linked ichthyosis (XLI) |
| STXBP1 | 6812 | 9 | 26865513 | infantile epileptic encephalopathy-4 |
| SYN1 | 6853 | | 22807112 | neuronal degeneration such as Rett syndrome |
| SYNGAP1 | 8831 | 6 | 23161826 | intellectual disability and autism |
| TAB2 | 23118 | 6 | 25940952 | congenital heart defects |
| TBX20 | 57057 | 7 | 26118961 | cardiac pathologies |
| TBX22 | 50945 | X | 22851992 | Cleft palate |
| TBX4 | 9496 | 17 | 15106123 | Small patella syndrome |
| TCF12 | 6938 | 15 | 26068201 | Anaplastic oligodendroglioma |
| TDGF1 | 6997 | 3 | 12073012 | forebrain defects |
| TFAP2B | 7021 | 6 | 24507797 | Char syndrome |
| TGFBR1 | 7046 | 9 | 21358634 | Ferguson-Smith disease (FSD) |
| TGFBR2 | 7048 | 3 | 28344185 | Syndrome, Loeys-Deitz Aortic Aneurysm Syndrome |
| TGIF1 | 7050 | 18 | 16962354 | holoprosencephaly type 4 |
| TIMM8A | 1678 | X | 20301395 | Jensen syndrome |
| TNNI3 | 7137 | 19 | 18006163 | cardiomyopathy |
| TP63 | 8626 | 3 | 11462173 | ectodermal dysplasia, cleft lip/palate, and split-hand/foot malformation |
| TSPAN7 | 7102 | X | 19339915 | cognitive disability and neuropsychiatric diseases |
| UBE2A | 7319 | X | 16909393 | cognitive disability |
| UBE3A | 7337 | 15 | 28559284 | autism |
| UPF3B | 65109 | X | 22609145 | Mental retardation |
| VEGFA | 7422 | 6 | 20420808 | Cardiovascular defects |
| WDR45 | 11152 | X | 27030146 | neurodegeneration |
| XIAP | 331 | X | 26182687 | dysgammaglobulinemia |
| YAP1 | 10413 | 11 | 24462371 | hearing loss, intellectual disability, hematuria, and orofacial clefting |
| ZC4H2 | 55906 | X | 23623388 | cognitive disability |
| ZDHHC9 | 51114 | X | 28687527 | cognitive disability |
| ZEB2 | 9839 | 2 | 15121779 | Mowat-Wilson syndromw |
| ZFPM2 | 23414 | | 24769157 | Cardiovascular malformations |
| ZIC1 | 7545 | 3 | 24782033 | Hepatocellular carcinoma |
| ZIC3 | 7547 | X | 24123890 | X-linked visceral heterotaxy |
| ZIC4 | 84107 | 3 | 21204220 | Danny-Walker malformation |
| ZNF41 | 7592 | X | 14628291 | cognitive disability |
| ZNF674 | 641339 | X | 22126752 | cognitive disability |
| ZNF711 | 7552 | X | 21384559 | cognitive disability |
| CACNA1A | 773 | 19 | | Neurological disorders |

### Compositions

### Episomal Vectors

Described herein are compositions useful as components for targeting transcriptional activation domains to genetic control elements to increase transcription of an endogenous gene and thereby treat a disease or condition associated with, exacerbated by, or caused by reduced transcription of a gene, reduced amount of a gene product, or reduced activity of a gene product. The components include guide RNAs, scaffold RNAs, scaffold RNA ligands, CRISPR nucleases, transcriptional activation domains, affinity tag(s), affinity tag ligand(s), fusion proteins of one or more thereof, and combinations thereof. The components also include episomal vectors that encode one or more guide RNAs, scaffold RNAs, scaffold RNA ligands, CRISPR nucleases, transcriptional activation domains, affinity tag(s), affinity tag ligand(s), fusion proteins of one or more thereof, and combinations thereof. The episomal vectors can be single- or double-stranded DNA, single-stranded RNA, or double-stranded RNA.

In one embodiment, an episomal vector encoding a CRISPR nuclease, such as a catalytically inactive CRISPR nuclease is be provided. In some cases, the episomal vector encodes a CRISPR nuclease fused to one or more transcriptional activation domains. In some cases, the episomal vector encodes a CRISPR nuclease fused to one or more affinity tags. In some cases, the episomal vector encodes a CRISPR nuclease fused to one or more affinity tags and one or more transcriptional activation domains. CRISPR nuclease fusion proteins can contain transcriptional activator domain(s) and/or affinity tag(s) fused at the amino-terminus of the CRISPR nuclease, at the carboxy terminus, or a combination thereof. Additionally or alternatively, the CRISPR nuclease can be modified by the insertion of transcriptional activator domain(s) and/or affinity tag(s) within a surface loop. The episomal vector (*e*.*g*., AAV vector) can contain a promoter that is operably linked to the CRISPR nuclease or CRISPR nuclease fusion protein. The promoter can be a promoter that is endogenous to a viral source from which the episomal vector is derived. For example, where the episomal vector is an AAV vector, the promoter can be an endogenous AAV promoter. Alternatively, the promoter can be a promoter that is heterologous to the viral source form which the episomal vector is derived. For example, where the episomal vector is an AAV vector, the promoter can be a non-AAV promoter. The promoter can be a promoter of a gene targeted for transcriptional activation (*e*.*g*., a gene selected from Table 1) or a promoter that is heterologous to the targeted gene. The promoter can be constitutive (*e*.*g*., a CMV promoter, CAG promoter, CBA promoter, EF1a promoter, PGK promoter, *etc.),* tissue specific (*e.g.,* a synapsin, camKIIa, GFAP, RPE, ALB, TBG, MBP, MCK, TNT, or aMHC, promoter, and the like), or inducible (*e*.*g*., tetracycline inducible).

In one aspect, an episomal vector encoding a zinc finger nuclease is provided. In some cases, the episomal vector encodes a zinc finger nuclease fused to one or more transcriptional activation domains. In some cases, the episomal vector encodes a zinc finger nuclease fused to one or more affinity tags. In some cases, the episomal vector encodes a zinc finger nuclease fused to one or more affinity tags and one or more transcriptional activation domains. Zinc finger nuclease fusion proteins can contain transcriptional activator domain(s) and/or affinity tag(s) fused at the amino-terminus of the zinc finger nuclease, at the carboxy terminus, or a combination thereof. The episomal vector (*e*.*g*., AAV vector) can contain a promoter that is operably linked to the zinc finger nuclease or zinc finger nuclease fusion protein. The promoter can be a promoter that is endogenous to a viral source from which the episomal vector is derived. For example, where the episomal vector is an AAV vector, the promoter can be an endogenous AAV promoter. Alternatively, the promoter can be a promoter that is heterologous to the viral source form which the episomal vector is derived. For example, where the episomal vector is an AAV vector, the promoter can be a non-AAV promoter. The promoter can be a promoter of a gene targeted for transcriptional activation (*e*.*g*., a gene selected from Table 1) or a promoter that is heterologous to the targeted gene. The promoter can be constitutive (*e*.*g*., a CMV promoter, CAG promoter, CBA promoter, EF1a promoter, PGK promoter, *etc.),* tissue specific (*e.g., a* synapsin, camKIIa, GFAP, RPE, ALB, TBG, MBP, MCK, TNT, or aMHC, promoter, and the like), or inducible (*e*.*g*., tetracycline inducible).

In one aspect, an episomal vector encoding a TALEN is provided. In some cases, the episomal vector encodes a TALEN fused to one or more transcriptional activation domains. In some cases, the episomal vector encodes a TALEN fused to one or more affinity tags. In some cases, the episomal vector encodes a TALEN fused to one or more affinity tags and one or more transcriptional activation domains. TALENs can contain transcriptional activator domain(s) and/or affinity tag(s) fused at the amino-terminus, at the carboxy terminus, or a combination thereof. The episomal vector (*e*.*g*., AAV vector) can contain a promoter that is operably linked to the TALEN. The promoter can be a promoter that is endogenous to a viral source from which the episomal vector is derived. For example, where the episomal vector is an AAV vector, the promoter can be an endogenous AAV promoter. Alternatively, the promoter can be a promoter that is heterologous to the viral source form which the episomal vector is derived. For example, where the episomal vector is an AAV vector, the promoter can be a non-AAV promoter. The promoter can be a promoter of a gene targeted for transcriptional activation (*e*.*g*., a gene selected from Table 1) or a promoter that is heterologous to the targeted gene. The promoter can be constitutive (*e*.*g*., a CMV promoter, CAG promoter, CBA promoter, EF1a promoter, PGK promoter, *etc.),* tissue specific *(e.g.,* a synapsin, camKIIa, GFAP, RPE, ALB, TBG, MBP, MCK, TNT, or aMHC, promoter, and the like), or inducible (*e*.*g*., tetracycline inducible).

In one embodiment, an episomal vector encoding a guide RNA is provided. The guide RNA can be a small guide RNA. The guide RNA can be a component of a synergistic activation mediator (*e.g.,* as described in Zhang et al., Scientific Reports 5, Article No. 16277 (2015); and Konermann et al., 2015, Nature 517:583-8). The episomal vector *(e.g.,* AAV vector) can contain a promoter that is operably linked to the guide RNA. The promoter can be a promoter that is endogenous to a viral source from which the episomal vector is derived. For example, where the episomal vector is an AAV vector, the promoter can be an endogenous AAV promoter. Alternatively, the promoter can be a promoter that is heterologous to the viral source form which the episomal vector is derived. For example, where the episomal vector is an AAV vector, the promoter can be a non-AAV promoter. The promoter can be a promoter of a gene targeted for transcriptional activation (*e*.*g*., a gene selected from Table 1) or a promoter that is heterologous to the targeted gene. The promoter can be constitutive (*e*.*g*., a CMV promoter, CAG promoter, CBA promoter, EF1a promoter, PGK promoter, *etc.),* tissue specific *(e.g.,* a synapsin, camKIIa, GFAP, RPE, ALB, TBG, MBP, MCK, TNT, or aMHC, promoter, and the like), or inducible (*e*.*g*., tetracycline inducible ).

In some embodiments, the episomal vector encodes both a CRISPR nuclease and a guide RNA. In some cases, the CRISPR nuclease is operably linked to a promoter and the guide RNA is operably linked to a different promoter. In some cases, the two promoters are the same. In some cases, the two promoters are different. In some cases, both promoters are inducible. In some cases, both promoters are tissue specific. In some cases, both promoters are constitutive. In some cases, one promoter is constitutive and the other promoter is tissue specific. In some cases, one promoter is constitutive and the other promoter is inducible. In some cases, one promoter is tissue specific and the other is inducible.

In some embodiments, the episomal vector encodes a scaffold RNA, such as a scaffold RNA described in WO 2016/054106. In some embodiments, the episomal vector also encodes a CRISPR nuclease. Additionally or alternatively, the episomal vector can also encode one or more transcriptional activation domain(s). In some cases, the transcriptional activation domain(s) are fused to a binding element that binds to the scaffold RNA (*e*.*g*., binds to an ms2,f6, PP7, com, or L7a sequence of a scaffold RNA).

In some embodiments, two or more different episomal vector are provided. For example, an episomal vector encoding a CRISPR nuclease and a separate episomal vector encoding a guide RNA can be provided. Alternatively, an episomal vector encoding a CRISPR nuclease and a guide RNA can be provided and a separate episomal vector encoding one or more transcriptional activation domain(s) can be provided. In some cases, the one or more transcriptional activation domains are fused to a binding element that binds a scaffold RNA *(e.g.,* binds a guide RNA of an SAM). In some cases, the one or more transcriptional activation domains are fused to a binding element that binds an affinity tag of a CRISPR nuclease. In some embodiments, an episomal vector encoding a scaffold RNA is provided and a separate episomal vector is provided that encodes one or more transcriptional activation domain(s) fused to a binding element that binds the scaffold RNA.

In some aspects, the episomal vector encodes a zinc finger nuclease or TALEN that hybridizes to or specifically hybridizes to *(e.g.,* under stringent hybridization conditions) a gene listed in Table 1, or a gene in the same pathway or a parallel pathway as a gene listed in Table 1. In some cases, the episomal vector encodes a zinc finger nuclease or TALEN that hybridizes to or specifically hybridizes to *(e.g.,* under stringent hybridization conditions) a control region *(e.g.,* promoter region or enhancer region) of a gene listed in Table 1, or a gene in the same pathway or a parallel pathway as a gene listed in Table 1.

In some cases, the episomal vector encodes a zinc finger nuclease or TALEN that hybridizes to or specifically hybridizes to *(e.g.,* under stringent hybridization conditions) to *SIM1, Leptin, Leptin receptor, MC4R, SCN2A, SETD5, PAX6, PKD1, MC3R, POMC, STAT3, STATS, SOCS3, GHR, NPY, NPY1R, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPT1B, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.*

In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *SIM1, Leptin, Leptin receptor, MC4R, SCN2A, SETD5, PAX6, PKD1, MC3R, POMC, STAT3, STATS, SOCS3, GHR, NPY, NPY1R, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPTIB, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.*

In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *SIM1.* In some cases, the the episomal vector encodes a zinc finger nuclease or TALEN that hybridizes to or specifically hybridizes to *(e.g.,* under stringent hybridization conditions) a promoter region of *SIM1.* In some cases, the episomal vector encodes a zinc finger nuclease or TALEN that hybridizes to or specifically hybridizes to *(e.g.,* under stringent hybridization conditions) an enhancer region of *SIM1.* In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *MC4R.* In some cases, the episomal vector encodes a zinc finger nuclease or TALEN that hybridizes to or specifically hybridizes to *(e.g.,* under stringent hybridization conditions) a promoter region of *MC4R.* In some cases, the episomal vector encodes a zinc finger nuclease or TALEN that hybridizes to or specifically hybridizes to *(e.g.,* under stringent hybridization conditions) an enhancer region of *MC4R.* In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *PDK1.* In some cases, the the episomal vector encodes a zinc finger nuclease or TALEN that hybridizes to or specifically hybridizes to *(e.g.,* under stringent hybridization conditions) a promoter region of *PDK1.* In some cases, the episomal vector encodes a zinc finger nuclease or TALEN that hybridizes to or specifically hybridizes to *(e.g.,* under stringent hybridization conditions) an enhancer region of *PDK1.* In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *SETD5.* In some cases, the episomal vector encodes a zinc finger nuclease or TALEN that hybridizes to or specifically hybridizes to *(e.g.,* under stringent hybridization conditions) a promoter region of *SETD5.* In some cases, the episomal vector encodes a zinc finger nuclease or TALEN that hybridizes to or specifically hybridizes to *(e.g.,* under stringent hybridization conditions) an enhancer region of *SETD5.* In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *SCN2A.* In some cases, the episomal vector encodes a zinc finger nuclease or TALEN that hybridizes to or specifically hybridizes to *(e.g.,* under stringent hybridization conditions) a promoter region of *SCN2A.* In some cases, the episomal vector encodes a zinc finger nuclease or TALEN that hybridizes to or specifically hybridizes to *(e.g.,* under stringent hybridization conditions) an enhancer region of *SCN2A.* In some cases, the episomal vector encodes a zinc finger nuclease or TALEN that hybridizes to or specifically hybridizes to *(e.g.,* under stringent hybridization conditions) a control region *(e.g.,* promoter region or enhancer region) of *PAX6.* In some cases, the episomal vector encodes a zinc finger nuclease or TALEN that hybridizes to or specifically hybridizes to *(e.g.,* under stringent hybridization conditions) a promoter region of *PAX6.* In some cases, the episomal vector encodes a zinc finger nuclease or TALEN that hybridizes to or specifically hybridizes to *(e.g.,* under stringent hybridization conditions) an enhancer region of *PAX6.*

In some aspects, the episomal vector encodes a guide or scaffold RNA that hybridizes to or specifically hybridizes to *(e.g.,* under stringent hybridization conditions) a gene listed in Table 1, or a gene in the same pathway or a parallel pathway as a gene listed in Table 1. In some cases, the episomal vector encodes a guide or scaffold RNA that hybridizes to or specifically hybridizes to *(e.g.,* under stringent hybridization conditions) a control region *(e.g.,* promoter region or enhancer region) of a gene listed in Table 1, or a gene in the same pathway or a parallel pathway as a gene listed in Table 1.

In some cases, the episomal vector encodes a guide or scaffold RNA that hybridizes to or specifically hybridizes to *(e.g.,* under stringent hybridization conditions) to *SIM1, Leptin, Leptin receptor, MC4R, SCN2A, SETD5, PAX6, PKD1, MC3R, POMC, STAT3, STATS, SOCS3, GHR, NPY, NPY1R, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPTIB, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.*

In some cases, the episomal vector encodes a guide or scaffold RNA that hybridizes to or specifically hybridizes to *(e.g.,* under stringent hybridization conditions) a control region *(e.g.,* promoter region or enhancer region) of *SIM1, Leptin, Leptin receptor, MC4R, SCN2A, SETD5, PAX6, PKD1, MC3R, POMC, STAT3, STATS, SOCS3, GHR, NPY, NPY1R, NPY2R, NPY5R, PYY, AMPK (PRKAA1, PRKAA2, PRKAB1, PRKAB2, PRKAG1, PRKAG2, PRKAG3), OXT, JAK2, SHP2, NOS3, NROB2, BRS3, CARTPT, FABP4, HTR2C, IL6, NHLH2, NMU, NPB, NPBWRI, PNPLA2, UCP3, ADIPOQ, APOA5, ARNT2, ASIP, C1QTNF2, C3AR1, CCK, CPTIB, CSF2, DGAT1, DGAT2, GHRL, GHSR, HSD11B1, HTR7, INSIG1, INSIG2, LIPC, NMURI, NMUR2, NPBWR2, NTS, PPARGC1A, PPY, RETN, SIRT1, TGFBR2, WDTC1, or FOXO1.*

In some cases, the episomal vector encodes a guide or scaffold RNA that hybridizes to or specifically hybridizes to *(e.g.,* under stringent hybridization conditions) a control region *(e.g.,* promoter region or enhancer region) of *SIM1.* In some cases, the the episomal vector encodes a guide or scaffold RNA that hybridizes to or specifically hybridizes to *(e.g.,* under stringent hybridization conditions) a promoter region of *SIM1.* In some cases, the episomal vector encodes a guide or scaffold RNA that hybridizes to or specifically hybridizes to *(e.g.,* under stringent hybridization conditions) an enhancer region of *SIM1.* In some cases, the episomal vector encodes a guide or scaffold RNA that hybridizes to or specifically hybridizes to *(e.g.,* under stringent hybridization conditions) a control region (*e*.*g*., promoter region or enhancer region) of *MC4R.* In some cases, the episomal vector encodes a guide or scaffold RNA that hybridizes to or specifically hybridizes to *(e.g.,* under stringent hybridization conditions) a promoter region of *MC4R.* In some cases, the episomal vector encodes a guide or scaffold RNA that hybridizes to or specifically hybridizes to *(e.g.,* under stringent hybridization conditions) an enhancer region of *MC4R.* In some cases, the episomal vector encodes a guide or scaffold RNA that hybridizes to or specifically hybridizes to *(e.g.,* under stringent hybridization conditions) a control region *(e.g.,* promoter region or enhancer region) of *PDK1.* In some cases, the the episomal vector encodes a guide or scaffold RNA that hybridizes to or specifically hybridizes to *(e.g.,* under stringent hybridization conditions) a promoter region of *PDK1.* In some cases, the episomal vector encodes a guide or scaffold RNA that hybridizes to or specifically hybridizes to *(e.g.,* under stringent hybridization conditions) an enhancer region of *PDK1.* In some cases, the episomal vector encodes a guide or scaffold RNA that hybridizes to or specifically hybridizes to *(e.g.,* under stringent hybridization conditions) a control region *(e.g.,* promoter region or enhancer region) of *SETD5.* In some cases, the episomal vector encodes a guide or scaffold RNA that hybridizes to or specifically hybridizes to *(e.g.,* under stringent hybridization conditions) a promoter region of *SETD5.* In some cases, the episomal vector encodes a guide or scaffold RNA that hybridizes to or specifically hybridizes to *(e.g.,* under stringent hybridization conditions) an enhancer region of *SETD5.* In some cases, the episomal vector encodes a guide or scaffold RNA that hybridizes to or specifically hybridizes to *(e.g.,* under stringent hybridization conditions) a control region *(e.g.,* promoter region or enhancer region) of *SCN2A.* In some cases, the episomal vector encodes a guide or scaffold RNA that hybridizes to or specifically hybridizes to *(e.g.,* under stringent hybridization conditions) a promoter region of *SCN2A.* In some cases, the episomal vector encodes a guide or scaffold RNA that hybridizes to or specifically hybridizes to *(e.g.,* under stringent hybridization conditions) an enhancer region of *SCN2A.* In some cases, the episomal vector encodes a guide or scaffold RNA that hybridizes to or specifically hybridizes to *(e.g.,* under stringent hybridization conditions) a control region *(e.g.,* promoter region or enhancer region) of *PAX6.* In some cases, the episomal vector encodes a guide or scaffold RNA that hybridizes to or specifically hybridizes to *(e.g.,* under stringent hybridization conditions) a promoter region of *PAX6.* In some cases, the episomal vector encodes a guide or scaffold RNA that hybridizes to or specifically hybridizes to *(e.g.,* under stringent hybridization conditions) an enhancer region of *PAX6.*

In some cases, the targeting region of the guide RNA is encoded by, specifically hybridizes to, or is fully complementary to: SEQ ID NO: 1 (GACACGGAATTCATTGCCAG), SEQ ID NO:2 ( CTGCGGGTTAGGTCTACCGG), SEQ ID NO:3 (GTTGAGCGCTCAGTCCAGCG), SEQ ID NO:4 (TCCCGACGTCGTGCGCGACC), or SEQ ID NO:5 (GCTCTGAATCTTACTACCCG). In some cases, the targeting region of the guide RNA is encoded by, specifically hybridizes to, or is fully complementary to: SEQ ID NO:6 (GCTGTTAACTAAAGACAGGG), SEQ ID NO:7 (GTGGTCTGGGTGATCTCATG), SEQ ID NO:8 (GACAAAGGAACATCTGAGAGG), SEQ ID NO:9 (GTGATCTCATGGGGAAGAGG), or SEQ ID NO:10 (GGCTTTGATCGTGGTCTGGG). In some cases, the targeting region of the guide RNA is encoded by, specifically hybridizes to, or is fully complementary to: SEQ ID NO:11 (GCGAGCCCAGTCGCGTGGGG), SEQ ID NO: 12 (GCCAAGAATTGGCCAAAGGG), SEQ ID NO:34 (GTCAAAGGGGCATATGGAAGG), SEQ ID NO:35 (GGGAAGAAAGCCCCACTTGG), SEQ ID NO:36 (GCCCAGTCGCGTGGGGGGGG), or SEQ ID NO:37 (GGAGCGCGAGTGTCACTCGG). In another embodiment, the targeting region of the guide RNA is encoded by, specifically hybridizes to, or is fully complementary to: SEQ ID NO:38 (GCTCACTGTAGGACCCGAGCC), SEQ ID NO:39 (GACGCGGCGCTCATTGGCCAA), SEQ ID NO:40 (CGAGCCGCGAGCCCAGTCGCG), SEQ ID NO:41 (TCCCCCCCCCCCCCCACGCGA), SEQ ID NO:42 (GTCACTCACCCCGATTGGCCA), or SEQ ID NO:43 (CGCGAGCCCAGTCGCGTGGGG). In some embodiments, the targeting region of the guide RNA is encoded by, specifically hybridizes to, or is fully complementary to: SEQ ID NO:44 (GTTGGCTTATCCAAACATCTC), SEQ ID NO:45 (ATGTTAAGCAAGGGTAATAGA), SEQ ID NO:46 (CTGTGAAAGGAATACAATTCA), SEQ ID NO: 47 (GCCAATTCTTGGCAACCGAGC), or SEQ ID NO:49 (AATTAGCAGACAGCTTGGTAC). In some aspects, the targeting region of the guide RNA is encoded by, specifically hybridizes to, or is fully complementary to: SEQ ID NO:48 (GAATTGGCCAAAGGGAGGGGT). In some embodiments, the targeting region of the guide RNA is encoded by, specifically hybridizes to, or is fully complementary to: SEQ ID NO:51 (CACTGAATACGGATTGGTCAG), SEQ ID NO:52 (GATGTCTCAGAACCACTGAAT), or SEQ ID NO:54 (ACCAATCCGTATTCAGTGGTT). In some aspects, the targeting region of the guide RNA is encoded by, specifically hybridizes to, or is fully complementary to: SEQ ID NO:50 (CTGGCTGATTCCCGAGGATTT) or SEQ ID NO:53 (AACCACTGAATACGGATTGGT). In some aspects, the targeting region of the guide RNA is encoded by, specifically hybridizes to, or is fully complementary to: SEQ ID NO:55 (GGCGCGGGGCGGACGGGGCGA), SEQ ID NO:56 (GCGCCCCGGGAACGCGTGGGG), SEQ ID NO:57 (CGCCCCGCGCCGCGCGGGGAG), SEQ ID NO:58 (TCCGCCCCGCGCCGCGCGGGG), SEQ ID NO:59 (GGAACGCGTGGGGCGGAGCTT), SEQ ID NO:60 (GCCCCGCGCCGCGCGGGGAGG), SEQ ID NO:61 (TGCGCCCCGGGAACGCGTGGG), SEQ ID NO:62 (GAACGCGTGGGGCGGAGCTTC), SEQ ID NO:63 (GCGGCGCGGGGCGGACGGGGC), or SEQ ID NO:64 (CCCGTCCGCCCCGCGCCGCGC). In some aspects, the targeting region of the guide RNA is encoded by, specifically hybridizes to, or is fully complementary to: SEQ ID NO:65 (GGCCCACTCGCCGCCAATCAG), SEQ ID NO:66 (GGAAGCCGCCGGGGCCGCCTA), SEQ ID NO:67 (TGATTGGCGGCGAGTGGGCCA), SEQ ID NO:68: (GCCGCCAATCAGCGGAAGCCG), SEQ ID NO:69: (GGCGGCTTCCGCTGATTGGCG), SEQ ID NO:70: (CCGCCAATCAGCGGAAGCCGC), SEQ ID NO:71: (AGCCGCCGGGGCCGCCTAGAG), SEQ ID NO:72: (GCTTCCGCTGATTGGCGGCGA), SEQ ID NO:73: (CGGCGAGTGGGCCAATGGGTG), or SEQ ID NO:74: (CCAATGGGTGCGGGGCGGTGG). In some aspects, the targeting region of the guide RNA is encoded by or specifically hybridizes to: SEQ ID NO:75 (GGCTGCCGGGGCCGCCTAAAG), SEQ ID NO:76 (GGAGGCTGCCGGGGCCGCCTA), SEQ ID NO:77 (GCCGCCAATCAGCGGAGGCTG), SEQ ID NO:78 (CCGCCAATCAGCGGAGGCTGC), SEQ ID NO:79 (TGGCCGGTGCGCCGCCAATCA), SEQ ID NO:80 (GGCCGGTGCGCCGCCAATCAG), SEQ ID NO: 8 1 (CGGCGCACCGGCCAATAAGTG), SEQ ID NO:82(ATAAGTGTGGGGCGGTGGGCG), SEQ ID NO:83 (CCAATAAGTGTGGGGCGGTGG), or SEQ ID NO:84 (CAATAAGTGTGGGGCGGTGGG). In some embodiments, the targeting region of the guide RNA is encoded by or specifically hybridizes to: SEQ ID NO:85: CCTTTCTATGACCTAGTCGG, SEQ ID NO:86: CAGAATCAGTAACGCACTGT, SEQ ID NO:87: GAAACCAGGAGAGATAACCC, SEQ ID NO:88: GGACCCCAGATATTCTGGAA, SEQ ID NO:89: TTATTGTTGACTTAACGAAG. In some aspects, the targeting region of the guide RNA is encoded by or specifically hybridizes to: SEQ ID NO:90: AAAAAGAAGCAAATAGCTAA, or SEQ ID NO:91: (AGAATCAGTAACGCACTGTA). In some embodiments, the targeting region of the guide RNA is encoded by, specifically hybridizes to, or is fully complementary to: SEQ ID NO:92 (TGTTGGTTTATTGGACCCCAGATATTC), SEQ ID NO:93 (TGTTGGAGAAAATTAACTTAGTGCATA), or SEQ ID NO:94 (TGTTGGTATAACTGCCACTAGAGGGCT). In some aspects, the targeting region of the guide RNA is encoded by, specifically hybridizes to, or is fully complementary to SEQ ID NO:95 (AGGAGCCGGGACCCACCGG).

In some cases, the targeting region of the guide RNA is encoded by, specifically hybridizes to, or is fully complementary to a sequence that is orthologous and/or homologous to a region of a mouse or human genome corresponding to, or targeted by an sgRNA comprising, one of SEQ ID NOs:1-12, or 34-95. In some cases, the guide RNA is encoded by, specifically hybridizes to, or is fully complementary to a sequence that is 90%, 95%, or 99% identical to, or differs by 1, 2, or 3 nucleotides from, or is 1, 2, or 3 nucleotides longer or shorter at a 5' and/or 3' end than one of SEQ ID NOs:1-12, or 34-95.

One or more of the episomal vectors described herein can be provided as a kit for treatment of a disease in a mammalian subject associated with, exacerbated by, or caused by reduced transcription of a gene, reduced amount of a gene product, or reduced activity of a gene product. For example, an episomal vector encoding a CRISPR nuclease, a zinc finger nuclease, a TALEN, a TAL effector, a guide RNA, a transcriptional activation domain, a scaffold RNA, a scaffold RNA ligand, an affinity tag ligand, fusion proteins of one or more thereof, or a combination thereof, can be provided as a component of a kit containing an episomal vector packaging plasmid, cell line, or helper virus, or a combination thereof.

In some cases, an episomal vector in which the encoded polypeptide(s) and/or RNA(s) are flanked by AAV inverted terminal repeats is provided as a component of a kit containing additional materials for packaging the episomal vector into functional AAV particles. Such additional materials can include one or more plasmids encoding AAV rep and cap genes, one or more plasmids encoding adenovirus helper factors E1A, E1B, E2A, E4ORF6 and VA, adenovirus, or a combination thereof. In some cases, the trans-activing elements and/or helper elements for AAV packaging are provided in a stable cell line as a component of the kit.

In some embodiments, the cap gene is an AAV-DJ, AAV1, AAV2, AAV3, AAV4, AAV5, AAV6, AAV7, AAV8, or AAV9 cap gene. In some embodiments, the cap gene is an AAV-DJ, AAV1, AAV2, AAV5, AAV7, AAV8 or AAV9 cap gene. In some embodiments, the cap gene is an AAV2 cap gene. In some embodiments, the cap gene is an AAV-DJ cap gene. In some embodiments, the inverted terminal repeats (ITRs) are AAV1, AAV2, AAV3, AAV4, AAV5, AAV6, AAV7, AAV8, or AAV9 ITRs. In some embodiments, the ITRs are AAV1, AAV2, AAV5, AAV7, AAV8 or AAV9 ITRs. In some embodiments, the ITRs are AAV2 ITRs. In some cases, the capsid protein encoded by the cap gene is the same serotype as the ITRs. For example, the cap gene can be an AAV2 cap gene and the ITRs can be AAV2 ITRs. In some cases, the capsid protein encoded by the cap gene is a different serotype from the serotype of the ITRs. Thus, for example, the cap gene can be an AAV5 cap gene and the ITRs can be AAV2 ITRs. As another example, the cap gene can be an AAV-DJ cap gene and the ITRs can be AAV2 ITRs.

In some cases, the episomal vector can be in a target cell or cell of the target tissue. In some cases, the target cell or cell of a target tissue is a dividing cell. In some cases, the cell is a non-dividing cell. In some cases, the cell is a neuron. In some cases, the cell is a cell of the hypothalamus. In some cases, the target cell or cell of the target tissue is a mammalian cell that contains a genome having at least one functional copy of a target gene, wherein the functional cop(y/ies) in the absence of transcriptional activation by a heterologous complex do not produce enough of a corresponding gene product to produce a wild-type phenotype in an organism. In some cases, the mammalian cell further comprises a scaffold RNA encoded by an episomal vector described herein, a guide RNA encoded by an episomal vector described herein, a CRISPR nuclease encoded by an episomal vector described herein, a SunTag encoded by an one or more episomal vectors described herein, a synergistic activation mediator (SAM) encoded by one or more episomal vectors described herein, a transcriptional activation domain encoded by an episomal vector described herein, an affinity tag ligand encoded by an episomal vector described herein, a fusion of one or more polypeptides described herein encoded by an episomal vector described herein, or a combination thereof.

**In** some cases, the episomal vector in a target cell or a cell of a target tissue is converted to a circular form, a circular concatemer, or a linear concatemer, e.g., through recombination of repeat elements, such as ITRs. In some cases, the episomal vector in the target cell or the cell of a target tissue is converted from a single-stranded DNA vector into a double-stranded DNA. In some cases, the double-stranded DNA is converted into a circular form, a circular concatemer, or a linear concatemer. In some cases, the episomal vector in the target cell or cell of the target tissue persists as an episomal element providing persistent transgene (*e*.*g*., CRISPR nuclease, transcriptional activator, guide RNA, scaffold RNA, *etc.)* expression. In some cases, the episomal elements is one of the foregoing circular forms, circular concatemers, or linear concatemers.

### Viral Particles

One or more of the foregoing episomal vectors can be packaged in a viral particle. For example, the viral particle can contain an episomal vector encoding a CRISPR nuclease, a guide RNA, a scaffold RNA, a transcriptional activator, an affinity tag, an affinity tag ligand, a scaffold RNA ligand, a fusion protein of one or more thereof, or a combination of one or more thereof. The viral particle can be a viral particle that is capable of delivering the episomal vector to a target cell or tissue, such that the episomal vector enter the nucleus of a target cell or a cell of a target tissue and do not, or do not substantially integrate into the genome of the cell.

In some cases, the viral particle delivers the episomal vector to the target cell or cell of the target tissue and the episomal vector is converted to a circular form, a circular concatemer, or a linear concatemer, *e.g*., through recombination of repeat elements, such as ITRs. In some cases, the episomal vector delivered by the viral particle is converted from a single-stranded DNA vector into a double-stranded DNA. In some cases, the double-stranded DNA is converted into a circular form, a circular concatemer, or a linear concatemer. In some cases, the viral particle delivers an episomal vector to a target cell or cell of the target tissue, and the episomal vector persists as an episomal element providing persistent transgene expression.

The viral particles can be EBV or AAV viral particles. In some cases, the viral particles are AAV viral particles. In some cases, the viral particles are AAV-DJ, AAV1, AAV2, AAV3, AAV4, AAV5, AAV6, AAV7, AAV8, or AAV9 viral particles. In some cases, the viral particles are AAV-DJ, AAV1, AAV2, AAV5, AAV7, AAV8 or AAV9 viral particles. In some cases, the viral particles are AAV2 viral particles.. In some cases, the viral particles are AAV-DJ viral particles. The genome packed in the viral particle and encoding the one or more transgenes (the episomal vector) can be an AAV1, AAV2, AAV3, AAV4, AAV5, AAV6, AAV7, AAV8, or AAV9 genome. In some cases, the genome is an AAV1, AAV2, AAV5, AAV7, AAV8 or AAV9 genome. In some cases, the genome is an AAV2 genome. In some cases the genome is the same serotype as the viral particle in which it is packaged. In other cases, the genome and viral particle are of different serotypes. For example, the capsid can be AAV5 serotype and the episomal vector can be AAV2 serotype. As another example, the capsid can be an AAV-DJ serotype and the episomal vector can be an AAV2 serotype.

One or more of the viral particles described herein can be provided as a kit for treatment of a disease in a mammalian subject associated with, exacerbated by, or caused by reduced transcription of a gene, reduced amount of a gene product, or reduced activity of a gene product. For example, an episomal vector encoding a CRISPR nuclease, a guide RNA, a transcriptional activation domain, a scaffold RNA, a scaffold RNA ligand, an affinity tag ligand, fusion proteins of one or more thereof, or a combination thereof, can be packaged into one or more viral particles and provided as a component of a kit containing a suitable pharmaceutical excipient, carrier, diluent, or buffer for delivery to a subject.

In one embodiment, the viral particles are in a suitable pharmaceutical excipient, carrier, diluent, or buffer for delivery to a subject. Such excipients, carriers, diluents, and buffers include any pharmaceutical agent that can be administered without undue toxicity. Pharmaceutically acceptable excipients include, but are not limited to, liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A wide variety of pharmaceutically acceptable excipients are known in the art and need not be discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins Pharmaceutical Dosage Forms and Drug Delivery Systems (1999) H. C. Ansel et al., eds., 7th ed., Lippincott, Williams, & Wilkins and Handbook of Pharmaceutical Excipients (2000) A. H. Kibbe et al., eds., 3rd ed. Amer. Pharmaceutical Assoc.

### Methods

Described herein are methods for treating a disease in a mammalian subject associated with, exacerbated by, or caused by reduced transcription of a gene, reduced amount of a gene product, or reduced activity of a gene product by increasing transcription of a target gene. The methods generally include contacting a target cell or a cell of a target tissue with one or more of the foregoing episomal vectors. In some cases, the episomal vectors are non-integrating or substantially non-integrating. In some cases, the episomal vectors are packaged into viral particles and the viral particles are contacted with the target cell or the cell of a target tissue. In some cases, the contacting is performed *in vivo.* In some cases, the contacting is performed *in vitro* (*e.g.,* using primary cells obtained from the subject) and the contacted cells are delivered to a subject, or optionally cultured and delivered to the subject.

The episomal vectors (*e*.*g*., packaged into viral particles) can be delivered by any means known in the art. In some cases, the episomal vectors are contacted with a cell *in vivo* by systemic delivery *(e.g.,* intravenous delivery). In some cases, the episomal vectors *(e.g.,* packaged into viral particles) are contacted with a cell *in vivo* by site-specific delivery to an affected cell or tissue. For example, viral particles in which episomal vectors are packaged can be injected into a site of an affected cell or tissue. In some cases, two or more episomal vectors are packaged into viral particles such that each viral particle contains a single copy of one of the two or more episomal vectors or is empty (contains no genome or a genome that lacks a functional transgene). Such viral particles can be delivered as a mixture or individually. In some cases, the particles are delivered simultaneously. In some cases, the particles are delivered sequentially. Typically, the particles are delivered such that the delivered transgenes encoded by the episomal vectors are co-expressed in the subject such that a disease is treated.

In one cases, one or more different viral particles (*e*.*g*., viral particles having the same capsid but containing vectors that encode different transgenes) are injected into a brain of a subject. In some cases, the one or more viral particles are injected into a hypothalamus of a subject. The viral particles can be delivered to an anterior portion of the hypothalamus, a posterior portion of the hypothalamus, a ventromedial portion of the hypothalamus, or a combination thereof. The viral particles can be delivered bilaterally (*e*.*g*., via bilateral injections to a hypothalamus of a subject). In some cases, the one or more viral particles are delivered to a neuron of the subject. In some case, the one or more viral particles are delivered by stereotactic injection.

The dose of viral particle delivered to a subject can be from 1x10³ viral particles/kg subject to 1x10²⁰ viral particles/kg subject. The dose of episomal vector delivered to a subject can be from 1x10³ vector molecules/kg subject to 1x10²⁰ vector molecules/kg subject. In some cases, the dose is from 1x10⁴ to 1x10¹⁸, from 1x10⁵ to 1x10¹⁶, from 1x10⁶ to 1x10¹⁵ viral particles/kg subject or vector molecules/kg subject. In some cases, the dose is at least 1x10⁴, 1x10⁵ , 1x10⁶ , 1x10⁷, 1x10⁸, 1x10⁹, 1x10¹⁰, 1x10¹¹, 1x10¹², 1x10¹³, 1x10¹⁴, or 1x10¹⁵ viral particles/kg subject or vector molecules/kg subject. In some cases, vector molecules are in the form of viral genomes delivered in a viral particle. In some cases, the dose is a dose of delivered viral genome *(e.g.,* packaged in a viral particle) encoding a CRISPR nuclease *(e.g.,* dCas9 fused to an activation domain) and a guide RNA (*e.g*., sgRNA). In some cases, the dose is a dose of delivered viral genome (*e.g.,* packaged in a viral particle) encoding a CRISPR nuclease *(e.g.,* dCas9 fused to an activation domain), and a second dose, such as one or more of the foregoing doses is a dose of delivered viral genome (*e*.*g*., packaged in a viral particle) encoding guide RNA *(e.g.,* sgRNA).

In some cases, a systemic does can be higher as compared to a dose applied directly to a tissue or organ to be treated. For example, for treatment of obesity dysregulated by a haploinsufficient sim1 gene in hypothalamus tissue or cell, a lower dose can be delivered to the hypothalamus as compared to a systemic dose. In humans, systemic delivery can, *e.g.,* be about 6.7 X 10¹³- 2.0 X 10¹⁴ viral genomes (vg)/kg (see, clinicaltrials.gov/ct2/show/NCT02122952) and neurosurgical delivery can, *e.g.,* be about 7.5 x 10¹¹- 8.8 x 10¹² vg/kg (see clinicaltrials.gov/ct2/show/NCT01973543).

A dose can be administered once, or multiple times. In some cases, the dose is delivered at least once within a period of 30 days, 60 days, 90 days, 120 days, or 180 days. In some cases, a dose is delivered at least once every 10 weeks, 20 weeks, 30 weeks, 40 weeks, 52 weeks, or 75 weeks, or 100 weeks. In some cases, a dose is delivered at least once every 6 months, 12 months, 18 months, 2 years, 3 years, 5 years, or 10 years. In some cases, a single dose or 2, or 3, or 4 doses results in persistent and sufficient expression of the otherwise haploinsufficient target gene to treat at least one symptom of a disease or condition caused by the haploinsufficiency for a period of months or years. In some cases, a dose is administered, the sufficiency of expression of a target haploinsufficient gene *(e.g.,* a gene in Table 1 such as sim1) is assessed (*e*.*g*., in a target tissue such as hypothalamus) and additional doses are delivered as needed by the same or different route. In some cases, one or more doses of viral particles as described herein are delivered, in sufficient amount to increase transcription of a target gene and thereby treat at least one symptom of a disease associated with, exacerbated by, or caused by reduced transcription of a gene, reduced amount of a gene product, or reduced activity of a gene product, and one or more doses are re-administered when transcription of the target gene has reduced from its maximal expression by at least 10%, 25%, 50%, 75%, 90%, or more.

### EXAMPLES

### Rescue of Haploinsufficiency-caused Obesity

### I. Introduction

Over 300 genes are known to cause human disease due to haploinsufficiency (1, 2), leading to a wide range of phenotypes that include cancer, neurological diseases, developmental disorders, immunological diseases, metabolic disorders, infertility, kidney disease, limb malformations and many others (1). Large-scale exome sequencing analyses estimate that a total of 3,230 human genes could be heterozygous loss-of-function (LoF) intolerant (3). Gene therapy holds great promise in correcting haploinsufficient diseases, by inserting a functional recombinant copy or copies of the mutant gene. Currently, there are a total of 2,300 clinical trials underway for gene therapy, the majority of them using adeno-associated virus (AAV) to deliver the recombinant gene (4). AAV is a preferred gene delivery method due to its ability to deliver DNA without integrating into the genome, not causing pathogenicity and providing long lasting gene expression of the transgene (5). However, AAV has an optimal 4.7 kilo base (kb) packaging capacity, limiting its gene therapy use for genes longer than 3.5kb (taking into account additional regulatory sequences needed for its stable expression). Analysis of the 3,230 heterozygous LoF genes finds 715 (22%) of them to have coding sequence longer than 3.5kb, rendering them not suitable for AAV gene therapy.

CRISPR gene editing can potentially fix haploinsufficient mutations, however it would require the need to custom tailor the editing strategy for each mutation. Moreover, it's not a feasible therapy for heterozygous LoF micro-deletions. To address these challenges, we devised a novel therapeutic strategy for haploinsufficiency using CRISPR activation (CRISPRa). CRISPRa takes advantage of the RNA-guided targeting ability of CRISPR to direct a nuclease deficient Cas9 (dCas9) along with a transcriptional activator to regulatory element/s of a specific gene, thus increasing its expression (6-10). Here, we tested whether we can use this system to increase the transcription of the unaffected endogenous gene in a haploinsufficient disease to rescue the disease phenotype.

*SIM1* is a transcription factor that is expressed in the developing kidney and central nervous system, and is essential for the formation of the supraoptic (SON) and paraventricular (PVN) nuclei of the hypothalamus (11). It is also thought to play a major role in the leptin pathway (12). In humans, haploinsufficiency of *SIM1* due to chromosomal aberrations (12, 13) results in hyperphagic obesity (13) and *SIM1* coding mutations, many of them being loss-of-function, are thought to be a major cause of severe obesity in humans (14-16). *Sim1* homozygous null mice die perinatally, while *Sim1* heterozygous mice *(Sim1*^{*+*/}*⁻)* survive, are hyperphagic and develop early-onset obesity with increased linear growth, hyperinsulinemia and hyperleptinemia (17). A postnatal conditional knockout of hypothalamic *Sim1* leads to a similar phenotype in heterozygous mice (18), implicating *Sim1* to be an important regulator of energy homeostasis. Overexpression of *SIM1,* using a human bacterial artificial chromosome in mice, rescues diet-induced obesity and reduced food intake (19), suggesting a potential role for *Sim1* as a general therapeutic target for obesity. Here, we used *Sim1* as our proof of concept model for our CRISPRa therapeutic strategy. We tested the ability of CRISPRa to rescue the obesity phenotype in *Sim1*^{*+*/*-*} mice using both transgenic and AAV based approaches targeting the *Sim1* promoter or its hypothalamus specific enhancer. Our results present a novel therapeutic approach for treating haploinsufficient diseases, or other diseases caused by altered gene dosage.

### II. Results

### A. Upregulation of Sim1 in vitro

We first set out to optimize our CRISPRa conditions *in vitro. SIM1* has a well characterized promoter (20) and distant hypothalamus enhancer (~270kb from the transcription start site), *Sim1* candidate enhancer 2 (SCE2 (21)), both of which were chosen as targets for CRISPRa **(****Fig. 1A****).** We designed sgRNAs for either the *Sim1* promoter or enhancer (SCE2). Using these guides we tested if dCas9 fused to VP64 (dCas9-VP64), a transcriptional activator that carries four tandem copies of VP16 (a herpes simplex virus type 1 transcription factor) (22), can overexpress *Sim1* in mouse neuroblastoma cells (Neuro-2a). This activator was chosen due to its lower activation levels compared to other known activators (23), as we wanted to obtain therapeutic *Sim1* dosage levels *in vivo* that are similar to wild-type. Cells were transfected with dCas9-VP64 and the various guides and following 48 hours *Sim1* mRNA levels were measured using quantitative PCR (qPCR). We identified one sgRNA for either promoter or SCE2 that was able to overexpress endogenous *Sim1* by 13 and 4 fold respectively **(****Fig. 1B****).** Additionally, we identified four sgRNAs for the *Sim1* promoter that were able to overexpress endogenous *Sim1* by over 4-fold **(****Fig. 7A****)** and at least one sgRNA for SCE2 that was able to overexpress endogenous *Sim1* by over 2-fold **(****FIG. 8A****).**

### B. Transgenic CRISPRa rescues obesity

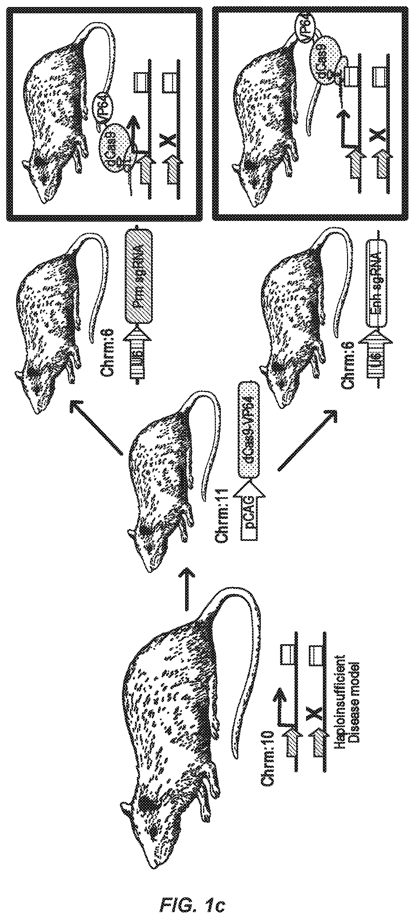

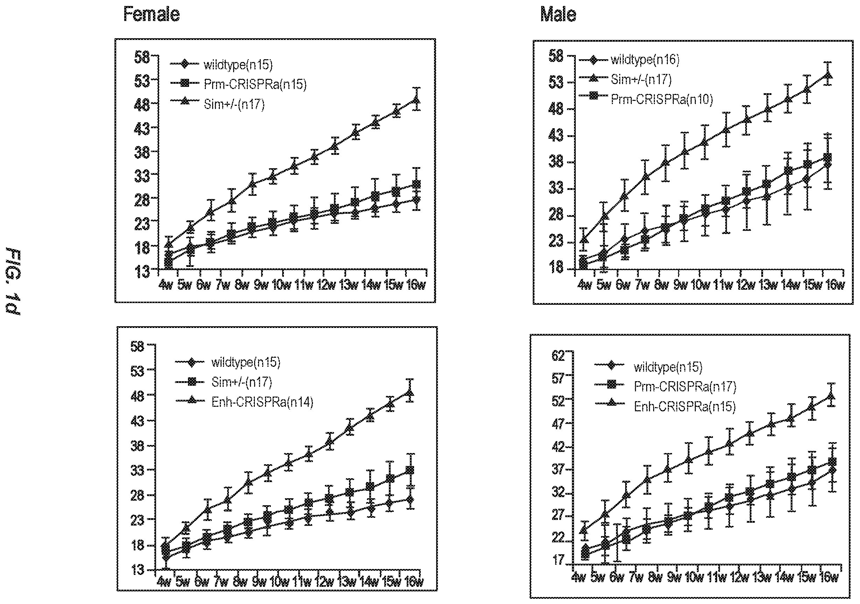

To test the ability of our CRISPRa system to activate *Sim1 in vivo,* we generated knockin mouse lines using TARGATT technology (24) that have dCas9-VP64 inserted into the mouse *Hipp11 (H11P^{CAG-dCas9-VP64})* locus and either sgRNA, targeting the *Sim1* promoter (*ROSA26^{Sim1Pr-sgRNA}*) or SCE2 *(ROSA26^{SCE2En-sgRNA}),* in the *Rosa26* locus **(****Fig. 1C****).** We then crossed these mice to *Sim1*^{*+*/*-*} mice that develop severe obesity (17). Mice having all three alleles (*Sim1*^{*+*/}*⁻ X H11P^{CAG-dCas9-VP64}* and *ROSA26^{Sim1Pr-sgRNA}* or *ROSA26^{SCE2En-sgRNA})* were maintained using breeders chow (picodiet-5058) and weighed on a weekly basis until 16 weeks of age along with wild-type littermates and *Sim1*^{*+*/}*⁻ X H11P^{CAG-dCas9-VP64}* mice and *Sim1*^{*+*/}*⁻,* both of which become severely obese (negative controls). Analysis of at least seven females and seven males per condition showed that *Sim1*^{*+*/*-*} mice carrying both dCas9-VP64 and either *Sim1* promoter or enhancer sgRNA have a significant reduction in body weight compared to *Sim1*^{*+*/}*⁻ X H11P^{CAG-dCas9-VP64}* and Sim1^{+/-} littermates **(****Figs. 1D-F).**

### C. CRISPRa corrects Sim1^{+/-} metabolic profile

To relate body weight reduction with body composition and metabolic parameters, we next performed metabolic profiling for *Sim1*^{*+*/}*⁻ X H11P^{CAG-dCas9-VP64}* X *ROSA26^{Sim1Pr-sgRNA}* (Prm CRISPRa) *Sim1*^{*+*/*-*} X *H11P^{CAG-dCas9-VP64} X ROSA26^{SCE2En-sgRNA}* (Enh-CRISPRa) and our other mouse lines. Three mice for each genotype were analyzed for body composition and metabolic profiling, right at the onset of the obesity phase, 6-8 weeks of age. Both Prm-CRISPRa and Enh-CRISPRa mice showed a significant reduction in body fat content compared to *Sim1*^{*+*/*-*} in both females and males **(****Fig. 2A****).** Metabolic chamber analyses of other hallmarks of *Sim1*^{*+*/*-*} obese mice such as oxygen consumption and food intake showed a shift towards wild-type metabolic parameters in the Prm-CRISPRa and Enh-CRISPRa mice **(****Fig. 2B-C).** In addition, their respiratory exchange ratio (RER; VCO2/VO2), an indirect method of defining basic metabolic rate, also showed parameters similar to their wild-type littermates **(****Fig. 2D****).** However, we did not observe any significant differences for their physical activity in individual chambers. Combined, these results show that both Prm-CRISPRa and Enh-CRISPRa mice have less body fat and demonstrate an improvement in their metabolic parameters that contribute towards a reduction in their overall body weight.

### D. Sim1 activation is tissue-specific

To test for *Sim1* activation levels and tissue-specificity in our mice, we measured its mRNA expression levels in different tissues. We selected two tissues where *Sim1* is known to be expressed, hypothalamus and kidney, and two tissues where it is not expressed, lung and liver (25) **(****Fig. 3A****).** We first measured *dCas9* expression, and found it to be expressed in all four tissues, as expected, since we used a ubiquitous CMV enhancer chicken beta-Actin (CAG) promoter to drive its expression **(****Fig. 3B****).** In contrast, for *Sim1,* we observed significantly higher mRNA levels in the hypothalamus and kidney in Prm-CRISPRa mice and only in the hypothalamus of Enh-CRISPRa mice compared to *Sim1*^{*+*/*-*} mice **(****Fig. 3C-D).** Since we did not observe any significant differences between the obesity phenotype of Prm-CRISPRa and Enh-CRISPRa mice, we could speculate that the activation of *Sim1* in the hypothalamus is sufficient to rescue the *Sim1*^{*+*/*-*} obesity phenotype. Interestingly, in tissues where *Sim1* is not expressed (*i.e.* liver and lung), we could not detect *Sim1* expression in Prm-CRISPRa or Enh-CRISPRa mice despite observing Cas9 expression. These results imply that in the *in vivo* conditions of our study, dCas9-VP64 could only upregulate expression in tissues where the cis-regulatory elements of its target gene are active. This suggests that cis-regulatory elements could be used to determine the tissue-specificity of CRISPRa.

### E. CRISPRa AAV reduces Sim1^{+/-} weight gain

To further translate this approach to a therapeutic strategy for haploinsufficiency, we took advantage of AAV to deliver CRISPRa into the hypothalamus of *Sim1*^{*+*/*-*} mice. We generated the following three AAV vectors: 1) dCas9-VP64 driven by a cytomegalovirus (CMV) promoter (*pCMV-dCas9-VP64*); 2) *Sim1* promoter sgRNA along with mCherry (*pU6-Sim1Pr-CMV-mCherry*); 3) SCE2 sgRNA along with mCherry (*pU6-SCE2-CMV-mCherry*)*.* For the *pCMV-dCas9-VP64* vector, due to the size of dCas9-VP64 expression cassette, we obtained a 5.4kb insert. While this insert size is above the 4.7kb limit, it was shown that going above 5kb reduces transgene expression levels but still could be used for delivery (26). These vectors were packaged individually into AAV-DJ serotype, which is a chimera of type 2, 8 and 9 that was shown to achieve high expression levels in multiple tissues (27) **(****Fig. 4A****).** We did observe lower but usable viral titers for *pCMV-dCas9-VP64* AAV (see methods). We first tested if of our AAV CRISPRa vectors could overexpress *Sim1 in vitro* using Neuro-2a cells. We observed a 4 and 5 fold upregulation of *Sim1* mRNA expression when targeting the promoter or enhancer respectively **(****Fig. 4A****).** Using additional sgRNAs (SEQ ID NOS:38, 40 or 42), we observed that our AAV CRISPRa vectors could overexpress *Sim1 in vitro* using Neuro-2a cells. We observed a 2-fold to 6-fold upregulation of *Sim1* mRNA expression when targeting the promoter **(****Fig. 7B****)** and a 2-fold to 4.5-fold upregulation of *Sim1* mRNA expression when targeting the enhancer (SCE2) **(****Fig. 8B****).**

Next, we performed stereotactic injections to deliver virus carrying *pCMV-dCas9-VP64* and either *pU6-Sim1Pr-CMV-mCherry* (Prm-CRISPRa-AAV) or *pU6-SCE2-CMV-mCherry* (Enh-CRISPRa-AAV) into the PVN of the hypothalamus of *Sim1*^{*+*/*-*} mice at four weeks of age, before they start developing obesity. As negative controls, we also injected *Sim1*^{*+*/*-*} mice with *pCMV-dCas9-VP64* virus only. We tested for the expression of our sgRNA-CMV-mCherry cassette by performing immunostaining on the hypothalamus of injected mice and found it to be expressed in the PVN **(****Fig. 4B-C)**. To test whether *Sim1* expression levels were increased by delivering CRISPRa-AA V to the hypothalamus of *Sim1*^{*+*/*-*} mice, we measured mRNA expression levels for both *dCas9* and *Sim1* from 11 week old AAV injected mice. *dCas9* was found to be expressed in the hypothalamus of all our *pCMV-dCas9-VP64* AAV injected mice **(****Fig. 4D****).** *Sim1* upregulation was observed in both Prm-CRISPRa-AAV and Enh-CRISPRa-AAV injected hypothalami, but not in mice injected with only *pCMV-dCas9-VP64-AAV* **(****Fig. 4E****).** The injected mice were measured for body weight up to 11 weeks of age **(****Fig. 5A****).** We observed a significant weight reduction in the Prm-CRISPRa-AAV or Enh-CRISPRa-AAV injected mice compared to the *Sim1*^{*+*/*-*} or *pCMV-dCas9-VP64-AAV* injected *Sim1*^{*+*/*-*} mice **(****Fig, 5B-C**). These results show that CRISPRa-AAV mediated upregulation could be used as a viable gene therapy tool to treat haploinsufficiency.

### F. Upregulation of Mc4r in vitro

Over 70% of obesity that has genetic basis is caused by defects in the leptin pathway. *MC4R* is part of the leptin pathway and mutations in it are the most commonly found mutations in obese individuals (~5% of the 1 percentile obese population). Since it is a downstream factor, upregulation of *MC4R* and *SIM1* could possibly rescue obesity caused by mutations in these other leptin pathway genes. Here, we have shown that we can upregulate *MC4R* by targeting its promoter and have also shown that upregulation of *SIM1* can increase *MC4R* expression. We were also able to rescue the obesity phenotype in Mc4r heterozygos mice (performed essentially as set forth in the upregulation of *Sim1 in vitro,* discussed above). As such, *MC4R* upregulation could be used as therapy for obesity.

We designed sgRNAs for the *Mc4r* promoter (See, SEQ ID NOS:50-54). Using these guides we tested if dCas9 fused to VP64 (dCas9-VP64) can overexpress *Mc4r* in mouse neuroblastoma cells (Neuro-2a). Cells were transfected with dCas9-VP64 and the various guides and following 48 hours *Mc4r* mRNA levels were measured using quantitative PCR (qPCR). We identified one sgRNA for the *Mc4r* promoter that was able to overexpress endogenous *Mc4r* by 7-fold **(****Fig. 9A****).**

### G. CRISPRa AAV induces upregulation of Mc4r

We next tested if of our AAV CRISPRa vectors (prepared essentially as described under *Sim1* CRISPRa AAV, above) containing sgRNAs, SEQ ID NOS:51, 52 or 54, could overexpress *Mc4r in vitro* using Neuro-2a cells. We observed between a 3.4-fold and 6.6-fold upregulation of *Mc4r* mRNA expression when targeting the promoter **(****Fig. 9B****).**

### H. Upregulation of SCN2A in vitro

Mutations in *SCN2A* are the most commonly found mutations in individuals with autism spectrum disorder (ASD) and epilepsy. The majority of mutations are loss of function leading to ASD due to haploinsufficiency. Here, we have shown that we can upregulate *SCN2A* by targeting its promoter. As such, SCN2A upregulation could be used as therapy for ASD and epilepsy.

We designed sgRNAs for the *Scn2a* promoter (See, SEQ ID NOS:85-91). Using these guides we tested if dCas9 fused to VP64 (dCas9-VP64) can overexpress *Scn2a* in mouse neuroblastoma cells (Neuro-2a). Cells were transfected with dCas9-VP64 and the various guides and following 48 hours *Scn2a* mRNA levels were measured using quantitative PCR (qPCR). We identified four sgRNAs for the *Scn2a* promoter that were able to overexpress endogenous *Scn2a* by over 2-fold **(****Fig. 12A****).**

### I. CRISPRa AAV induces upregulation of Scn2A

We next tested if of our AAV CRISPRa vectors (prepared essentially as described under *Sim1* CRISPRa AAV, above) containing sgRNAs, SEQ ID NOS:92-94, could overexpress *Scn2a in vitro* using Neuro-2a cells. Two different multiplicity of infection (MOI) were used: 5,000 and 1,750 viral genome (vg/ml). We observed a slight upregulation of *Scn2a* mRNA expression when targeting the promoter with a MOI of 5,000 viral genomes per ml **(****Fig. 12B****).**

### J. Upregulation of SETDS in vitro

Mutations in *SETDS* lead to mental retardation-23 (OMIM #615761) which include intellectual disability and dysmorphic features. Here, we have shown that we can upregulate *SETDS* by targeting its promoter. As such, SETD5 upregulation could be used as therapy for intellectual disability.

We designed sgRNAs for the *Setd5* promoter (See, SEQ ID NOS:75-84). Using these guides we tested if dCas9 fused to VP64 (dCas9-VP64) can overexpress *Setd5* in mouse neuroblastoma cells (Neuro-2a). Cells were transfected with dCas9-VP64 and the various guides and following 48 hours *Setd5* mRNA levels were measured using quantitative PCR (qPCR). We identified two sgRNAs for the *Setd5* promoter that were able to overexpress endogenous *Setd5* by over 1.5-fold **(****Fig. 11B****).**

Next, we designed sgRNAs for the *SETD5* promoter in humans (See, SEQ ID NOS:65-74). Using these guides we tested if dCas9 fused to VP64 (dCas9-VP64) can overexpress *SETDS* in human HEK293T cells. Cells were transfected with dCas9-VP64 and the various guides and following 48 hours *SETD5* mRNA levels were measured using quantitative PCR (qPCR). We identified at least one sgRNA for the *SETD5* promoter that was able to overexpress endogenous *SETDS* by over 2.5-fold **(****Fig. 11A****).**

### K. Upregulation of PKD1 in vitro

Mutations in *PKD1* lead to autosomal dominant polycystic kidney disease (ADPKD; OMIM #173900) which is the most frequent hereditary kidney disorder affecting 1 to 400-1000 individuals. 85% of ADPKD is caused by mutations in *PKD1,* the majority of which are loss-of-function. *PKD1* is 13kb long and as such cannot be packaged in standard gene therapy vectors. Using the CRISPRa technology disclosed herein, we have shown that we can upregulate *PKD1* by targeting its promoter. As such, PKD1 upregulation could be used as therapy for autosomal dominant polycystic kidney disease.

We designed sgRNAs for the *PKD1* promoter in humans (See, SEQ ID NOS:55-64). Using these guides we tested if dCas9 fused to VP64 (dCas9-VP64) can overexpress *PKD1* in human HEK293T cells. Cells were transfected with dCas9-VP64 and the various guides and following 48 hours *PKD1* mRNA levels were measured using quantitative PCR (qPCR). We identified at least three sgRNAs for the *PKD1* promoter that were able to overexpress endogenous *PKD1* by over 2-fold **(****Fig. 10****).**

### L. Upregulation of PAX6 in vitro

Loss-of-function mutations in *PAX6* lead to Aniridia 1 (OMIM #106210) due to haploinsufficiency. Here, we have shown that we can upregulate *PAX6* by targeting its promoter. As such, PAX6 upregulation could be used as therapy for aniridia 1.

We designed one sgRNA for the *PAX6* promoter in humans (SEQ ID NO:95). Using this guide we tested if dCas9 (*S. pyogenes*) fused to VP64 (dCas9-VP64) can overexpress *PAX6* in Human H1-ESC cells differentiated into neurons. Cells were infected with lentivirus carrying the guide, and following 48 hours *PAX6* mRNA levels were measured using quantitative PCR (qPCR). Our exemplary sgRNA for the *PAX6* promoter was able to overexpress endogenous *PAX6 by* over 6-fold **(****Fig. 13).** Fig. 13 also demonstrates that additional neuronal markers (e.g., NES) were also capable of neural induction of H1-ESCs. **III. *Discussion***

CRISPR-based gene editing is a promising therapeutic technology to correct genetic mutations. However, it currently is not a feasible technology for haploinsufficiency, limited by low non-homologous end joining (NHEJ) efficiencies (*i.e.* editing only a small portion of cells) and the need to custom tailor specific guides and donor sequences for each individual mutation. In addition, it is not a feasible therapeutic strategy for micro-deletions, over 200 of which are known to cause human disease (28), primarily due to haploinsufficiency. In this study, we used a novel approach to tackle these hurdles and show how a haploinsufficient disease could be corrected by increasing the transcriptional output from the existing functional allele via CRISPRa.

Using CRISPRa targeting for either the promoter or enhancer of *Sim1,* we were able to rescue the obesity phenotype in a tissue-specific manner in mice that are haploinsufficient for *Sim1* **(****Fig. 6****).** As this therapeutic approach takes advantage of the existing functional allele, it has several benefits: 1)It overcomes the need to custom tailor CRISPR gene editing approaches for different haploinssufficient causing mutations in the same gene. 2) This approach could potentially be used to target two or more genes. As such, it could pose as a potential therapeutic strategy for micro-deletions related-diseases that are caused by the heterozygous LoF of more than one gene. 3) CRISPRa-AAV could be used to rescue haploinsufficient diseases caused by genes that are longer than its optimal packaging capability. 4) CRISPR-based therapies can take advantage of cis-regulatory elements to guide tissue-specificity. The availability of large-scale tissue-specific maps of gene regulatory elements could provide ample candidates to use for this therapeutic approach. We observed distinct difference in tissue specific activation of *Sim1* in our study, which can be attributed to chromatin accessibility of the locus in various tissues. Previous large-scale Cas9 and dCas9 cell culture screens have shown a targeting preference for regions with low nucleosome occupancy (29). Active promoters or enhancers would have lower nucleosome occupancy, thus being more amenable to dCas9 targeting.

Our dCas9-VP64 mouse and AAV vectors can be a useful tool for targeted gene activation *in vivo* by delivering sgRNA/s targeted to a specific gene/s in certain tissues/cell types. This approach could be used to assess gene-gene interactions or for the identification of the target gene/s of a specific regulatory element *in vivo* by measuring its expression level following activation. Another potential area of study could be neuronal circuit manipulation. Discrepancies between acute and chronic neuronal circuit manipulations have been observed (30) which can be addressed by our AAV-CRISPRa and Transgenic-CRISPRa strategies respectively.

Haploinsufficiency of *Sim1* causes obesity both in mice (17) and humans (13). Whether this is caused by the reduction in PVN size during development that is observed in *Sim1*^{*+*/*-*} mice (17) or by disturbed energy homeostasis during adulthood was an area of major research. The obesity phenotype observed in the postnatal conditional knockout of hypothalamic *Sim1* (18), reinforced the hypothesis that *Sim1* does indeed have a role in energy homeostasis later during adulthood. Our ability to rescue the obesity phenotype via CRISPRa AAV injections into the hypothalamus of 4 week old mice, further corroborates this role. Abrogation of melanocortin 4 receptor *(Mc4r)* signaling is the hallmark of most polygenic and monogeneic obesity phenotypes. Conditional postnatal deficiency of *Sim1* leads to reduced levels of Mc4r signaling. As *Sim1* was shown to be an integral downstream component of the leptin-Mc4r pathway (18), *Sim1* CRISPRa targeting could provide a potential therapy for conditions that disrupt the leptin signaling pathway.

Despite technological advances in CRISPR-based therapeutic intervention, our understanding of the long-term side effects of CRISPR expression and its off-targeting effects *in-vivo* still remains largely unknown, which also holds true for our current study. Anti-CRISPR genes (31) or conditional activation or silencing of our CRISPRa system could be able to address these concerns in future. Furthermore, there is also a need to develop CRISPRa/i tools to modulate gene dosage, so as to be able to optimize transcriptional output for certain diseases where higher or lower activation levels might be needed. In this study, we used VP64 as our activator, due to its known weak activation capacity (23) which fit with our need to obtain levels of gene expression that are similar to having two normal alleles. CRISPRa based gene activation is dependent upon the nature of the fused activator (23), sgRNA target (29) and may require optimization of the CRISPR system and delivery method.

As demonstrated in this study, CRISPRa can be used to activate genes not only by targeting their promoters, but by also targeting distal cis-regulatory elements such as enhancers. Previous studies have shown that these elements can be viable therapeutic targets. For example, by targeting a globin enhancer with zinc finger nucleases fused to a chromatin looping factor, the LIM domain binding 1 (*LDB1*) gene, activation of fetal hemoglobin was achieved *in vitro,* providing a potential therapy for sickle cell disease (37). In another study, re-activation of fetal hemoglobin was achieved by deactivating the enhancer of its repressor B-cell CLL/lymphoma 11A (*BCL11A*) using CRISPR gene editing (38). Our study provides a novel approach that also takes advantage of *cis*-regulatory elements for therapeutic purposes. There are numerous diseases that are caused by lower gene dosage that could potentially be treated with CRISPRa therapy. In addition, several human diseases could potentially be rescued by the activation of another gene with a similar function. These could include for example *Utrophin* for Duchenne Muscular Dystrophy (39), survival of motor neuron 2 (*SMA2*) for Spinal Muscular Atrophy (SMA; (40)) or the aforementioned fetal globin for sickle cell disease. Further development of this technology could provide a viable therapy for patients inflicted with these diseases.

### III. Materials and Methods

### Plasmids

The *pMSCV-LTR-dCas9-VP64-BFP* vector, encoding a mammalian codon-optimized *Streptococcus pyogenes* dCas9 fused to two C-terminal SV40 NLSs and tagBFP along with a VP64 domain and the U6-sgRNA-CMV-mCherry-T2A-Puro plasmids were used for cell line transfections (both kind gifts from Dr. Stanley Qi). sgRNAs were cloned using the In-Fusion HD-cloning kit (Clontech) following the manufacturer's protocol into the *BstX*I and *Xho*I sites. Mouse knockin vectors were generated by cloning dCas9-VP64 and U6-sgRNA-CMV-mCherry expression cassettes from the aforementioned vectors into the TARGATT (CAG + Poly A) plasmid (Applied StemCell). *pcDNA-dCas9-VP64* (Addgene 47107), and U6-sgRNA-CMV-mCherry-WPREpA were cloned replacing the *Efla-FAS-hChR2(H134R)-mCherry-WPRE-pA* with that of our U6-sgRNA-CMV-mCherry-WPREpA into the backbone of *pAAV-Ef1a-FAS-hChR2(H134R)-mCherry-WPRE-pA* (Addgene 37090).

### AAV production

AAV DJ serotype particles were produced using the Stanford Neuroscience viral vector core. The packaging load for *pCMV-dCas9-VP64* was 5.4kb and for *pU6-Sim1Pr-CMV-mCherry* and *pU6-SCE2-CMV-mCherry* 2.5kb. Genomic titers were ascertained by WPRE and ITR probes to be 1.40E¹⁰ viral genome (vg)/ml for *pCMV-dCas9-VP64* and around 3.30E¹³ vg/ml for *pU6-Sim1Pr-CMV-mCherry* and 2.20 E¹³ vg/ml for *pU6-SCE2-CMV-mCherry .*

### Cell culture

Neuroblastoma 2a cells (Neuro-2a; ATCC^{®} CCL-131) were grown following ATCC guidelines. Plasmids were transfected into Neuro-2a cells using X-tremeGENE HP DNA transfection reagent (Roche) following the manufacturer's protocol. AAV particles were infected into Neuro2a cells at different MOIs. Neuro2a cells were harvested 48 hours post transfection and 5 days post infection to isolate RNA for qRT-PCR analysis.

Human HEK293T cells were grown following ATCC guidelines. Plasmids were transfected into these cells using X-tremeGENE HP DNA transfection reagent (Roche) following the manufacturer's protocol.

### Quantitative reverse-transcription PCR

RNA was isolated from cells or tissues using RNeasy Mini Kit (Qiagen) following the manufacturer's protocol. For mice, animals were euthanized and tissues were harvested directly into the RNA lysis buffer of the RNeasy Mini Kit. The hypothalamus was dissected using a mouse Brain Matrix and slicers from Zivic Instruments. cDNA was prepared using SuperScript III First-Strand Synthesis System (Invitrogen) using the manufacturer's protocol along with DNaseI digestion. qPCR was performed using SsoFast EvaGreen Supermix (Biorad). The results were expressed as fold-increase mRNA expression of the gene of interest normalized to either beta-actin, *Rpl38* or *Elf3* expression by the ΔΔCT method followed by ANOVA and Tukey test for statistical analysis. Reported values are the mean and standard error of the mean from three independent experiments performed on different days (N=3) with technical duplicates that were averaged for each experiment.

### Mice

*Sim1*^{*+*/*-*} mice (17) on a mixed genetic background were obtained as a kind gift from Dr. Jacques Michaud lab. In these mice, a 1kb fragment containing 750bp of the 5' region, the initiation codon, and the sequence coding for the basic domain (the first 17 amino acids) was replaced by a *Pgk-neo* cassette, that was used for genotyping using KAPA mouse genotyping kit (KAPA Biosystems). To generate dCas9-VP64 and sgRNA mice we used TARGATT technology (24). DNA for injection was prepared and purified as mini-circles using the TARGATT Transgenic Kit, V6 (Applied StemCell). The injection mix contained 3 ng/µL DNA and 48 ng/µL of *in vitro* transcribed φC31o mRNA in microinjection TE buffer (0.1 mM EDTA, 10 mM Tris, pH 7.5) and injections were done using standard mouse transgenic protocols (41). dCas9-VP64 was inserted into the mouse *Hipp11* locus and sgRNAs into the *Rosa26* locus. Mice were genotyped using the using the KAPA mouse genotyping kit. F0 TARGATT knock-ins were assessed using PCR7+8, PCR1 described in (PMID: 21464299) along with vector insertion specific dCas9-VP64 primers as well as mCherry specific primers. All mice were fed *ad libitum* Picolab mouse diet 20, 5058 containing 20% protein, 9% fat, 4% fibre for whole study. Calories provided by: Protein, % 23.210 Fat (ether extract), % 21.559 Carbohydrates, % 55.231. All animal work was approved by the UCSF Institutional Animal Care and Use Committee.

### Mouse body weight measurements.

*H11P^{CAG-dCas9-VP64}, ROSA26^{Sim1Pr-sgRNA}* and *ROSA26^{SCE2En-sgRNA}* mice were mated with FVB mice for 3-5 generations to assess germline transmission. Three independent integrants were used from each line to set up matings. *H11P^{CAG-dCas9-VP64}* were mated with *Sim1*^{*+*/*-*} and subsequent *Sim1*^{*+*/*-*} X *H11P^{CAG-dCas9-VP64}* mice were rossed with either *ROSA26^{Sim1Pr-sgRNA}* or *ROSA26^{SCE2En-sgRNA}* to generate mice having all three unlinked alleles. Mice were maintained at Picodiet 5058 throughout the study and at least 6 females and 6 males from all genotypes (wild-type littermates, *Sim1*^{*+*/}*⁻, Sim1*^{*+*/}*⁻ X H11P^{CAG-dCas9-VP64} Sim1*^{*+*/}*⁻ X H11P^{CAG-dCas9-VP64}* X *ROSA26^{Sim1Pr-sgRNA}, Sim1*^{*+*/*-*} X *H11P^{CAG-dCas9-VP64} X ROSA26^{SCE2En-sgRNA})* were measured for their body weights from 4-16 weeks of age on a weekly basis.

### Mouse metabolic profiling

Metabolic rates from individual mice were measured using the Columbus Instruments Comprehensive Lab Animal Monitoring System (CLAMS; Columbus Instruments). Mice were single housed and acclimatized on powdered picodiet 5058 for 3-4 days before performing the metabolic monitoring. We individually housed mice in CLAMS units and measurements were carried out over 4-5 days. The temperature was maintained at 22°C and oxygen and carbon dioxide were calibrated with 'Air reference' set at 20.901 and 0.0049. Three males and three females from each genotype: wild-type littermates, *Sim1*^{*+*/}*⁻, Sim1*^{*+*/}*⁻ X H11P^{CAG-dCas9-VP64} X ROSA26^{Sim1Pr-sgRNA}, Sim1*^{*+*/}*⁻ X H11P^{CAG-dCas9-VP64} X ROSA26^{SCE2En-sgRNA}* were measured. with metabolic parameter (VCO2, VO2, RER, food intake, and activity monitoring). Metabolic data was analyzed using CLAX support software (Columbus Instruments).

### Body composition analysis

Body composition was measured using either Dual Energy X-ray Absorptiometry (DEXA) or Echo Magnetic Resonance Imaging (EchoMRI; Echo Medical System). For DEXA, mice anesthetized using isoflurane were measured for bone mineral density and tissue composition (fat mass and lean mass) using the Lunar PIXImus. EchoMRI (Echo Medical System) was used to measure whole body composition parameters such as total body fat, lean mass, body fluids, and total body water in live mice without the need for anesthesia or sedation.

### Stereotaxic injections

Four week-old *Sim1*^{*+*/*-*} males or females, weighing between 22 and 26 g, were housed individually in cages for at least 2 days before surgical interventions. Mice were anesthetized with a 100 mg/kg Avertin intraperitoneal injection. The skull was immobilized in a stereotaxic apparatus (David Kopf Instruments). The stereotaxic coordinates for injection into the PVN were 0.80 mm caudal to bregma, 0 mm at the midline, and 5.2 mm below the surface of the skull. A 1.5 mm hole was created in the cranium by circular movements using hand-held Dumont 5-45 tweezers (Fine Science Tools). Using a 31 gauge 1ul Hamilton microsyringe, we injected a dose of 0.5X10⁷ vg/ml of sgRNA-AAV along with 2.5X10⁶ vg/kg of dCas-VP64-AAV, in a total injection volume of 1ul per animal into the PVN unilaterally over a 10 minute period. After AAV delivery, the needle was left in place for 20 minutes to prevent reflux and slowly withdrawn in several steps, over 10 minutes. Mice were administered two doses of buprenorphine (100mg/kg) before and 24 hours post surgery. Immunostaining for mCherry, as described below, was used to validate PVN injection coordinates 2-12 weeks following injection in several mice. Mice were maintained on a picodiet 5058 and weighed on a weekly basis.

### Immunostaining

For immunostaining, mice were anesthetized with pentobarbital (7.5 mg/0.15 ml, i.p.) and transcardially perfused with 10ml of heparinized saline (10 U/ml, 2 ml/min) followed by 10ml of phosphate-buffered 4% paraformaldehyde (PFA). Brains were removed, postfixed for 24 hours in 4% PFA, and then equilibrated in 30% sucrose in PBS for 72 hours. Brains were coronally sectioned (35microns for immunostaining, 50m for stereology) on a sliding microtome (Leica SM 2000R). Immunohistochemistry was performed as previously described (19, 42, 43). Coronal brain sections that had been stored in PBS at 4°C were permeabilized and blocked in 3% normal goat serum/0.3% Triton X-100 for 1 hour and incubated at 4°C overnight using an mCherry antibody at a dilution of 1:500 (Abcam ab167453). Sections were placed in 4,6-diamidino-2-phenylindole (DAPI) (0.2 g /ml; 236276; Roche) for 10 minutes and then mounted on plus coated slides and coverslipped using Vectashield (H-1000; Vector Laboratories). Images of sections containing PVN were captured on a Zeiss Apotome.

### References

1 Dang, V. T., Kassahn, K. S., Marcos, A. E. & Ragan, M. A. Identification of human haploinsufficient genes and their genomic proximity to segmental duplications. Eur J Hum Genet. 16, 1350-1357. doi: 1310.1038/ejhg.2008.1111. Epub 2008 Jun 1354. (2008).
2 Huang, N., Lee, I., Marcotte, E. M. & Hurles, M. E. Characterising and predicting haploinsufficiency in the human genome. PLoS Genet. 6, e1001154. doi: 1001110.1001371/journal .pgen.1001154. (2010).
3 Lek, M. et al. Analysis of protein-coding genetic variation in 60,706 humans. Nature. 536, 285-291. doi: 210.1038/nature19057. (2016).
4 Bender, E. Gene therapy: Industrial strength. Nature. 537, S57-59. doi: 10.1038/1537S1057a. (2016).
5 Kotterman, M. A. & Schaffer, D. V. Engineering adeno-associated viruses for clinical gene therapy. Nat Rev Genet. 15, 445-451. doi: 410.1038/nrg3742. Epub 2014 May 1020. (2014).
6 Gilbert, L. A. et al. CRISPR-mediated modular RNA-guided regulation of transcription in eukaryotes. Cell. 154, 442-451. doi: 410.1016/j.cell.2013.1006.1044. Epub 2013 Jul 1011. (2013).
7 Perez-Pinera, P. et al. RNA-guided gene activation by CRISPR-Cas9-based transcription factors. Nat Methods. 10, 973-976. doi: 910.1038/nmeth.2600. Epub 2013 Jul 1025. (2013).
8 Konermann, S. et al. Genome-scale transcriptional activation by an engineered CRISPR-Cas9 complex. Nature (2014)*.*
9 Hilton, I. B. et al. Epigenome editing by a CRISPR-Cas9-based acetyltransferase activates genes from promoters and enhancers. Nat Biotechnol. 33, 510-517. doi: 510.1038/nbt.3199. Epub 2015 Apr 1036. (2015).
10 Chavez, A. et al. Highly efficient Cas9-mediated transcriptional programming. Nat Methods. 12, 326-328. doi: 310.1038/nmeth.3312. Epub 2015 Mar 1032. (2015).
11 Michaud, J. L., Rosenquist, T., May, N. R. & Fan, C. M. Development of neuroendocrine lineages requires the bHLH-PAS transcription factor SIM1. Genes Dev 12, 3264-3275 (1998).
12 Beckers, S., Zegers, D., Van Gaal, L. F. & Van Hul, W. The role of the leptin-melanocortin signalling pathway in the control of food intake. Crit Rev Eukaryot Gene Expr. 19, 267-287. (2009).
13 Holder, J. L., Jr., Butte, N. F. & Zinn, A. R. Profound obesity associated with a balanced translocation that disrupts the SIM1 gene. Hum Mol Genet 9, 101-108 (2000).
14 Ahituv, N. et al. Medical sequencing at the extremes of human body mass. Am J Hum Genet. 80, 779-791. (2007).
15 Ramachandrappa, S. et al. Rare variants in single-minded 1 (SIM1) are associated with severe obesity. J Clin Invest. 123, 3042-3050. doi: 3010.1172/JCI68016. Epub 62013 Jun 68017. (2013).
16 Bonnefond, A. et al. Loss-of-function mutations in SIM1 contribute to obesity and Prader-Willi-like features. J Clin Invest. 123, 3037-3041. doi: 3010.1172/JCI68035. Epub 62013 Jun 68017. (2013).
17 Michaud, J. L. et al. Sim1 haploinsufficiency causes hyperphagia, obesity and reduction of the paraventricular nucleus of the hypothalamus. Hum Mol Genet 10, 1465-1473 (2001).
18 Tolson, K. P. et al. Postnatal Sim1 deficiency causes hyperphagic obesity and reduced Mc4r and oxytocin expression. J 30, 3803-3812. (2010).
19 Kublaoui, B. M., Holder, J. L., Jr., Tolson, K. P., Gemelli, T. & Zinn, A. R. SIM1 overexpression partially rescues agouti yellow and diet-induced obesity by normalizing food intake. Endocrinology. 147, 4542-4549. Epub 2006 May 4518. (2006).
20 Yang, C., Boucher, F., Tremblay, A. & Michaud, J. L. Regulatory interaction between arylhydrocarbon receptor and SIM1, two basic helix-loop-helix PAS proteins involved in the control of food intake. J Biol Chem. 279, 9306-9312. Epub 2003 Dec 9301. (2004).
21 Kim, M. J., Oksenberg, N., Hoffmann, T. J., Vaisse, C. & Ahituv, N. Functional characterization of SIM1-associated enhancers. Hum Mol Genet (2013).
22 Flint, J. & Shenk, T. Viral transactivating proteins. Annu Rev Genet. 31, 177-212. (1997).
23 Chavez, A. et al. Comparison of Cas9 activators in multiple species. Nat Methods. 13, 563-567. doi: 510.1038/nmeth.3871. Epub 2016 May 1023. (2016).
24 Tasic, B. et al. Site-specific integrase-mediated transgenesis in mice via pronuclear injection. Proc Natl Acad Sci U S A. 108, 7902-7907. doi: 7910.1073/pnas.1019507108. Epub 1019502011 Apr 1019507104. (2011).
25 Su, A. I. et al. A gene atlas of the mouse and human protein-encoding transcriptomes. Proc Natl Acad Sci U S A. 101, 6062-6067. Epub 2004 Apr 6069. (2004).
26 Wu, Z., Yang, H. & Colosi, P. Effect of genome size on AAV vector packaging. Mol Ther. 18, 80-86. doi: 10.1038/mt.2009.1255. Epub 2009 Nov 1010. (2010).
27 Zincarelli, C., Soltys, S., Rengo, G. & Rabinowitz, J. E. Analysis of AAV serotypes 1-9 mediated gene expression and tropism in mice after systemic injection. Mol Ther. 16, 1073-1080. doi: 1010.1038/mt.2008.1076. Epub 2008 Apr 1015. (2008).
28 Weise, A. et al. Microdeletion and microduplication syndromes. J Histochem Cytochem. 60, 346-358. doi: 310.1369/0022155412440001. Epub 0022155412442012 Mar 0022155412440006. (2012).
29 Horlbeck, M. A. et al. Nucleosomes impede Cas9 access to DNA and. Elife. 5., e12677. doi: 12610.17554/eLife.12677. (2016).
30 Otchy, T. M. et al. Acute off-target effects of neural circuit manipulations. Nature. 528, 358-363. doi: 310.1038/nature16442. Epub 12015 Dec 16449. (2015).
31 Bondy-Denomy, J., Pawluk, A., Maxwell, K. L. & Davidson, A. R. Bacteriophage genes that inactivate the CRISPR/Cas bacterial immune system. Nature. 493, 429-432. doi: 410.1038/nature11723. Epub 12012 Dec 11716. (2013).
32 Wang, D. et al. Adenovirus-Mediated Somatic Genome Editing of Pten by CRISPR/Cas9 in Mouse Liver in Spite of Cas9-Specific Immune Responses. Hum Gene Ther. 26, 432-442. doi: 410.1089/hum.2015.1087. (2015).
33 Chew, W. L. et al. A multifunctional AAV-CRISPR-Cas9 and its host response. Nat Methods. 13, 868-874. doi: 810.1038/nmeth.3993. Epub 2016 Sep 1035. (2016).
34 Donsante, A. et al. AAV vector integration sites in mouse hepatocellular carcinoma. Science. 317, 477. (2007).
35 Chandler, R. J. et al. Vector design influences hepatic genotoxicity after adeno-associated virus gene therapy. J Clin Invest. 125, 870-880. doi: 810.1172/JCI79213. Epub 72015 Jan 79220. (2015).
36 Nault, J. C. et al. Recurrent AAV2-related insertional mutagenesis in human hepatocellular carcinomas. Nat Genet. 47, 1187-1193. doi: 1110.1038/ng.3389. Epub 2015 Aug 1124. (2015).
37 Deng, W. et al. Reactivation of developmentally silenced globin genes by forced chromatin looping. Cell. 158, 849-860. doi: 810.1016/j.cell.2014.1005.1050. (2014).
38 Canver, M. C. et al. BCL11A enhancer dissection by Cas9-mediated in situ saturating mutagenesis. Nature. 527, 192-197. doi: 110.1038/nature15521. Epub 12015 Sep 15516. (2015).
39 Hirst, R. C., McCullagh, K. J. & Davies, K. E. Utrophin upregulation in Duchenne muscular dystrophy. Acta Myol. 24, 209-216. (2005).
40 Sproule, D. M. & Kaufmann, P. Therapeutic developments in spinal muscular atrophy. Ther Adv Neurol Disord. 3, 173-185. doi: 110.1177/1756285610369026. (2010).
41 Nagy, A., Gertsenstein, M., Vintersten, K. & Behringer, R. Manipulating the mouse embryo: A laboratory manual. 3rd edition edn, (Cold Spring Harbor, 2002).
42 Beuckmann, C. T. et al. Expression of a poly-glutamine-ataxin-3 transgene in orexin neurons induces narcolepsy-cataplexy in the rat. J Neurosci. 24, 4469-4477. (2004).
43 Kublaoui, B. M., Gemelli, T., Tolson, K. P., Wang, Y. & Zinn, A. R. Oxytocin deficiency mediates hyperphagic obesity of Sim1 haploinsufficient mice. Mol Endocrinol. 22, 1723-1734. doi: 1710.1210/me.2008-0067. Epub 2008 May 1721. (2008).

## Claims

1. A composition comprising:
i) a guide RNA, wherein the guide RNA comprises:
a) a targeting region that, under conditions present in a nucleus of the cell, specifically hybridizes to a promoter region or an enhancer region operably linked to a wild-type copy of a haploinsufficient gene, wherein the haploinsufficient gene is SCN2A, SIM1 or MC4R; and
b) a CRISPR nuclease-binding region that specifically binds a CRISPR nuclease under conditions present in a nucleus of the cell or a region that specifically binds to the CRISPR nuclease-binding region; and
ii) the CRISPR nuclease,
for use in a method of treating haploinsufficiency disease in a mammalian subject, the method comprising contacting a cell of the subject with the composition;
- wherein the contacting forms a complex comprising the CRISPR nuclease bound to the guide RNA, wherein the targeting region of the guide RNA in the complex is hybridized to the promoter or enhancer;
- wherein the complex comprises a catalytically inactive CRISPR nuclease and a transcriptional activation domain, and
- wherein the complex activates transcription of the wild-type copy of the SCN2A, SIM1 or MC4R gene that in the absence of transcriptional activation by the complex does not produce enough of a corresponding gene product to produce a wild-type phenotype in the subject, in an amount and for a duration sufficient to treat the haploinsufficiency disease in the subject.

2. The composition for use according to claim 1, wherein the contacting comprises contacting the cell with an episomal vector encoding the guide RNA or the CRISPR nuclease; optionally wherein:
(a) the contacting comprises contacting the cell with an episomal vector encoding the guide RNA and the CRISPR nuclease; or
(b) the contacting comprises contacting the cell with an episomal vector encoding the guide RNA and a second episomal vector encoding the CRISPR nuclease.

3. The composition for use according to any one of the preceding claims, wherein:
(a) the episomal vector(s) are non-integrating;
(b) the episomal vector(s) are non-replicating;
(c) the episomal vector(s) are adeno-associated virus (AAV) vectors; and/or
(d) the episomal vector(s) independently comprise a first and a second end, wherein the first end and second end each independently comprise an AAV inverted terminal repeat.

4. The composition for use according to any one of the preceding claims, wherein:
(a) the CRISPR nuclease comprises (i) a nuclease domain that has been modified to eliminate nuclease and nicking activity and (ii) a transcriptional activation domain;
(b) the CRISPR nuclease has a modification and the modification comprises a mutation at positions corresponding to D10 and H840 of *S. pyogenes* Cas9;
(c) the CRISPR nuclease comprises a D10A, H840A *S. pyogenes* dCas9;
(d) the guide RNA comprises a dead guide sequence; and/or
(e) the guide RNA comprises a transcriptional activation binding domain, wherein the transcriptional activation binding domain specifically binds a composition comprising one or more transcriptional activation domains.

5. The composition for use according to any one of the preceding claims, wherein:
(a) the complex comprising the CRISPR nuclease bound to the guide RNA further comprises a transcriptional activation domain selected from the group consisting of HSF1, VP16, VP64, p65, MyoD1, RTA, SET7/9, VPR, histone acetyltransferase p300, an hydroxylase catalytic domain of a TET family protein *(e.g.,* TET1 hydroxylase catalytic domain), LSD1, CIB1, AD2, CR3, EKLF1, GATA4, PRVIE, p53, SP1, MEF2C, TAX, and PPARγ; and/or
(b) the CRISPR nuclease is a CRISPR nuclease-VP64 fusion polypeptide.

6. The composition for use according to any one of the preceding claims, wherein the guide RNA comprises a scaffold region;
optionally wherein the scaffold region comprises an ms2, f6, PP7, com, or L7a ligand sequence;
further optionally wherein the scaffold region of the guide RNA in the complex is bound to a transcriptional activation domain fused to an MCP polypeptide, a COM polypeptide, a PCP polypeptide, or an L7a polypeptide.

7. The composition for use according to any one of claims 1 - 6, wherein the targeting region of the guide RNA is encoded by or specifically hybridizes to:
SEQ ID NO:38 (GCTCACTGTAGGACCCGAGCC),
SEQ ID NO:39 (GACGCGGCGCTCATTGGCCAA),
SEQ ID NO:40 (CGAGCCGCGAGCCCAGTCGCG),
SEQ ID NO:41 (TCCCCCCCCCCCCCCACGCGA),
SEQ ID NO:42 (GTCACTCACCCCGATTGGCCA), or
SEQ ID NO:43 (CGCGAGCCCAGTCGCGTGGGG).

8. The composition for use according to any one of claims 1 - 6, wherein:
(a) the targeting region of the guide RNA is encoded by or specifically hybridizes to:
SEQ ID NO:44 (GTTGGCTTATCCAAACATCTC),
SEQ ID NO:45 (ATGTTAAGCAAGGGTAATAGA),
SEQ ID NO:46 (CTGTGAAAGGAATACAATTCA),
SEQ ID NO:47 (GCCAATTCTTGGCAACCGAGC), or
SEQ ID NO:49 (AATTAGCAGACAGCTTGGTAC); or
(b) the targeting region of the guide RNA is encoded by or specifically hybridizes to:
SEQ ID NO:51 (CACTGAATACGGATTGGTCAG),
SEQ ID NO:52 (GATGTCTCAGAACCACTGAAT), or
SEQ ID NO:54 (ACCAATCCGTATTCAGTGGTT).

9. The composition for use according to any one of claims 1 - 6, wherein:
(a) the targeting region of the guide RNA is encoded by or specifically hybridizes to:
SEQ ID NO:85 (CCTTTCTATGACCTAGTCGG),
SEQ ID NO:86 (CAGAATCAGTAACGCACTGT),
SEQ ID NO:87 (GAAACCAGGAGAGATAACCC),
SEQ ID NO:88 (GGACCCCAGATATTCTGGAA), or
SEQ ID NO:89 (TTATTGTTGACTTAACGAAG); or
(b) the targeting region of the guide RNA is encoded by or specifically hybridizes to:
SEQ ID NO:92 (TGTTGGTTTATTGGACCCCAGATATTC),
SEQ ID NO:93 (TGTTGGAGAAAATTAACTTAGTGCATA), or
SEQ ID NO:94 (TGTTGGTATAACTGCCACTAGAGGGCT).

10. The composition for use according to any one of the preceding claims, wherein:
(a) the cell is a non-dividing cell;
(b) the cell is a neuron;
(c) the cell is a hypothalamus cell;
(d) the contacting comprises injection of nucleic acid encoding the guide RNA and/or the CRISPR nuclease into a region of a brain containing a hypothalamus;
(e) the contacting comprises injection of an adeno-associated viral vector comprising nucleic acid encoding the guide RNA and/or the CRISPR nuclease into a region of a brain containing a hypothalamus; and/or
(f) the haploinsufficiency disease is selected from the group consisting of obesity, autism, and epilepsy.

11. An isolated mammalian host cell comprising:
I.) a genome comprising at least one functional copy of a target gene, wherein the target gene is selected from the group consisting of SCN2A, SIM1 and MC4R, wherein the functional cop(y/ies) in the absence of transcriptional activation by a heterologous complex do not produce enough of a corresponding gene product to produce a wild-type phenotype in an organism; and
II.) the heterologous complex, wherein the heterologous complex comprises:
a) a guide RNA, wherein the guide RNA comprises:
i.) a targeting region that specifically hybridizes to a promoter region or an enhancer region operably linked to the functional cop(y/ies) of the target gene under conditions present in a nucleus of the cell; and
ii.) a CRISPR nuclease-binding region that specifically binds a CRISPR nuclease under conditions present in a nucleus of the cell; and
b) the CRISPR nuclease,
- wherein the guide RNA of the heterologous complex comprising the CRISPR nuclease bound to the guide RNA is hybridized to the promoter or enhancer;
- wherein the CRISPR nuclease is catalytically inactive, and
- wherein the complex activates transcription of the functional cop(y/ies) of the SCN2A, SIM1 or MC4R target gene in an amount and for a duration sufficient to produce a wild-type phenotype when the host cell is present in an organism.

## Patentansprüche

1. Zusammensetzung umfassend:
i) eine Leit-RNA, wobei die Leit-RNA Folgendes umfasst:
a) eine Zielregion, die unter Bedingungen, die in einem Kern der Zelle vorliegen, spezifisch mit einer Promoter-Region oder einer Enhancer-Region hybridisiert, die mit einer Wildtyp-Kopie eines haploinsuffizienten Gens wirkverbunden ist, wobei das haploinsuffiziente Gen SCN2A, SIM1 oder MC4R ist; und
b) eine CRISPR-Nukleasebindungsregion, die eine CRISPR-Nuklease spezifisch unter Bedingungen bindet, die in einem Kern der Zelle oder einer Region vorliegen, die spezifisch an die CRISPR-Nukleasebindungsregion bindet; und
ii) die CRISPR-Nuklease,
zur Verwendung bei einem Verfahren zum Behandeln der Haploinsuffizienzkrankheit bei einem Säugetiersubjekt, wobei das Verfahren das Inkontaktbringen einer Zelle des Subjekts mit der Zusammensetzung umfasst;
- wobei das Inkontaktbringen einen Komplex bildet, der die CRISPR-Nuklease umfasst, die an die Leit-RNA gebunden ist, wobei die Zielregion der Leit-RNA in dem Komplex mit dem Promoter oder Enhancer hybridisiert wird;
- wobei der Komplex eine katalytisch inaktive CRISPR-Nuklease und eine Transkriptionsaktivierungsdomäne umfasst, und
- wobei der Komplex die Transkription der Wildtyp-Kopie des SCN2A-, SIM1- oder MC4R-Gens aktiviert, die bei fehlender Transkriptionsaktivierung durch den Komplex nicht genügend von einem entsprechenden Genprodukt produziert, um einen Wildtyp-Phänotyp in dem Subjekt in einer Menge und während einer Dauer zu produzieren, die ausreichen, um die Haploinsuffizienzkrankheit bei dem Subjekt zu behandeln.

2. Zusammensetzung zur Verwendung nach Anspruch 1, wobei das Inkontaktbringen das Inkontaktbringen der Zelle mit einem episomalen Vektor umfasst, der die Leit-RNA oder die CRISPR-Nuklease codiert; wobei optional:
(a) das Inkontaktbringen das Inkontaktbringen der Zelle mit einem episomalen Vektor umfasst, der die Leit-RNA und die CRISPR-Nuklease codiert; oder
(b) das Inkontaktbringen das Inkontaktbringen der Zelle mit einem episomalen Vektor, der die Leit-RNA codiert, und einem zweiten episomalen Vektor, der die CRISPR-Nuklease codiert, umfasst.

3. Zusammensetzung zur Verwendung nach einem der vorherigen Ansprüche, wobei:
(a) der/die episomale(n) Vektor(en) nicht-integrierend ist/sind;
(b) der/die episomale(n) Vektor(en) nicht-replizierend ist/sind;
(c) der/die episomale(n) Vektor(en) adeno-assoziierte Virusvektoren (AAV-Vektoren) ist/sind; und/oder
(d) der/die episomale(n) Vektor(en) unabhängig ein erstes und ein zweites Ende umfasst/umfassen, wobei das erste Ende und das zweite Ende jeweils unabhängig eine invertierte terminale Wiederholung des AAV umfassen.

4. Zusammensetzung zur Verwendung nach einem der vorherigen Ansprüche, wobei:
(a) die CRISPR-Nuklease (i) eine Nukleasedomäne, die modifiziert wurde, um die Nuklease- und Nicking-Aktivität zu entfernen, und (ii) eine Transkriptionsaktivierungsdomäne umfasst;
(b) die CRISPR-Nuklease eine Modifikation aufweist und die Modifikation eine Mutation in Positionen entsprechend D10 und H840 von *S. pyogenes* Cas9 umfasst;
(c) die CRISPR-Nuklease ein D10A, H840A *S. pyogenes* dCas9 umfasst;
(d) die Leit-RNA eine tote Leitsequenz umfasst; und/oder
(e) die Leit-RNA eine Transkriptionsaktivierungsbindungsdomäne umfasst, wobei die Transkriptionsaktivierungsbindungsdomäne spezifisch eine Zusammensetzung bindet, die eine oder mehrere Transkriptionsaktivierungsdomänen umfasst.

5. Zusammensetzung zur Verwendung nach einem der vorherigen Ansprüche, wobei:
(a) der Komplex, der die CRISPR-Nukleasebindung an die Leit-RNA umfasst, ferner eine Transkriptionsaktivierungsdomäne umfasst, die aus der Gruppe bestehend aus HSF1, VP16, VP64, p65, MyoD1, RTA, SET7/9, VPR, Histonacetyltransferase p300, einer Hydroxylase-katalytischen Domäne eines Proteins der TET-Familie (z. B. Hydroxylasekatalytische TET1-Domäne), LSD1, CIB1, AD2, CR3, EKLF1, GATA4, PRVIE, p53, SP1, MEF2C, TAX und PPARγ ausgewählt ist; und/oder
(b) die CRISPR-Nuklease ein CRISPR-Nuklease-VP64-Fusionspolypeptid ist.

6. Zusammensetzung zur Verwendung nach einem der vorherigen Ansprüche, wobei die Leit-RNA eine Gerüstregion umfasst;
wobei optional die Gerüstregion eine ms2-, f6-, PP7-, com- oder L7a-Ligandensequenz umfasst;
wobei ferner optional die Gerüstregion der Leit-RNA in dem Komplex an eine Transkriptionsaktivierungsdomäne gebunden ist, die mit einem MCP-Polypeptid, einem COM-Polypeptid, einem PCP-Polypeptid oder einem L7a-Polypeptid fusioniert ist.

7. Zusammensetzung zur Verwendung nach einem der Ansprüche 1 - 6, wobei
die Zielregion der Leit-RNA durch Folgendes codiert ist oder spezifisch mit Folgendem hybridisiert:
SEQ ID NO:38 (GCTCACTGTAGGACCCGAGCC),
SEQ ID NO:39 (GACGCGGCGCTCATTGGCCAA),
SEQ ID NO.40 (GCAGCCGCGAGCCCAGTCGCG),
SEQ ID NO:41 (TCCCCCCCCCCCCCCACGCGA),
SEQ ID NO: 42 (GTCACTCACCCCGATTGGCCA), oder
SEQ ID NO: 43 (CGCGAGCCCAGTCGCGTGGGG).

8. Zusammensetzung zur Verwendung nach einem der Ansprüche 1 - 6, wobei:
(a) die Zielregion der Leit-RNA durch Folgendes codiert ist oder spezifisch mit Folgendem hybridisiert:
SEQ ID NO: 44 (GTTGGCTTATCCAAACATCTC),
SEQ ID NO:45 (ATGTTAAGCAAGGGTAATAGA),
SEQ ID NO:46 (CTGTGAAAGGAATACAATTCA),
SEQ ID NO: 47 (GCCAATTCTTGGCAACCGAGC), oder
SEQ ID NO: 49 (AATTAGCAGACAGCTTGGTAC); oder
(b) die Zielregion der Leit-RNA durch Folgendes codiert ist oder spezifisch mit Folgendem hybridisiert:
SEQ ID NO:51 (CACTGAATACGGATTGGTCAG),
SEQ ID NO: 52 (GATGTCTCAGAACCACTGAAT), oder
SEQ ID NO: 54 (ACCAATCCGTATTCAGTGGTT).

9. Zusammensetzung zur Verwendung nach einem der Ansprüche 1 - 6, wobei:
(a) die Zielregion der Leit-RNA durch Folgendes codiert ist oder spezifisch mit Folgendem hybridisiert:
SEQ ID NO: 85 (CCTTTCTATGACTAGTCGG),
SEQ ID NO: 86 (CAGAATCAGTAACGCACTGT),
SEQ ID NO: 87 (GAAACCAGGAGAGATAACCC),
SEQ ID NO: 88 (GGACCCCAGATATTCTGGAA), oder
SEQ ID NO: 89 (TTATTGTTGACTTAACGAAG); oder
(b) die Zielregion der Leit-RNA durch Folgendes codiert ist oder spezifisch mit Folgendem hybridisiert:
SEQ ID NO: 92 (TGTTGGTTTATTGGACCCCAGATATTC),
SEQ ID NO: 93 (TGTTGGAGAAAATTAACTTAGTGCATA), oder
SEQ ID NO: 94 (TGTTGGTATAACTGCCACTAGAGGGCT).

10. Zusammensetzung zur Verwendung nach einem der vorherigen Ansprüche, wobei:
(a) die Zelle eine nicht-teilende Zelle ist;
(b) die Zelle eine Nervenzelle ist;
(c) die Zelle eine Hypothalamuszelle ist;
(d) das Inkontaktbringen die Injektion von Nukleinsäure, die die Leit-RNA und/oder die CRISPR-Nuklease codiert, in eine Region eines Gehirns, das einen Hypothalamus enthält, umfasst;
(e) das Inkontaktbringen die Injektion eines adeno-assoziierten viralen Vektors, der Nukleinsäure umfasst, die die Leit-RNA und/oder die CRISPR-Nuklease codiert, in eine Region eines Gehirns, das einen Hypothalamus enthält, umfasst; und/oder
(f) die Haploinsuffizienzkrankheit aus der Gruppe bestehend aus Fettleibigkeit, Autismus und Epilepsie ausgewählt wird.

11. Isolierte Säugetierstammzelle umfassend:
I.) ein Genom umfassend mindestens eine funktionale Kopie eines Zielgens, wobei das Zielgen aus der Gruppe bestehend aus SCN2A, SIM1 und MC4R ausgewählt ist, wobei die funktionale(n) Kopie(n) bei fehlender Transkriptionsaktivierung durch einen heterologen Komplex nicht genügend eines entsprechenden Genprodukts produzieren, um einen Wildtyp-Phänotyp in einem Organismus zu produzieren; und
II.) den heterologen Komplex, wobei der heterologe Komplex Folgendes umfasst:
a) eine Leit-RNA, wobei die Leit-RNA Folgendes umfasst:
i.) eine Zielregion, die spezifisch mit einer Promoter-Region oder einer Enhancer-Region hybridisiert, die mit der/den funktionalen Kopie(n) des Zielgens unter Bedingungen wirkverbunden ist, die in einem Kern der Zelle vorliegen; und
ii.) eine CRISPR-Nukleasebindungsregion, die spezifisch eine CRISPR-Nuklease unter Bedingungen bindet, die in einem Kern der Zelle vorliegen; und
b) die CRISPR-Nuklease,
- wobei die Leit-RNA des heterologen Komplexes umfassend die CRISPR-Nuklease, die an die Leit-RNA gebunden ist, mit dem Promoter oder Enhancer hybridisiert wird;
- wobei die CRISPR-Nuklease katalytisch inaktiv ist, und
- wobei der Komplex die Transkription der funktionalen Kopie(n) des SCN2A-, SIM1- oder MC4R-Zielgens in einer Menge und während einer Dauer aktiviert, die ausreichen, um einen Wildtyp-Phänotyp zu produzieren, wenn die Stammzelle in einem Organismus vorhanden ist.

## Revendications

1. Composition comprenant :
i) un ARN guide, dans laquelle l'ARN guide comprend :
a) une région de ciblage qui, dans des conditions présentes dans un noyau de la cellule, s'hybride spécifiquement à une région promotrice ou à une région amplificatrice liée de manière fonctionnelle à une copie de type sauvage d'un gène haploinsuffisant, dans laquelle le gène haploinsuffisant est SCN2A, SIM1 ou MC4R ; et
b) une région de liaison à une nucléase CRISPR qui se lie spécifiquement à une nucléase CRISPR dans des conditions présentes dans un noyau de la cellule ou une région qui se lie spécifiquement à la région de liaison à une nucléase CRISPR ; et
ii) la nucléase CRISPR,
destinée à être utilisée dans une méthode de traitement d'une maladie d'haploinsuffisance chez un sujet mammifère, la méthode comprenant la mise en contact d'une cellule du sujet avec la composition ;
- dans laquelle la mise en contact forme un complexe comprend la nucléase CRISPR liée à l'ARN guide, dans laquelle la région de ciblage de l'ARN guide dans le complexe est hybridée au promoteur ou à l'amplificateur ;
- dans laquelle le complexe comprend une nucléase CRISPR catalytiquement inactive et un domaine d'activation transcriptionnelle, et
- dans laquelle le complexe active une transcription de la copie de type sauvage du gène SCN2A, SIM1 ou MC4R qui, en l'absence d'activation transcriptionnelle par le complexe, ne produit pas une quantité suffisante du produit génique correspondant pour induire un phénotype de type sauvage chez le sujet, en une quantité et pendant une durée suffisantes pour traiter la maladie d'haploinsuffisance chez le sujet.

2. Composition destinée à être utilisée selon la revendication 1, dans laquelle la mise en contact comprend la mise en contact de la cellule avec un vecteur épisomique codant pour l'ARN guide ou la nucléase CRISPR ; éventuellement dans laquelle :
(a) la mise en contact comprend la mise en contact de la cellule avec un vecteur épisomique codant pour l'ARN guide et la nucléase CRISPR ; ou
(b) la mise en contact comprend la mise en contact de la cellule avec un vecteur épisomique codant pour l'ARN guide et un deuxième vecteur épisomique codant pour la nucléase CRISPR.

3. Composition destinée à être utilisée selon l'une quelconque des revendications précédentes, dans laquelle :
(a) le ou les vecteurs épisomiques sont non intégrants ;
(b) le ou les vecteurs épisomiques sont non réplicatifs ;
(c) le ou les vecteurs épisomiques sont des vecteurs de virus adéno-associés (AAV) ; et/ou
(d) le ou les vecteurs épisomiques comprennent indépendamment une première et une deuxième extrémité, dans laquelle la première extrémité et la deuxième extrémité comprennent chacune indépendamment une répétition terminale inversée d'AAV.

4. Composition destinée à être utilisée selon l'une quelconque des revendications précédentes, dans laquelle :
(a) la nucléase CRISPR comprend (i) un domaine nucléase qui a été modifié pour éliminer l'activité nucléase et l'activité d'encochage et (ii) un domaine d'activation transcriptionnelle ;
(b) la nucléase CRISPR présente une modification et la modification comprend une mutation aux positions correspondant à D10 et H840 de *S. pyogenes* Cas9 ;
(c) la nucléase CRISPR comprend une *S. pyogenes* Cas9 D10A, H840A ;
(d) l'ARN guide comprend une séquence guide inactive ; et/ou
(e) l'ARN guide comprend un domaine de liaison d'activation transcriptionnelle, dans laquelle le domaine de liaison d'activation transcriptionnelle se lie spécifiquement à une composition comprenant un ou plusieurs domaines d'activation transcriptionnelle.

5. Composition destinée à être utilisée selon l'une quelconque des revendications précédentes, dans laquelle :
(a) le complexe comprenant la nucléase CRISPR liée à l'ARN guide comprend en outre un domaine d'activation transcriptionnelle sélectionné parmi le groupe constitué de HSF1, VP16, VP64, p65, MyoD1, RTA, SET7/9, VPR, l'histone acétyltransférase p300, un domaine catalytique hydroxylase d'une protéine de la famille TET (par exemple, le domaine catalytique hydroxylase de TET1), LSD1, CIB1, AD2, CR3, EKLF1, GATA4, PRVIE, p53, SP1, MEF2C, TAX, et PPARγ ; et/ou
(b) la nucléase CRISPR est un polypeptide de fusion nucléase CRISPR-VP64.

6. Composition destinée à être utilisée selon l'une quelconque des revendications précédentes, dans laquelle l'ARN guide comprend une région d'échafaudage ;
éventuellement dans laquelle la région d'échafaudage comprend une séquence ligand ms2, f6, PP7, com, ou L7a ;
éventuellement en outre dans laquelle la région d'échafaudage de l'ARN guide dans le complexe est liée à un domaine d'activation transcriptionnelle fusionné à un polypeptide MCP, un polypeptide COM, un polypeptide PCP, ou un polypeptide L7a.

7. Composition destinée à être utilisée selon l'une quelconque des revendications 1 à 6, dans laquelle la région de ciblage de l'ARN guide est codée par ou s'hybride spécifiquement à :
SEQ ID N° : 38 (GCTCACTGTAGGACCCGAGCC),
SEQ ID N° : 39 (GACGCGGCGCTCATTGGCCAA),
SEQ ID N° : 40 (CGAGCCGCGAGCCCAGTCGCG),
SEQ ID N° : 41 (TCCCCCCCCCCCCCCACGCGA),
SEQ ID N° : 42 (GTCACTCACCCCGATTGGCCA), ou
SEQ ID N° : 43 (CGCGAGCCCAGTCGCGTGGGG).

8. Composition destinée à être utilisée selon l'une quelconque des revendications 1 à 6, dans laquelle :
(a) la région de ciblage de l'ARN guide est codée par ou s'hybride spécifiquement à :
SEQ ID N° : 44 (GTTGGCTTATCCAAACATCTC),
SEQ ID N° : 45 (ATGTTAAGCAAGGGTAATAGA),
SEQ ID N° : 46 (CTGTGAAAGGAATACAATTCA),
SEQ ID N° : 47 (GCCAATTCTTGGCAACCGAGC), ou
SEQ ID N° : 49 (AATTAGCAGACAGCTTGGTAC) ; ou
(b) la région de ciblage de l'ARN guide est codée par ou s'hybride spécifiquement à :
SEQ ID N° : 51 (CACTGAATACGGATTGGTCAG),
SEQ ID N° : 52 (GATGTCTCAGAACCACTGAAT), ou
SEQ ID N° : 54 (ACCAATCCGTATTCAGTGGTT).

9. Composition destinée à être utilisée selon l'une quelconque des revendications 1 à 6, dans laquelle :
(a) la région de ciblage de l'ARN guide est codée par ou s'hybride spécifiquement à :
SEQ ID N° : 85 (CCTTTCTATGACCTAGTCGG),
SEQ ID N° : 86 (CAGAATCAGTAACGCACTGT),
SEQ ID N° : 87 (GAAACCAGGAGAGATAACCC),
SEQ ID N° : 88 (GGACCCCAGATATTCTGGAA), ou
SEQ ID N° : 89 (TTATTGTTGACTTAACGAAG) ; ou
(b) la région de ciblage de l'ARN guide est codée par ou s'hybride spécifiquement à :
SEQ ID N° : 92 (TGTTGGTTTATTGGACCCCAGATATTC),
SEQ ID N° : 93 (TGTTGGAGAAAATTAACTTAGTGCATA), ou
SEQ ID N° : 94 (TGTTGGTATAACTGCCACTAGAGGGCT).

10. Composition destinée à être utilisée selon l'une quelconque des revendications précédentes, dans laquelle :
(a) la cellule est une cellule qui ne se divise pas ;
(b) la cellule est un neurone ;
(c) la cellule est une cellule de l'hypothalamus ;
(d) la mise en contact comprend l'injection d'acide nucléique codant pour l'ARN guide et/ou la nucléase CRISPR dans une région du cerveau contenant l'hypothalamus ;
(e) la mise en contact comprend l'injection d'un vecteur viral adéno-associé comprenant un acide nucléique codant pour l'ARN guide et/ou la nucléase CRISPR dans une région du cerveau contenant l'hypothalamus ; et/ou
(f) la maladie d'haploinsuffisance est sélectionnée parmi le groupe constitué de l'obésité, de l'autisme, et de l'épilepsie.

11. Cellule hôte de mammifère isolée comprenant :
I.) un génome comprenant au moins une copie fonctionnelle d'un gène cible, dans laquelle le gène cible est sélectionné parmi le groupe constitué de SCN2A, SIM1 et MC4R, dans laquelle la ou les copies fonctionnelles, en l'absence d'activation transcriptionnelle par un complexe hétérologue, ne produisent pas une quantité suffisante du produit génique correspondant pour produire un phénotype de type sauvage dans un organisme ; et
II.) le complexe hétérologue, dans laquelle le complexe hétérologue comprend :
a) un ARN guide, dans lequel l'ARN guide comprend :
i.) une région de ciblage qui s'hybride spécifiquement à une région promotrice ou à une région amplificatrice liée de manière fonctionnelle à la ou aux copies fonctionnelles du gène cible dans des conditions présentes dans un noyau de la cellule ; et
ii.) une région de liaison à une nucléase CRISPR qui se lie spécifiquement à une nucléase CRISPR dans des conditions présentes dans un noyau de la cellule ; et
b) la nucléase CRISPR,
- dans laquelle l'ARN guide du complexe hétérologue comprenant la nucléase CRISPR liée à l'ARN guide est hybridé au promoteur ou à l'amplificateur ;
- dans laquelle la nucléase est catalytiquement inactive, et
- dans laquelle le complexe active une transcription de la ou des copies fonctionnelles du gène cible SCN2A, SIM1 ou MC4R en quantité et pendant une durée suffisantes pour produire un phénotype de type sauvage lorsque la cellule hôte est présente dans un organisme.
